(19) **Europäisches Patentamt / European Patent Office / Office européen des brevets**

(11) **EP 1 858 489 B1**

(12) **EUROPEAN PATENT SPECIFICATION**

(45) Date of publication and mention of the grant of the patent:
**27.02.2019 Bulletin 2019/09**

(21) Application number: **05724464.2**

(22) Date of filing: **03.03.2005**

(51) Int Cl.:
**A61K 9/22** *(2006.01)*    **A23P 10/28** *(2016.01)*

(86) International application number:
**PCT/US2005/006924**

(87) International publication number:
**WO 2006/096161 (14.09.2006 Gazette 2006/37)**

(54) **METHODS AND COMPOSITIONS FOR MODIFIED RELEASE OF NUTRITIONAL SUPPLEMENTS**

VERFAHREN UND ZUSAMMENSETZUNGEN FÜR DIE MODIFIZIERTE FREISETZUNG VON NAHRUNGSERGÄNZUNGSMITTELN

PROCÉDÉS ET COMPOSITIONS POUR UNE LIBÉRATION MODIFIÉE DE SUPPLÉMENTS NUTRITIONNELS

(84) Designated Contracting States:
**AT BE BG CH CY CZ DE DK EE ES FI FR GB GR HU IE IS IT LI LT LU MC NL PL PT RO SE SI SK TR**

(43) Date of publication of application:
**28.11.2007 Bulletin 2007/48**

(73) Proprietor: **Mannatech, Inc.**
**Coppell, TX 75019 (US)**

(72) Inventors:
• **McANALLEY, Bill, H.**
  **Grand Prairie, TX 75052 (US)**
• **VENNUM, Eileen**
  **Grand Prairie, TX 75050 (US)**
• **McANALLEY, Shayne, A.**
  **Galveston, TX 77550 (US)**
• **KOEPKE, C., Michael**
  **Houston, Texas 77030 (US)**
• **EDWARDS, Josh**
  **Lewisville, TX 75057 (US)**

(74) Representative: **Sonn & Partner Patentanwälte**
**Riemergasse 14**
**1010 Wien (AT)**

(56) References cited:
WO-A1-98/06418     WO-A2-97/02829
US-A- 6 063 403     US-B1- 6 805 880

## Description

FIELD OF THE INVENTION

The present invention relates in general to the field of modified-release of nutritional supplements, and more particularly, to compositions, formulations and methods for providing nutritional supplementation that more closely resembles that of food during digestion.

BACKGROUND OF THE INVENTION

Without limiting the scope of the invention, its background is described in connection with antioxidant sensor technology and methods for detection.

Biological systems have developed antioxidant systems to combat the effects of radicals and other pro-oxidative species. An antioxidant is any substance that significantly delays or prevents oxidation of an oxidizable substrate when present at low concentrations compared to that of the oxidizable substrate. There are enzymes that are antioxidants, such as superoxide dismutase and catalase, which are coded for by many organisms. Substances such as vitamin C and plant phenols are antioxidants introduced through the diet into biological systems. It has been proposed that naturally occurring levels of these substances are not adequately produced in the body or ingested in the normal diet. The normal diet often does not provide enough antioxidants because it is deficient in fruits and vegetables and/or the fruits and vegetables that are in the diet are depleted of their antioxidants due to modern-day processing. The normal diet could be improved, however, today's lifestyles and poor composition of western foods, make supplementation the most practical way to deliver the anti-oxidants required by the body. To supplement the normal diet, it is necessary to determine the antioxidant capacities of the components included in the supplements.

WO 97/02829 A2 describes a method for preventing and treating diarrhea by enterally administering indigestible oligosaccharides.

WO 98/06418 A1 discloses glyconutritional compositions comprising plant carbohydrates for dietary supplements and nutritional support.

US 6 063 403 A discloses compressed dry-granulation tablets or granules containing desogestrel and a method of manufacture of such tablets or granules.

US 6 805 880 B1 discloses a pharmaceutical delivery system that comprises a slow release formulation of vitamin C and a plain-release formulation of vitamin E.

SUMMARY OF THE INVENTION

The present invention includes compositions, methods and formulations for the modified-release of nutritionally effective amounts of, e.g., anti-oxidants, vitamins, saccharides, minerals, amino acids, nucleic acids, mixtures and combinations thereof, wherein the supplement comprises a nutritionally effective amount of one or more saccharides comprising one or more long-chain polysaccharides and a nutritionally effective amount of one or more of said nutritional supplements, wherein the dietary supplement is compressed at a pressure greater than 100 psi, wherein the long-chain polysaccharides comprise both a time releasing agent for bioactive molecules and a portion of the dietary supplement, wherein one or more long-chain polysaccharides are isolated from gum tragacanth, guar gum, grain flour, Larch tree extract, aloe vera extract, gum ghatti, starch, gum arabic, xanthan gum, chondroitin sulfate, acetylated polymannose, and mixtures or combinations thereof; wherein the one or more of nutritional supplements antioxidants comprise an isolated and purified lipophilic oxygen-radical quencher and an isolated and purified lipophilic oxygen-radical quencher, wherein the lipophobic and the lipophilic oxygen-radical quencher combined have an oxygen-radical quencher value of greater than 6,000 $\mu$Mol Trolox Equivalents (TE)/gram, and wherein the long-chain polysaccharides that comprise the time releasing agent release the nutritional supplement after the dietary supplement is digested. It was found that using identical conditions: capsule, weights, etc., using indicator nutrients, e.g., an anti-oxidant, the present invention allowed for the dissolution over time of the nutrient when compressed with long-chain polysaccharides. Examples of long-chain polysaccharides, which may also be part of the nutritional supplementation regimen, may have monomer subunits having length of, e.g., 2 to about 50,000 saccharide monomers and may be compressed from between about 100, 500, 1000, 2000 or even 10,000 psi or greater. Unlike the majority of nutritional supplements in use today, which are released immediately, the present invention permits for a more natural release of the one or more anti-oxidants, vitamins, minerals, amino acids, nucleic acids, saccharides, mixtures and combinations thereof. Unlike food stuffs, the present invention can be used to supplement specific deficiencies in essential nutrients in a manner that is both scientific, but also more natural.

The supplement may be placed inside a capsule, a vegetable capsule or a hard gelatin capsule. When compressed at a higher pressure, e.g., greater than 5,000 psi, it was found that over 85% of the nutritional supplements are released from between about 1 to about 8 hours and in other formulations of over 85% of the nutritional supplements are released from between about 2 to about 6 hours. The long-chain polysaccharides many include monomers selected from galactose, galactosamine, glucosamine, glucose, mannose, acetylated-mannose, N-acetylneuraminic acid, fucose, N-acetylgalactosamine, N-acetylglucosamine, arabinose, arabinogalactan, galacturonic acid, glucuronic acid, iduronic acid, xylose and mixtures or combinations thereof. The supplement may be compressed by roller compaction at a pressure between about 2,000 to 10,000; 5,000 to 10,000; or greater than 10,000 psi. The one or more long-chain polysaccharides may have between about 0.1 to about 75 weight percent of, e.g., galactose, galactosamine, glucosamine, glucose, mannose, acetylated-mannose, N-acetylneuraminic acid, fucose, N-acetylgalactosamine, N-acetylglucosamine, arabinose, arabinogalactan, galacturonic acid, glucuronic acid, iduronic acid, xylose and mixtures or combinations thereof.

The supplement may include one or more long-chain polysaccharides selected from galactose, glucose, mannose, N-acetylneuraminic acid, fucose, N-acetylgalactosamine, N-acetylglucosamine and xylose, from, e.g., isolated and purified gum tragacanth, guar gum, grain flour, rice flour, sugar cane, beet sugar, potato, milk, agar, algin, locust bean gum, psyllium, karaya gum, seed gums, Larch tree extract, aloe vera extract, gum ghatti, starch, cellulose, degraded cellulose, fructose, high fructose corn syrup, pectin, chitin, acacia, gum arabic, alginic acid, carrageenan, dextran, xanthan gum, chondroitin sulfate, sucrose, acetylated polymannose, maltose, glucan, lentinan, mannan, levan, hemicellulose, inulin, fructan, lactose and mixtures or combinations thereof. In one specific example, the long-chain polysaccharides and/or the nutritionally effective amount of one or more saccharides includes between about 1 to about 10 weight percent of each of isolated and purified galactose, galactosamine, glucosamine, glucose, mannose, acetylated-mannose, N-acetylneuraminic acid, fucose, N-acetylgalactosamine, N-acetylglucosamine, arabinose, arabinogalactan, galacturonic acid, glucuronic acid, iduronic acid, xylose and mixtures or combinations thereof.

In another embodiment of the present disclosure, the modified-release dietary supplement includes one or more isolated and purified long-chain polysaccharides selected from isolated and purified gum tragacanth, guar gum, grain flour, rice flour, sugar cane, beet sugar, potato, milk, agar, algin, locust bean gum, psyllium, karaya gum, seed gums, Larch tree extract, aloe vera extract, gum ghatti, starch, cellulose, degraded cellulose, fructose, high fructose corn syrup, pectin, chitin, acacia, gum arabic, alginic acid, carrageenan, dextran, xanthan gum, chondroitin sulfate, sucrose, acetylated polymannose, maltose, glucan, lentinan, mannan, levan, hemicellulose, inulin, fructan, lactose and mixtures or combinations thereof; and one or more nutritional supplements selected from anti-oxidants, vitamins, minerals, amino acids, nucleic acids, saccharides, mixtures and combinations thereof, wherein the long-chain polysaccharides and the nutritional supplements are roller compacted at a pressure greater that 2,000 psi.

Another modified-release dietary supplement of the present disclosure includes a nutritionally effective amount of one or more isolated and purified long-chain polysaccharides, one or more lipophilic anti-oxidant, and one or more lipophobic anti-oxidant, wherein the supplement is roller compacted at a pressure greater that 5,000 psi.

The present invention also includes a method of making a modified-release dietary supplement by mixing one or more saccharides comprising one or more long-chain polysaccharides and a nutritionally effective amount of one or more nutritional supplements selected from anti-oxidants, vitamins, minerals, amino acids, nucleic acids, herbal extracts, mixtures and combinations thereof, wherein the long-chain polysaccharides comprise both a time releasing agent for bioactive molecules and a portion of the dietary supplement, wherein the one or more nutritional supplements comprise anti-oxidants that comprise an isolated and purified lipophobic oxygen-radical quencher and an isolated and purified lipophilic oxygen-radical quencher, wherein the lipophobic and the lipophilic oxygen-radical quencher combined have an oxygen-radical quencher value of greater than 6,000 μMol Trolox Equivalents (TE)/gram; and which is compressed at a pressure greater than 2,000, psi, wherein the long-chain polysaccharides that comprise the time releasing agent release the nutritional supplement after the dietary supplement is digested. The method may be used to make a modified-release dietary supplement that includes a nutritionally effective amount of one or more isolated and purified polysaccharides from gum tragacanth, guar gum, grain flour, rice flour, sugar cane, beet sugar, potato, milk, agar, algin, locust bean gum, psyllium, karaya gum, seed gums, Larch tree extract, aloe vera extract, gum ghatti, starch, cellulose, degraded cellulose, fructose, high fructose corn syrup, pectin, chitin, acacia, gum arabic, alginic acid, carrageenan, dextran, xanthan gum, chondroitin sulfate, sucrose, acetylated polymannose, maltose, glucan, lentinan, mannan, levan, hemicellulose, inulin, fructan, lactose and mixtures or combinations thereof and one or more nutritional supplements selected from anti-oxidants, vitamins, minerals, amino acids, nucleic acids, mixtures and combinations thereof, wherein the polysaccharides and the nutritional supplements are compressed to a pressure greater that 2,000 psi.

The present disclosure also includes antioxidant sensors and methods that measure directly total sample oxidation levels and the effect of anti-oxidants on total sample oxidation state. The invention also includes compositions and methods that are useful for providing effective amounts of anti-oxidants to an individual for optimal health. The apparatus and method disclosed herein detect the total anti-oxidant capacity of a sample, concurrently and directly in real-time. The present inventors recognized that the art has artificially created two mutually exclusive categories of anti-oxidants (lipophilic and lipophobic) and has measured them separately. Furthermore, the art has also generally measured the existence of the radicals indirectly, that is, using a detectable reporter molecule.

The present disclosure overcomes the limitations in prior art detectors and methods by using a rapid, inexpensive and direct detection system. To address these limitations, the present inventors developed the Oxygen Radical Absorbance Capacity-Oxygen (ORAC(o)) apparatus and method disclosed herein. Using the ORAC(o) apparatus, the present inventors were able to measure, for the first time, the effect of both the effect of both lipophilic and lipophobic anti-oxidants on dissolved oxygen levels, concurrently and in real-time. Using the ORAC(o) assay, the inventors were also able to develop a synergistic anti-oxidant composition, which may be used alone or in combination with one or more anti-oxidant potentiators.

More particularly, the present disclosure includes an apparatus for detecting directly the antioxidant activity of both lipophilic and lipophobic anti-oxidants that includes a dissolved oxygen sensor in fluid communication with a sample and an oxygen radical sensitive molecule in a solvent/water/surfactant mixture; wherein the oxygen radical sensitive sensor concurrently detects both lipophilic and lipophobic anti-oxidants in the solvent/water/surfactant mixture. The oxygen radical sensitive molecule may be molecules that will react with oxygen, e.g., molecules with conjugated double bonds; or Nitrogen or Sulfur containing compounds. Examples of oxygen radical sensitive molecule, e.g., fluorescein, $\beta$-Phycoerythrin ($\beta$-PE), glutathione-S-transferase, linoleic acid or combinations thereof. The oxygen radical level is determined directly using a dissolved oxygen meter or sensor in a solvent/water/surfactant mixture. The level of dissolved oxygen may be determined using an oxygen sensor, e.g., an electrochemical, a chemiluminescent, a surface plasmon resonance, an infrared, a capacitance coupled, a dye-coupled fiber optic or a hyperspectral oxygen sensor. The dissolved oxygen meter or sensor may be placed in-line for high-throughput analysis, may be a single sample detector and/or be adapted for office or even home use. The solvent may be an organic solvent, e.g., acetone. The surfactant may be a detergent, e.g., a non-ionic detergent such as Tween-20. The solvent in the solvent/water/surfactant mixture is generally at least about 10 to 90 percent in volume of the solvent/water/surfactant mixture, e.g., 33%. The water in the solvent/water/surfactant mixture is generally at least about 10 to 90 percent in volume of the solvent/water/surfactant mixture, e.g., 33 to 67%. The surfactant (or detergent) in the solvent/water/surfactant mixture is at least about 0.1 to 10 percent in volume of the solvent/water/surfactant mixture and may be stored dissolved in water. In one specific example, the solvent/water/surfactant ratio is about 1:1:1.

The apparatus may further include one or more processors, e.g., a computer that may: control the detector, capture data, store data, perform calculations based on the data and/or a database of information, and/or display the data or summaries of the data in the form of tables, graphs, charts and the like. The processor/computer may also be connected and even control a fluidic system that is in fluid communication with the oxygen sensor and the solvent/water/detergent mixture. The disclosed apparatus measures an area under the curve that relates the relative disappearance of oxygen that results from the activity of the sample being tested for anti-oxidant capacity to the relative disappearance of oxygen observed as a result of the activity of a known standard. Using the present invention, and the methods described herein, the level of dissolved oxygen is measured directly in the solution that include both lipophilic and lipophobic anti-oxidants, concurrently. An examples of the formula for calculating the AUC may be:

$$ORAC(o) = \frac{\dfrac{AUC_{SMP} - AUC_{BLNK}}{AUC_{TRLX} - AUC_{BLNK}} \times 1000 \ (mg/gr) \times [TRLX \ (\mu mol/ml)]}{[SMP \ (mg/ml)]}$$

wherein $AUC_{SMP}$ is the area under the curve value of the sample;

wherein $AUC_{BLNK}$ is the area under the curve value of the blank;

wherein $AUC_{TRLX}$ is the area under the curve value for Trolox®; and

wherein SMP is the sample.

Disclosed is a method of determining directly the anti-oxidant activity including the steps of: determining the dissolved oxygen level in a test solution dissolved in a solvent/water/surfactant mixture in the presence of one or more anti-oxidants and an oxygen radical target, wherein both aqueous and lipid soluble anti-oxidant activity is measured with an oxygen detector. The dissolved oxygen radical level may be determined using an oxygen detector, e.g., electrochemical, chemiluminescent, surface plasmon resonance, capacitance coupled, a dye-coupled fiber optic or a hyperspectral oxygen sensor. The anti-oxidant activity may be measured at about 37 degrees Centigrade. Examples of radical intiators include, e.g., 2,2'-azobis[2-(5-methyl-2-imidazolin-2-yl)propane]dihydrochloride, 2,2' azobis (2-amidinopropane)dihydrochloride (AAPH), 2,2'-azobis(2-amidinopropane)[2-(N-stearyl)amidinopropane] dihydrochloride (SA-1), 2,2'-azo(2-(2-imidiazolin-2-yl)-propane)-[2-[2-(4-n-octyl)imidazolin-2-yl]-propane] dihydrochloride (C-8), 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane), salts, mixtures or equivalents thereof. The detector may even be disposable.

The present invention also includes a dietary supplement that includes any isolated and purified lipophobic anti-oxidant and any isolated and purified lipophilic anti-oxidant, wherein the lipophobic and the lipophilic antioxidants combined have a dissolved oxygen value of greater than 6,000 $\mu$Mol Trolox® Equivalents (TE)/gram. The skilled will recognize that the value may also be expressed as liquid equivalents, e.g., 6,000 $\mu$Mol Trolox® Equivalents (TE)/milliliter. The lipophobic and the lipophilic anti-oxidant are time-released and may include one or more vitamin E selected from alpha, beta, delta, epsilon, gamma, zeta, eta, xi1, xi2, and sigma tocopherols, and alpha, beta, delta and gamma tocotrienols, analogs thereof, pharmaceutically acceptable salts thereof, and combinations thereof. Examples of lipophilic anti-oxidants include quercetin, kaempferol, myricetin, apigenin and derivates, analogs, pharmaceutically acceptable salts thereof, and combinations thereof.

The dietary supplement that includes any isolated and purified lipophobic anti-oxidant and any isolated and purified lipophilic anti-oxidant, may also include two or more essential saccharide, e.g., galactose, galactosamine, glucosamine, glucose, mannose, acetylated-mannose, N-acetylneuraminic acid, fucose, N-acetylgalactosamine, N-acetylglucosamine and/or xylose. In one embodiment, the supplement also includes source of vitamin C, e.g., a plant source with a high level of natural, bioavailable vitamin C such as an Australian bush plum (*Terminalia ferdinandiana*). In another specific embodiment, the Vitamin C is a potentiator of anti-oxidant activity from a plant source of vitamin C such as wild-Australian bush plum (*Terminalia ferdinandiana*), which has a higher percentage of Vitamin C than farm grown bush plum. The supplement may also include one or more pro-biotics, e.g., *Lactobacillus sp.* and *Bifidobacterium sp.* The supplement may be compressed to provide a generally oxygen impermeable surface, e.g., a roller-compressed particle, a capsule, a tablet, a mini-tab, a caplet, an effervescent tablet or combinations thereof.

As a point of comparison with the ORAC(o) apparatus and method described hereinabove, the isolated and purified lipophilic and lipophobic anti-oxidants, will have an anti-oxidant ORAC(fl-lipo) value greater than 7000 $\mu$Mol Trolox® Equivalents (TE)/gram. The present invention was used in an open label study to measure the change in anti-oxidant levels in each individual taking an anti-oxidant/glyconutrient blend that included the anti-oxidants of the present invention in their diets. When provided to a patient, the lipophobic and the lipophilic antioxidants provided an average increase of over 13% as measured by ORAC($\beta$-PE) from the cumulative patient population's average baseline antioxidant level. The present invention also includes a number of compositions. The compositions disclosed herein are based on the recognition that dietary supplements available currently fail to combine lipophilic and lipophobic anti-oxidants with measurable activities above those expected from the individual components. Using the disclosed apparatus and the inventive method, the present inventors were able to develop synergistic combinations of not only lipophilic and lipophobic anti-oxidants, but also add potentiators of the anti-oxidant activity.

Flavonoids like quercetin have recently been shown to increase the transcription of the heavy subunit of the rate-limiting enzyme in glutathione synthesis, gamma-glutamylcysteine synthetase, through the gene's antioxidant responsive elements. The increased transcription is subsequently translated in increased intracellular levels of reduced (active) glutathione in tissue culture cells. Quercetin is a major component of red wine, grape skin, and onions. Studies of quercetin from red wine, grape skin, and onions suggest beneficial effects upon health. Quercetin has been shown to be well absorbed by humans. One study showed that all ingested quercetin was metabolized two hours following a meal (European Research on Functional Effects of Dietary Antioxidants, September 25-28, 2002, Cambridge, UK). Upon finding substantially decreased LDL oxidation in subjects consuming moderate amounts of red wine, researchers asserted that the protective effects could very likely be attributed to the activity of quercetin and its metabolites. Quercetin has also been shown to enhance the stability of erythrocytes.

The present inventors recognized that due to the numerous and diverse factors influencing oxidative stress, anti-oxidant supplementation would need to be tailored to an individual's need and chemistry. Furthermore, it was recognized that a combination of tocopherols was required to meet the differing needs of individuals. The combination of tocopherols is necessary because some antioxidants are "selected" by the body. For example, the predominant form of vitamin E in the North American diet is gamma-tocopherol, which is commonly found in vegetable oils as well as products derived form soybeans and corn. However, the body predominantly retains alpha-tocopherol. A specific method, leaning on alpha-tocopherol transfer protein has been found to regulate the concentration of alpha-tocopherol in the body. Unlike anti-oxidant compositions in the prior art, the present invention uses mixed tocopherols to provide the body with many forms of vitamin E, allowing for the selection, retention and use of the optimal amounts of each form. Furthermore, the synergistic combination of quercetin and mixed tocopherols provides the body with a wide array of anti-oxidant nutrients optimal selection, which may differ based upon an individual's needs.

The present inventors sought to maximize further the activity of the synergistic quercetin and mixed tocopherol combination by adding compounds that help potentiate the activity of these anti-oxidants. One such potentiator is Vitamin C. Vitamin C has significant antioxidant activity attributed to it as well as several non-antioxidant nutritive functions. Many attribute pro-oxidant properties to vitamin C, especially in the presence of transition metals. The pro-oxidant properties of vitamin C may not be entirely destructive. However, other researchers assert that they found that vitamin C behaves as an antioxidant, even in the presence of unbound metals. Other potentiators of anti-oxidant activity include natural extracts, such as grape seed extract and green tea extract. In one study, green tea exhibited potent antimutagenic activity in vitro and inhibited the development of carcinogen-induced preneoplastic lesions in the rat colon. Green tea also significantly inhibited the formation of intestinal polyps. Therefore, the present inventors combined not only a synergistic combination of purified and isolated anti-oxidants from natural sources, such as quercetin and mixed tocopherols, but further added potentiators that increased the detectable anti-oxidant activity of these agents.

More particularly, the compositions a dietary supplement that includes a nutritionally effective amount of two or more essential saccharides; an isolated and purified lipophobic oxygen-radical quencher; and an isolated and purified lipophilic oxygen-radical quencher, wherein the lipophobic and the lipophilic oxygen-radical quenchers combined have an oxygen-radical quencher value of greater than 6,000 $\mu$Mol Trolox® Equivalents (TE)/gram. In one assay, the lipophobic and the lipophilic oxygen-radical quencher when provided to a patient provide an average increase of over 13% as measured by ORAC(fl-lipo) from the patient population's baseline antioxidant level. The lipophobic and the lipophilic oxygen-radical quencher are packaged for extended-release, and may include one or more of the following vitamin E molecules: alpha, beta, delta, epsilon, gamma, zeta, eta, xi1, xi2, and sigma tocopherols, and alpha, beta, delta and gamma tocotrienols, analogs thereof, pharmaceutically acceptable salts thereof, and combinations thereof. The lipophilic oxygen-radical quencher may include one or more of the following: flavonols, quercetin, kaempferol, myricetin, apigenin and derivates, analogs, pharmaceutically acceptable salts thereof, and combinations thereof. The supplement may further include two or more saccharides selected from the group consisting of galactose, glucose, mannose, N-acetylneuraminic acid, fucose, N-acetylgalactosamine, N-acetylglucosamine and xylose, derivates, analogs, pharmaceutically acceptable salts thereof, and combinations thereof.

BRIEF DESCRIPTION OF THE DRAWINGS

For a more complete understanding of the features and advantages of the present invention, reference is now made to the detailed description of the invention along with the accompanying figures and in which:

FIG. 1 depictions an ORAC(o) direct anti-oxidant apparatus;

FIG. 2 is a flowchart of the ORAC(o) method of the present invention;

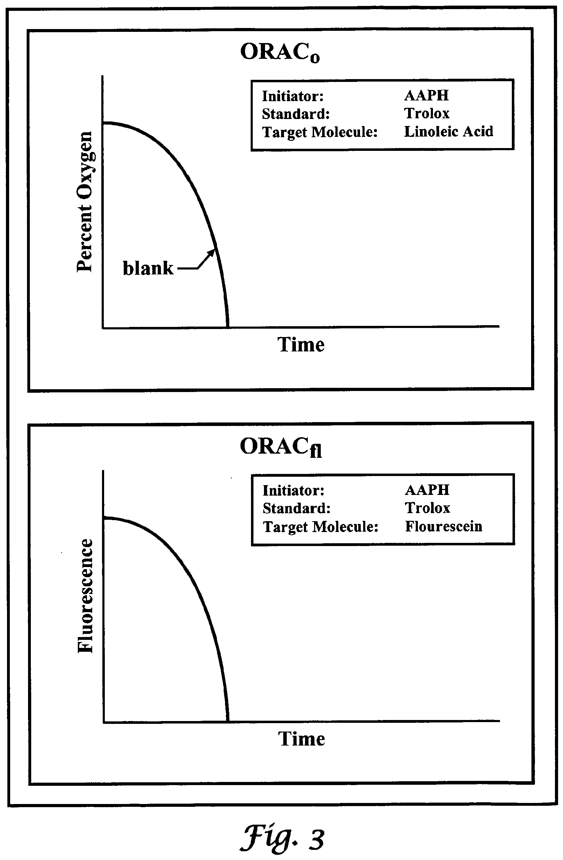

FIG. 3 are two graphs that compare the blank solution in an ORAC(o) (percent oxygen) with the ORAC(fl) (fluorescence) measurements using AAPH an initiator, Trolox® as the standard and linoleic acid or fluorescein, respectively as the oxygen radical targets;

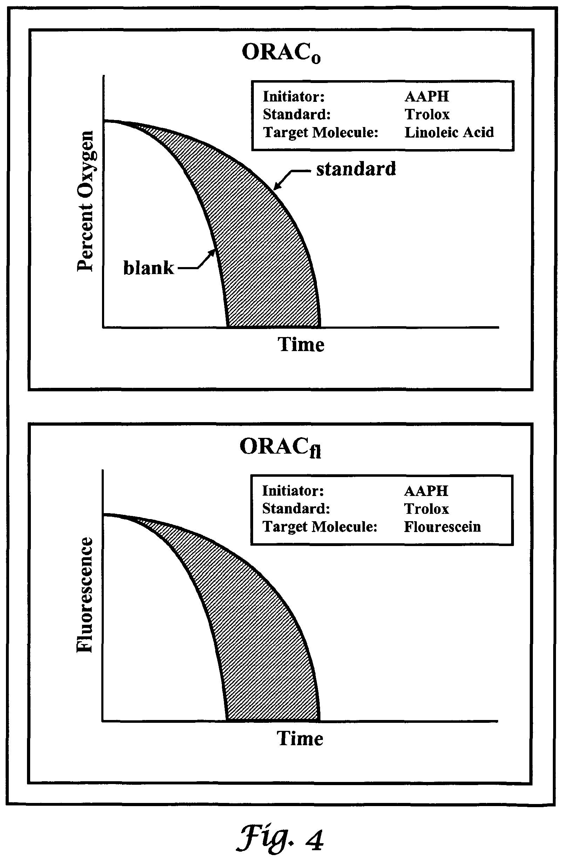

FIG. 4 are two graphs that compare the anti-oxidant standard Trolox® in an ORAC(o) (percent oxygen) with the ORAC(fl) (fluorescence) measurements using AAPH an initiator, Trolox® as the standard and linoleic acid or fluorescein, respectively as the oxygen radical targets;

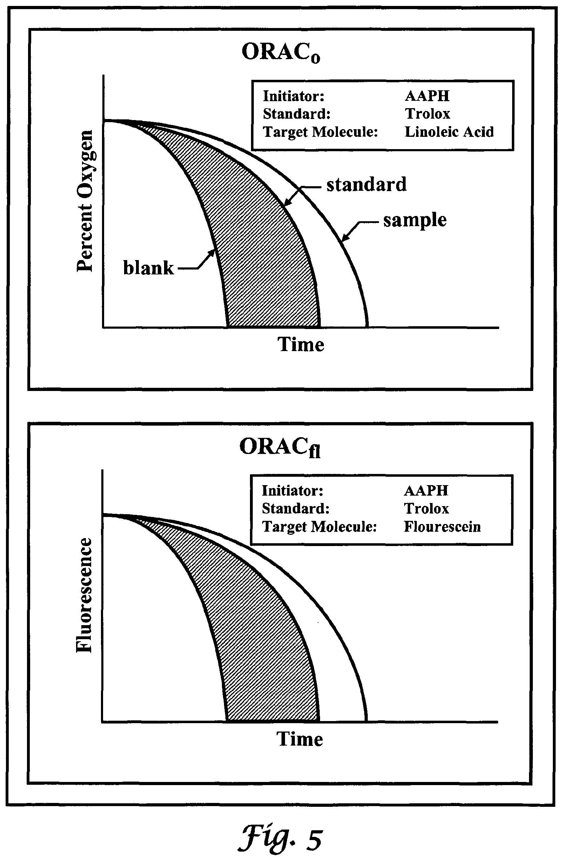

FIG. 5 are two graphs that compare the blank solution, the standard and the sample, in an ORAC(o) (percent oxygen) with the ORAC(fl) (fluorescence) measurements using AAPH as an initiator, Trolox® as the standard and linoleic acid or fluorescein, respectively as the oxygen radical targets;

FIG. 6 is a graph that shows an antioxidant effect of the combination of quercetin (Q) at 5 $\mu$g/mL and mixed tocopherols (MT) at 5 $\mu$g/mL as compared to each ingredient separately at a concentration of 10 $\mu$g/mL as measured by the ORAC(o) method of the present invention;

FIG. 7 is a graph that shows an antioxidant effect of the combination of quercetin (Q) at 5 $\mu$g/mL and mixed tocopherols (MT) at 5 $\mu$g/mL, and Trolox® as a control measured by the ORAC(o) method of the present invention;

FIG. 8 is a graph of the Area Under the Curve (AUC) results from titrated ratio of quercetin and mixed tocopherols using the ORAC(o) method to measure antioxidant capacity;

FIG. 9 is another graph of AUC results from titrated ratio of quercetin and mixed tocopherols using the ORAC(o) method to measure antioxidant capacity that demonstrated the expected results (line) and the extent of synergy detected above the line as compared to the expected results from titration of the quercetin and mixed tocopherols;

FIG. 10 is a graph that shows the results from ORAC(o) assays for varying ratios of grape skin extract and green tea extract in the presence of 49.18% quercetin, 32.79% mixed tocopherols, and 1.64% bush plum. The optimal ratio of grape skin extract to green tea extract is 60/40 to 80/20;

FIG. 11 is a graph that shows ORAC(fl) results of the combination of quercetin (Q) at 5 μg/mL and mixed tocopherols (MT) at 5 μg/mL as compared to each ingredient separately at a concentration of 10 μg/mL.

FIG. 12 is a graph of an ORAC(fl) assay measuring a titration of quercetin versus α-tocopherol dissolved in acetone:water;

FIG. 13 is a graph of an ORAC(fl) assay measuring the anti-oxidant activity measured for a fixed ratio of quercetin: α-tocopherol ratio dissolved in a solvent:water: detergent mixture;

FIG. 14 is a graph of an ORAC(fl) assay measuring the anti-oxidant activity measured for a fixed ratio of quercetin:α-tocopherol ratio dissolved in two different rations of solvent:water to detergent mixtures;

FIG. 15 is a graph showing the ORAC(o) values obtained with different ratios of quercetin and mixed tocopherols;

FIG. 16 is a graph showing the ORAC(o) values for different ration of grape seed extract and green tea extract;

FIG. 17 is a graph that shows the ORAC(o) values of the combination of the maximum quercetin:mixed tocopherol and the grape seed extract and green tea extract ratios;

FIG. 18 shows the three modules selected for initial investigation; and

FIGS. 19 and 20 are graphs that show the relative release profiles of quercetin by HPLC and UV/Vis, respectively.

DETAILED DESCRIPTION OF THE INVENTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of this invention, a number of terms are defined below. Terms defined herein have meanings as commonly understood by a person of ordinary skill in the areas relevant to the present invention. Terms such as "a", "an" and "the" are not intended to refer to only a singular entity, but include the general class of which a specific example may be used for illustration. The terminology herein is used to describe specific embodiments of the invention, but their usage does not delimit the invention, except as outlined in the claims.

The present invention is based, in part, on the recognition that the art has artificially created two mutually exclusive categories of anti-oxidants and has measured them separately. Furthermore, the art has also generally measured the existence of the radicals indirectly, that is, using a reporter molecule. The present invention provides a method that not only detects the total anti-oxidant capacity of a sample, concurrently, but does so directly.

As used herein, the term "essential saccharides," is used to define the monosaccharides commonly found in the oligosaccharide chains of cellular glycoproteins and which may not be readily available through diet or biochemical manufacture in the human body (see, e.g., Harper's Biochemistry (Murray et al., 1996)(listing eight) and Principles of Biochemistry, Vol II (Zubay, et al., 1995)(listing eleven). While over 200 monosaccharides have been found in nature, these eleven are believed to be important toward maintaining good health in mammals: galactose, glucose, mannose, N-acetylneuraminic acid, fucose, N-acetylgalactosamine, N-acetylglucosamine, xylose, iduronic acid, arabinose and glucuronic acid. The structures of these carbohydrates are well-known (see, e.g., Stryer's Biochemistry (Stryer, 1995) and the Merck Index, 12th Edition, 1996).

As used herein the terms, "long-chain saccharides" and "long-chain polysaccharides" are used to describe those chains of saccharides that have two or more saccharides that are capable of intrachain hydrogen binding. For example, U.S. Patent No. 4,735,935 teaches the methods of isolation of long-chain polysaccharides from Aloe vera, in which the precipitated, lyophilized long-chain polysaccarides have from 2 to about 50,000 monomers per chain. The long-chain polysaccharides can be isolated from a variety of plant and animal sources, as taught and disclosed herein. Isolating and purifying the long-chain polysaccharides of the present invention, and even isolating specific chain lengths or combinations thereof may be obtained by, e.g., hydrolyzing the long-chain polysaccharides, bulk isolation of specific lengths of longchain polysaccharides, polymerizing longer long-chain polysaccharides, selecting combinations of shorter and longer long-chain polysaccharides, separating the long-chain polysaccharides by, e.g., electroporation, FPLC, HPLC, size-exclusion, size-exclusion chromatography, precipitation and the like.

As used herein, the term "modified release" is used herein is used to describe a release profile to effect delivery of a nutritionally effective amount of a nutrient over an extended period of time, defined herein as being between about 60 minutes and about 2, 4, 6, 8 or more hours using the formulation of the present invention. Modified release may also be defined functionally as the release of over 80 to 90 percent (%) of the nutrient after about 60 minutes and about 2, 4, 6 or even 8 hours. Modified release may also be evaluated by making the nutrient available to the patient or subject regardless of uptake, as some actives may never be absorbed by the animal. Various modified release dosage forms may be designed readily by one of skill in art as disclosed herein to achieve delivery to both the small and large intestines, to only the small intestine, or to only the large intestine, depending upon the choice of coating materials and/or coating thickness. Examples of modifications that can be made to the long-chain polysaccharides include, e.g., changing the types or composition of saccharides in the long-chain polysaccharides, chemically modifying (organically or chemically) the side chains of the saccharides (e.g., acetylation), hydrolyzing the long-chain polysaccharides, sizing the long-chain polysaccharides, polymerizing longer long-chain polysaccharides, selecting combinations of shorter and longer long-chain polysaccharides, separating the long-chain polysaccharides by, e.g., electroporation, FPLC, HPLC, size-exclusion, size-exclusion chromatography, precipitation and the like. Extended release formulations may be prepared and delivered so that release is accomplished at some generally predictable location in the lower intestinal tract more distal to that which would have been accomplished if there had been no modified release alterations.

The present invention may be used alone or in combination with one or more method, techniques, mechanical, chemical and other modification, encapsulation, packaging and the like for delaying the release of the nutrient, e.g., a capsule, a gel cap or even a coating. Examples of capsules include animal, vegetable, polymeric, mixtures and combinations thereof. The coating (type, thickness, etc) may be applied to a sufficient thickness such that part or the entire coating does not dissolve in the gastrointestinal fluids at pH below about 5, but does dissolve at pH about 5 and above.

As used herein the term "nutritionally effective amount" is used to define the amount that will provide a beneficial nutritional effect or response in a mammal. For example, as nutritional response to vitamin- and mineral-containing dietary supplements varies from mammal to mammal, it should be understood that nutritionally effective amounts of the vitamins and minerals will vary, respectively. Likewise, the lack of an essential amino acid, vitamin-C, iron, iodine, vitamins, minerals, carbohydrates, lipids and the like are known to affect physiological and cellular functions. A nutritionally effective amount of the anti-oxidants and saccharides disclosed herein serve to preserve and/or elevate the levels of these critical nutrients in the diet of, e.g., a human that seeks to maintain or augment their diet for these nutritional supplements. Thus, while one mammal may require a particular profile of vitamins and minerals present in defined amounts, another mammal may require the same particular profile of vitamins and minerals present in different defined amounts.

As used herein, the "antioxidant" refers to any molecule that delays or prevents the oxidation of an oxidizable target molecule. Antioxidants act by: scavenging biologically important reactive free radicals or other reactive oxygen species (e.g., $O_2^-$, $H_2O_2$, HOCl, ferryl, peroxyl, peroxynitrite, and alkoxyl); preventing oxygen radical formation; or catalytically converting the free radical or other reactive oxygen species to a less reactive species. Antioxidants are generally divided into two classes: (1) lipid (lipophilic or hydrophobic) antioxidants; and (2) aqueous (lipophobic or hydrophilic) antioxidants. Examples of lipid antioxidants include, but are not limited to, carotenoids (e.g., lutein, zeaxanthin, β-cryptoxanthin, lycopene, α-carotene, and β-carotene), which are located in the core lipid compartment, and tocopherols (e.g., vitamin E, α-tocopherol, γ-tocopherol, and δ-tocopherol), which are located in the interface of the lipid compartment, and retinoids (e.g., vitamin A, retinol, and retinyl palmitate) and fat-soluble polyphenols, e.g., quercetin. Examples of aqueous antioxidants include, but are not limited to, ascorbic acid and its oxidized form, "dehydroascorbic acid," uric acid and its oxidized form "allantoin," bilirubin, albumin and vitamin C and water-soluble polyphenols such as catechins, which have high affinity to the phospholipid membranes, isoflavones and procyanidins.

A commonly used method for detecting the relative levels of oxygen radicals and anti-oxidant capacity is the Oxygen Radical Absorbance Capacity (ORAC) assay. In known ORAC-type assays, the anti-oxidant value is measured indirectly by measuring the effect of an oxygen radical on, e.g., a fluorescent or other detectable molecule, that may of may not be a good target for oxidation by the particular oxygen radical. Generally, when antioxidants are added to a test sample, a detectable decrease in the amount of a free radical, such as superoxide, or a non-radical reactive oxygen species, such as hydrogen peroxide, may be seen in the sample, compared with a sample untreated with the antioxidant (i.e., control sample). However, these indirect methods monitor the change in antioxidant status via an intermediary (e.g., fluorescein, β-phytoerythrin (β-PE), etc.) measured with the assumption that the effect of the radical on the intermediary is a true reflection of the relative level of oxidants and anti-oxidants. Controls for these assays are known concentrations of oxygen radical generators and known antioxidants that are measured and used as standards for the samples.

As used herein, the term "free radical" refers to molecules containing at least one unpaired electron. Most molecules contain even numbers of electrons, and their covalent bonds normally include shared electron pairs. Cleavage of such bonds produces two separate free radicals, each with an unpaired electron (in addition to any paired electrons). Free radicals may be electrically charged or neutral, are highly reactive and usually short-lived. Free radicals combine with one another or with atoms that have unpaired electrons. In reactions with intact molecules, free radicals try to complete their own electronic structure, generating new radicals, which go on to react with other molecules creating a chain reaction. Free radical chain reactions are particularly important in decomposition of substances at high temperatures and in polymerization. In the body, oxidized free radicals are responsible for damage tissues. Heat, ultraviolet light, and ionizing radiation all generate free radicals. Free radicals are generated as a secondary effect of oxidative metabolism. An excess of free radicals can overwhelm the natural protective enzymes such as superoxide dismutase, catalase, and peroxidase. Free radicals such as hydrogen peroxide ($H_2O_2$), hydroxyl radical (HO·), singlet oxygen $C^1O_2$), superoxide anion radical ($O·_2^-$), nitric oxide radical (NO·), peroxyl radical (ROO·), peroxynitrite (ONOO$^-$) can be in either the lipid or aqueous compartments. Antioxidant nutrients (e.g., vitamins C and E, selenium, polyphenols) may reduce these effects.

As used herein, the phrase "lipid compartment" refers to compounds that have cyclic or acyclic long-chain aliphatic hydrocarbons and their derivatives, such as fatty acids, alcohols, amines, amino alcohols and aldehydes. For example, common lipids include fatty acids, fats, phospholipids, steroids, eicosanoids, waxes and fat-soluble vitamins. Some lipids may be generally classified into two groups, the simple lipids and the complex lipids, e.g., triglycerides or fats and oils, fatty acid esters of glycerol, waxes, fatty acid esters of long-chain alcohols and steroids such as cholesterol and ergosterol. Complex lipids include, e.g., phosphatides or phospholipids (phosphorous containing lipids), glycolipids (carbohydrate containing lipids), and sphingolipids (sphingosine containing lipids).

As used herein, the term "lipid" includes fats or fat-like substances. The term is descriptive rather than a chemical name such as protein or carbohydrate. Lipids include true fats (i.e., esters of fatty acids and glycerol), lipoids (i.e., phospholipids, cerebrosides, waxes) and sterols (i.e., cholesterol, ergostrol). Lipids can be a target of oxidation through mechanisms, such as autoxidation. As used herein, the term "fatty acid" refers to a group of, e.g., negatively charged, generally linear hydrocarbon chains. The hydrocarbon chains of fatty acids vary in length and oxidation states. Generally, fatty acids have a negatively charged portion (e.g., at the carboxyl end), and a "tail" portion, which determines the water solubility and amphipathic characteristics of the fatty acid. For example, fatty acids are components of the phospholipids that include biological membranes, as fats, which are used to store energy inside cells, or for transporting fat in the bloodstream. As used herein, the term "phospholipid" refers to any of the class of esters of phosphoric acid that include at least one of the following side-groups: a fatty acid, an alcohol and a nitrogenous base.

As used herein, the term "fat" or "fats" refers to any of the glyceryl esters of fatty acids, e.g., the monoacylglycerol, diacylglycerol and triacylglycerol forms of fatty acids. Triglycerides refer to those molecules that are neutrally charged and entirely hydrophobic, i.e., reduced molecules. Monoacylglycerides and diacylglycerides are metabolic intermediates in phospholipid synthesis, while triglycerides form the fat molecules that are used to store chemical energy in a water free, compact state. As used herein, the term "fat-soluble vitamins" refers to, e.g., common fat-soluble vitamins include vitamin (A) (retinol), Vitamin D (e.g., vitamin D3 (cholecalciferol)), Vitamin E, Vitamin K and the like.

As used herein, the phrase "lipid antioxidant activity" or "lipid antioxidant capacity" are used interchangeably and refer to the measurement of antioxidant ability arising from the lipid compartment of a sample. As used herein, the phrase "aqueous antioxidant activity" or "aqueous antioxidant capacity" are used interchangeably and refer to the measurement of antioxidant ability arising from the aqueous compartment of a sample. As used herein, the phrase "total antioxidant activity" or "total antioxidant capacity" are used interchangeably and refer to the measurement of antioxidant ability arising from both the lipid and aqueous portions of a sample.

As used herein, the phrase "aqueous compartment" refers the portion of a fluid sample that does not interact with the lipid compartment. The aqueous compartment includes biologic fluid samples such as blood, plasma, serum, feces, cerebral spinal fluid, amniotic fluid, interstitial fluid, lymphatic fluid and synovial fluid. For example, the aqueous compartment of a fluid sample such as serum may include not only the liquid portion that remains after blood has been allowed to clot and is centrifuged to remove the blood cells and clotting elements, but also other compounds such as: proteins, e.g., albumin and globulins; antibodies; enzymes; small amounts of nutritive organic materials, such as amino acids and glucose; inorganic substances such as sodium, chloride, sulfates, phosphates, calcium, potassium, bicarbonate, magnesium, iodine, zinc, and iron; small amounts of waste products, such as urea, uric acid, xanthine, creatinine, creatine, bile pigments and ammonia; and trace amounts of gases such as oxygen and carbon dioxide. The fluid sample may also be a non-biological sample, for example, chemical formulations, synthetic compositions, or food products and cosmetic products.

As used herein, the term "sample" refers to a liquid or fluid biological sample, or a solid biological sample in which free radicals can be generated using a free radical generator, (e.g., a lipophilic free radical generator or an hydrophilic free radical generator) and can be detected using the (ORAC(o)) detector and method of the present invention. Biological samples include, e.g., blood, plasma, serum, cerebral spinal fluid, urine, amniotic fluid, interstital fluid, and synovial fluid. Solid biological samples include, e.g., a tissue, cells, tissue culture, fixed cells, cell supernatants, or even portions (or extracts) of tissue or cell matter. The term sample also includes non-biological samples such as a chemical solution, synthetic composition, and food. As used herein, the terms "relative ORAC(o)" and "ORAC(o)" refer to the same value, which is measured by equivalence to micro-moles of Trolox® per gram or milliliter. A negative value ORAC(o) reflects less radical quenching activity than obtained with a blank which indicates that a composition is a pro-oxidant, i.e., an agent that promotes oxidation, rather than acting as an antioxidant.

As used herein, the phrases "radical generator" or "radical initiator" are used interchangeably and refer to an agent, compound or molecule that produces free radicals. The radical generator is capable of producing free radicals at a measured level, for example, at a level at which antioxidants or oxidizable indicators can interact with the free radicals to produce a measurable or detectable output. Examples of radical generators include, e.g., azo radical generators, which are compounds that produce a flux of free radicals at a known constant rate. Examples of azo radical generators include, e.g., 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane), 2,2'-azobis[2-(5-methyl-2-imidazolin-2 yl)propane]dihydrochloride, iron, ascorbic acid and metal ions.

As used herein, the "subject" refers to any living organism. The term subject includes, e.g., fish, mammals, reptiles, birds, insects and the like. Specific examples include: humans, non-human primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

As used herein, the phrase "free radical associated disorder" refers to a pathological condition of from the production of or exposure to free radicals. As used herein, the term "free radical associated disorder" includes pathological states where damage from free radicals contributes to the pathology of the disease state, or wherein administration of a free radical inhibitor (e.g., desferrioxamine), scavenger (e.g., tocopherol, glutathione), or catalyst (e.g., SOD, catalase) are shown to produce a detectable benefit by decreasing symptoms, increasing survival, or providing other detectable clinical benefits in protecting or preventing the pathological state. Examples of free radical disorders include, but are not limited to, ischemic reperfusion injury, inflammatory diseases, systemic lupus erythematosis, myocardial infarction, stroke, traumatic hemorrhage, spinal cord trauma, Crohn's disease, autoimmune diseases (e.g., rheumatoid arthritis, diabetes), cataract formation, age-related macular degeneration, Alzheimer's disease, uveitis, emphysema, gastric ulcers, oxygen toxicity, neoplasia, undesired cell apoptosis and radiation sickness.

As used herein, the term "oxidative stress" refers to the level of damage produced by oxygen free radicals in a subject. The level of damage depends on how fast reactive oxygen species are created and then inactivated by antioxidants as well as the location and speed of repair. As used herein, the term "deviation" or "deviate" as related to oxidative state and oxidative stress are used interchangeably and refer to a change in the antioxidant activity of a sample. The change in oxidative state can be an increase, decrease, elevation or depression of antioxidant activity from a known normal value. For example, an increase or decrease of antioxidant activity in the lipid compartment of a sample, the aqueous compartment of a sample, or in both the lipid and aqueous compartment of the sample.

As used herein, the term "pharmaceutically acceptable salt" of the nutrients is used to describe those salts that are, within the scope of sound medical judgment, suitable for use in, on or with the tissues of humans and lower animals without undue toxicity, irritation, allergic response and the like and are commensurate with a reasonable benefit/risk ratio. Pharmaceutically acceptable salts are well-known in the art (see e.g., S. M. Berge, et al., J. Pharmaceutical Sciences, 1977). Suitable salts may be prepared during the final isolation and purification of the compounds of the invention or separately by reacting a free base function with a suitable organic acid. Representative acid addition salts include, but are not limited to acetate, adipate, alginate, citrate, aspartate, benzoate, benzene sulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluene sulfonate and undecanoate. Examples of basic nitrogen-containing groups that are used as quaternizing agents include: lower alkyl halides (methyl, ethyl, propyl, and butyl chlorides, bromides and iodides); dialkyl sulfates (dimethyl, diethyl, dibutyl and diamyl sulfates); long-chain halides (decyl, lauryl, myristyl and stearyl chlorides, bromides and iodides); arylalkyl halides (benzyl and phenethyl bromides) and the like. Examples of acids that may be employed to form pharmaceutically acceptable acid addition salts include inorganic acids, e.g., hydrochloric acid, hydrobromic acid, sulphuric acid and phosphoric acid and such organic acids as oxalic acid, maleic acid, succinic acid and citric acid. Basic addition salts can also be prepared in situ during the final isolation and purification of anti-oxidant compounds disclosed herein with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

As used herein, the term "potentiate" refers to one or more agent that act directly or indirectly to increase or enhance the activity of the lipophobic and/or lipophilic anti-oxidants of the present invention. One such potentiator is Vitamin C, which may act to reactivate or recycle the anti-oxidants and may itself have significant antioxidant activity. The pro-oxidant properties of vitamin C have been observed in the presence of transition metals. Other potentiators of anti-oxidant activity include natural extracts, such as grape seed extract and green tea extract. In one study, green tea exhibited potent antimutagenic activity in vitro and inhibited the development of carcinogen-induced preneoplastic lesions in the animal model. As such, the potentiator enhace or may even synergize with the purified and isolated anti-oxidants from natural sources, such as quercetin and mixed tocopherols.

As used herein, the terms "glyconutritional" or "glyconutrient" refer to complex carbohydrates or saccharides or simple sugars that are synthesized in nature and are necessary for the biochemical synthesis of various classes of communication and signal molecules that may be free in interstitial cellular fluids, active in cell to cell communication (i.e., cytokines, growth factors, etc.), or constitute the molecular configuration comprising foci of highly specific molecular activity of cell membranes (i.e., receptor sites, ion-transport channels, antigenic identification, and the like).

As used herein, the terms "phytonutritional" or "phytonutrient" refer to naturally synthesized molecules found only in plants that are produced to protect the plant's cells. Phytonutrients primarily have antioxidant, free-radical scavenger and vital micronutrient activity. These molecules, supplied through dietary supplementation, are found in mature plant tissues, and are most concentrated in seed coats and fruiting tissues surrounding the seed. In mammalian tissues, these molecules, when supplied in the diet, are active in optimizing the biochemistry, immunology and physiology in the cellular microenvironment.

As used herein, the terms "plant extract" and "herbal extract" are used interchangeably to refer to phytochemicals that are produced in plant tissues and that can be extracted by water, polar, or petroleum solvents, and that have some degree of beneficial health or therapeutic activity. Most herbal agents can be toxic, especially when concentrated, but are generally safe when utilized in their more traditional manner in teas and poultices as a "folk medicinal for the treatment of disease and promotion of good health." As used herein, the term "herbal body-toning agent" refers to substances that have been observed by the inventors to reduce and reverse elastic tissue and collagen fiber damage caused by aging or sun-damage as evidenced by a restoration of skin turgor and elasticity which effectively reduces or eliminates wrinkles, sagging, hyperpigmentation and reversal of other undesirable elements of lost cosmetic appearance.

The carbohydrates included in the dietary supplement of the invention are available from a wide variety of natural and synthetic sources such as shrubs, trees, plants, yeasts, fungi, molds, gums, resins, starch and cellulose derivatives and natural mucin sources. Specifically, some of the natural sources include: (a) shrub or tree exudates which contain acacia, karaya, tragacanth, or ghatti; (b) marine gums which include agar, algin, or carrageenan; (c) seed gums which include guar, locust bean, or psyllium; (d) plant extracts which contain pectins or acetylated polymannose; (e) starch and cellulose derivatives such as hetastarch, carboxymethylcellulose, ethylcellulose, hydroxypropyl methylcellulose, methylcellulose, oxidized cellulose; and microbial gums which contain dextrans, xanthan. However, it should be recognized that the composition of the invention is not intended to be limited by the source from which the respective carbohydrates are obtained.

The saccharides of the invention can be found in nature as mono-, oligo- and/or polysaccharides. Thus, the compositions of the invention can contain the saccharides in their monomeric, oligomeric and/or polymeric forms. For a list of known natural sources for the saccharides and their uses, please refer to U.S. Patent Application No. US2003072770.

As used herein, the term "carbohydrate" is used interchangeably with the terms "saccharide," "polysaccharide," "oligosaccharide" and "sugar" the definitions of which are well known to those skilled in the art of carbohydrate chemistry. Although the compositions of the invention are intended to include at least two or more essential saccharides, it should be noted that the saccharides can be in the form of mono-, oligo- and/or polysaccharides, e.g., a composition containing gum tragacanth and guar gum will be considered as containing galacturonic acid, sialic acid, mannose and galactose. Therefore, by controlling the amount of particular gums in a given dietary supplement, one can control the amount of the respective saccharides in the dietary supplement.

As used herein, the term "a mixture of at least two forms of vitamin E" is used to describe a mixture of at least two forms of tocopherol selected from alpha, beta, delta, epsilon, gamma, zeta, eta, xi1, xi2, and sigma tocopherols, and alpha, beta, delta and gamma tocotrienols, and combinations or derivatives thereof. In one embodiment, "a mixture of at least two forms of vitamin E" is a mixture of at least two forms of tocopherol selected from alpha, beta, delta, and gamma tocopherol. In another embodiment, "a mixture of at least two forms of vitamin E" is a mixture of alpha, beta, delta, and gamma tocopherol. "A mixture of at least two forms of vitamin E" may be obtained from VITAECAPS, SA, Spain, from Henkel Corporation; or from Cognis Corporation (Kankakee, IL), for example. COVITOL® F-350M is commercially available from Cognis and contains natural source alpha-tocopherol with mixed tocopherols which are obtained from edible vegetable oils. The particular mixture of tocopherols included in the antioxidant composition of the present invention is determined by running an ORAC(o) antioxidant determination.

Salts or derivatives of tocopherols include pharmaceutically acceptable salts such as acetate, sulfate, succinate, nicotinate, allophanate, phosphate, quinone, or halogenated derivatives; esters; stereoisomers; and the like. The invention encompasses the use of vitamin E derivatives in which substitutions, additions, and other alterations have been made in the 6-chromanol ring and/or side chain, with the proviso that the derivatives maintain antioxidant activity of a vitamin E. For example, tocopherols and their derivatives can vary by the number and position of alkyl groups, double bonds and other substituents and variations on the ring and side chain. An "alkyl" is a cyclic, branched or straight chain chemical group containing only carbon and hydrogen, such as methyl, butyl, and octyl. Alkyl groups can be either unsubstituted or substituted with one or more substituents, e.g., halogen, alkoxy, acyloxy, amino, hydroxyl, mercapto, carboxy, or benzyl. Alkyl groups can be saturated or unsaturated at one or several positions. Typically alkyl groups will comprise 1 to 8 carbons, 1 to 6, or 1 to 4 carbon atoms. Additional tocopherols can be constructed by conjugation to the ring structure or side chain of various other moieties, such as those containing oxygen, nitrogen, sulfur and/or phosphorus. Tocopherol derivatives can also be made by modifying the length of the side chain from that found in prototypical tocopherols such as alpha-, beta-, delta- and gamma-tocopherol. Tocopherols can also vary in stereochemistry and saturation of bonds in the ring structure and side chain.

Additional tocopherol derivatives, including prodrugs, can be made by conjugation of sugars or other moieties to the side chain or ring structure. Mixed tocopherols include without limitation mixtures of stereoisomers of a single tocopherol (e.g., +and - stereoisomers of alpha-tocopherol; (+/-) indicates a racemic mixture) or mixtures of structurally distinct tocopherols (e.g., alpha- plus gamma-tocopherol).

Although the present invention includes the above cited eleven essential saccharides, it should be noted that other saccharides, nutritional compounds or biologically active or inert compounds may be included in the dietary supplement of the invention. Such other nutritional compounds include any one or more of phytonutrients, dioscorea complex, plant extracts, herbal extracts, plant parts, herbal components, vitamins or minerals. These nutritional compounds can be added to the dietary supplement of the invention, or they can be provided separately to a mammal being administered the dietary supplement.

For example, a person receiving the glyconutrient-containing dosage form of the invention can also receive a phytonutrient in either the same or a separate dosage form. Inert compounds can include flavors, fillers, lubricants, buffers, gels, binders, excipients, carriers and/or other such compounds that facilitate the formulation or administration of the inventive dietary supplement. All of the glyconutrient containing dietary supplement compositions of the invention, even those containing additional compounds, agents or other substances, can be obtained directly from Mannatech, Inc. (Coppell, Tex.).

The present invention includes a method for directly and concurrently measuring the oxygen radical absorption capacity (ORAC) of a composition that includes both hydrophobic and/or hydrophilic antioxidants. The term "ORAC(o)" is used since the assay measures the ability of antioxidants to quench radicals by directly tracking the disappearance of oxygen in an oxygen radical absorption capacity assay the directly measures oxygen content in a sample (ORAC(o)). Current industry standard assays such as ORAC(fl) and ORAC($\beta$-PE), measure antioxidant capacity by indirectly measuring degradation of fluorescent emissions of a fluorescent compound (fluorescein or $\beta$-phycoerythrin) upon exposure to oxygen radicals. These assays work well with hydrophilic antioxidants, but have limited effectiveness in measuring hydrophobic antioxidants or mixtures of hydrophobic and hydrophilic antioxidants. Furthermore, unlike the known ORAC(fl) and ORAC($\beta$-PE) systems, the ORAC(o) disclosed herein is suitable for simplification and manufacturing as a disposable sensor. The system is robust enough to even permit the manufacture of an office and even a home system for immediate evaluation of oxidative capacity of a user from biological samples.

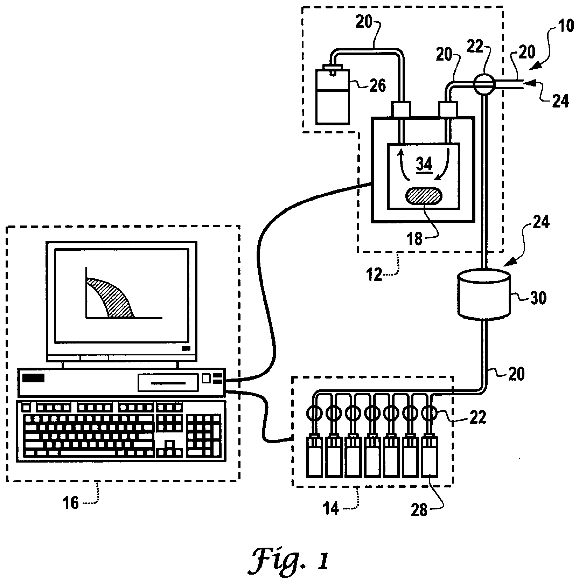

ORAC(o) Apparatus. FIG. 1 is a depiction of an ORAC(o) direct anti-oxidant apparatus 10. The apparatus 10 has, as depicted, three basic components: a detector system 12, a fluidics system 14 and a data processor system 16, which may be interconnected to provide data capture, fluidic and sample control and data processing. The detector system 12 has an oxygen sensor 18, which is in fluid communications with the fluidic system 14 via one or more conduits 20. Fluid flowing through the one or more conduits 20 is controlled using one or more valves 22, which may be manually controlled and/or under the control of the data processor system 16. In operation, a sample 24 enters the fluidic system and is directed into the detector 18, and after data capture is delivered to waste storage 26. The fluidics system 14 may also include one or more solutions 28 that directed into transit through the fluidic system 14 by pumps, by vacuum or by pressure, e.g., pressurized inert gas. The solutions 28 in the fluidics system 14 will generally be premixed or equilibrated, as for use with the present invention may generally include: water, a solvent, a detergent or water:detergent mix, an oxygen radical generator, an oxidation target, etc., and may be mixed at mixing chamber 30 prior to delivery to the oxygen detection chamber 34. The choice of fluidics system will depend on the extent of automatization desired or chosen, as will be known to those of skill in the art. The sample 24 may be pre-mixed with the same solution that is used to calibrate the oxygen sensor 18 or may even be pre-mixed at the mixing chamber 30.

Examples of oxygen detectors 18 for use with the present invention will include any dissolved oxygen sensor that is able to detect dissolved oxygen in the presence of a solvent, water and a detergent. Examples of dissolved oxygen sensors include, e.g., electrochemical, chemiluminescent, surface plasmon resonance, infrared, capacitance coupled, dye-coupled fiber optic or even hyperspectral oxygen sensors. In one specific example, the dissolved oxygen sensor is an YSI 5300A biological oxygen sensor (YSI, USA), a SPREETA sensor (Texas Instruments), a PASCO PS2108 (Pasco, USA), and the like. In one example, the dissolved oxygen sensor has the following specifications: Range of: 0-20 mg/L; Accuracy: $\pm 10\%$ of full scale; Resolution: 0.01 mg/L; Maximum Sample Rate: 20 sps; Default Sample Rate: 2 sps; Response: 98% in 60 seconds; Temperature Range: 0-50°C; Temperature Compensation: 10-40°C; Cathode: Platinum; Anode: Ag/AgCl; Membrane: 1 ml silicon, and may be used in conjunction with the software provided by the manufacturer, e.g., Dissolved Oxygen EZ (Pasco, USA). The system may also include pH, ORP, conductivity or turbidity sensors in fluid communication with the fluidics system

In operation, the ORAC(o) system disclosed herein may be used as follows: a user collects a sample and dissolves it in or with a ORAC(o) solvent kit (dry or liquid). An ORAC(o) sensor, e.g., a hand-held surface plasmon resonance oxygen sensor (see, e.g., Texas Instruments SPREETA sensor) is exposed to one or more calibration standards and then exposed to the user sample. The oxygen sensor is connected to a processor that evaluates the output from the detector surface on the sensor and provides the user with a read-out. The read-out may be displayed on a screen, printed and/or transmitted to a processor, memory and the like. The user sample may be a urine, saliva, tears, mucus secretions, sweat, blood (or blood products), tissue, feces or other biological samples suspected of having oxygen radicals. In one example, the sample is one or more breaths (one or more inhalations and/or exhalations) that are collected by a respirator, e.g., a closed respirator. The values detected by the sensor may even be saved in memory (volatile, semi-permanent or permanent) for future reference or for comparison to past or future values to evaluate the oxidative state of the user.

The basic components of the ORAC(o) assay for antioxidant activity of the present invention take advantage of existing ORAC-like methods and are therefore easily adaptable for use in laboratories without the need for extensive training, if any. Briefly, the ORAC(o) uses an oxygen sensor, for example a blood plasma oxygen sensor or a dissolvable oxygen sensor to measure pro-oxidant activity, e.g., by measuring directly the relative activities of one or more oxygen radical generating molecules in the sample solution and a oxidative quencher (anti-oxidant) as standards. It must be noted that certain agents, e.g., reducing or volatile agents, can lead to the absorption or production of oxygen in solution in the absence of a radical source, e.g. AAPH, can affect the amount of oxygen in the solution. Using the present invention the skilled artisan can differentiate between radical quenching and non-radical quenching activities of samples by, e.g., evaluating the behavior of the sample in solution before the addition of the free radical initiator. It should be noted that both the radical quenching and non-radical quenching activities of the samples tested, as determined by use of the present invention, relate to oxidative state. The relative activities of the oxygen radical generator and the oxygen radical quencher may be titrated and/or measured over time as with the indirect methods ORAC(fl), ORAC(fl-lipo), ORAC($\beta$-PE) and the like.

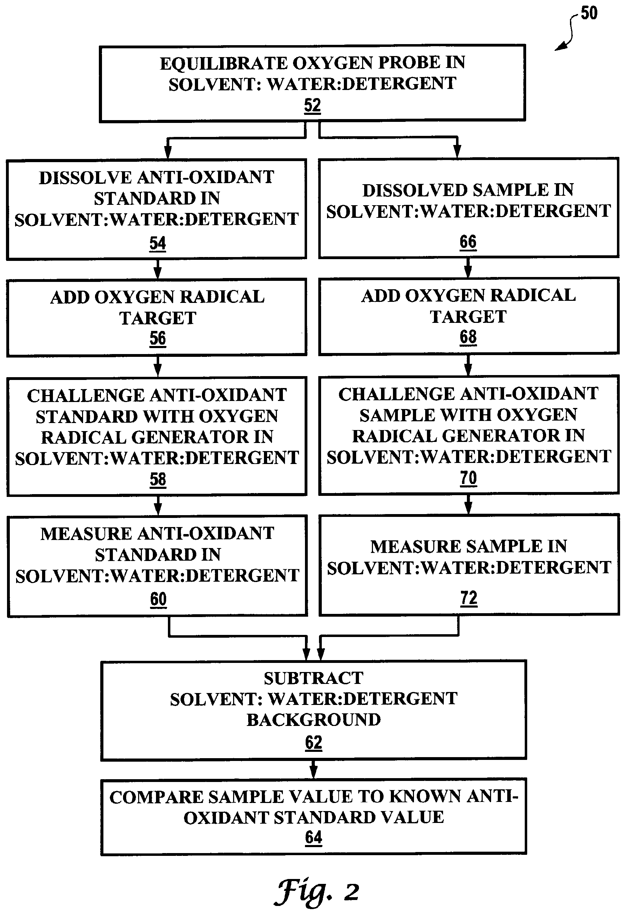

Figure 2 is a flowchart 50 that summarizes the basic steps of the method of the present invention. In step 52, the dissolvable oxygen probe is equilibrated and/or calibrated in the presence of the solvent:water:detergent mixture and a baseline measured. In one examples the solvent:water:detergent is an acetone:water:Tween-20 mixture at a 1:1:1 ratio. In step 54, a baseline determination of anti-oxidant activity serves as the positive control and baseline for comparison of anti-oxidant activity using, e.g., Trolox® as the anti-oxidant. One advantage of Trolox® and related molecules is that these Vitamin E derivatives are more stable from lot to lot, have less lot variation and are synthetic, thereby providing a reliable concentration of anti-oxidant activity. The mixture in step 54 is mixed, in step 56, with an oxygen radical target, e.g., linoleic acid prior to the addition of the oxygen radical generator in step 58. The assay is allowed to run and, in step 60, the area under the curve (AUC) is calculated by measuring the disappearance of dissolved oxygen over time, and the value for the sample stored. In series or parallel, a sample is dissolved in the solvent:water:detergent mixture (step 66) followed by the addition of the oxygen radical target in step 68. Generally, it is common to use the same type of oxygen radical target in steps 56 and 68, and the same type of oxygen radical generator in steps 58 and 70. In step 72, the AUC for the sample is detected, and the value for the sample stored. Now that the standard and the sample AUC have been determined, the values are normalized by subtracting the AUC for the blank. The normalized standard and sample AUC values are then used to compare and calculate the level of anti-oxidant activity in the sample.

The following discussion is used to help illustrate the invention and should not be used limit its scope. The detergent Tween 20 may aid in the dispersion of linoleic acid. Linoleic acid provides double bonds across which oxygen can be absorbed. Trolox® is a synthetic antioxidant used as an internal standard. The values obtained from all samples are related back to those of Trolox®. The oxygen radical generating molecule: 2,2' azobis (2-amidinopropane)dihydrochloride (AAPH reacts) with oxygen to create carbon-centered radicals. The radicals generated by AAPH cause the oxidation of linoleic acid. As a result of the oxidation of linoleic acid, linoleic acid's double bonds become ketones, bonding with oxygen molecules in the carbon-centered radical. The oxygen probe takes measurements of the rate at which the oxygen is being removed from the reaction chamber due to the oxidation of linoleic acid. The azo radical may react directly with linoleic acid, causing the formation of a linoleic acid radical. The linoleic acid radical then reacts with the oxygen present in the reaction chamber to form a ketone. Through either proposed mechanism, oxygen is consumed due to the oxidation of linoleic acid. The antioxidant slows the consumption of oxygen in the reaction chamber by deterring the oxidation of linoleic acid. The calculation of the area under the curve for dissolved oxygen versus time plot yields a measure of the sample's antioxidant capacity as demonstrated by its ability to slow the oxidation of linoleic acid.

The oxygen radical generators. Azo-radical generators are present in the ORAC(o) assay of the present invention at a known concentration to generate radicals for measurements of antioxidant activity. Azo initiators include, for example, 2,2'-azobis[2-(5-methyl-2-imidazolin-2-yl)propane]dihydrochloride, 2,2' azobis (2-amidinopropane)dihydrochloride (AAPH), 2,2'-azobis(2-amidinopropane)[2-(N-stearyl)amidinopropane] dihydrochloride (SA-1), 2,2'-azo(2-(2-imidiazolin-2-yl)-propane)-[2-[2-(4-n-octyl)imidazolin-2-yl]-propane] dihydrochloride (C-8), 2,2'-azobis(4-methoxy-2,4-dimethylvaleronitrile) (MeO-AMVN), 2,2'-azobis(2,4-dimethylvaleronitrile) (AMVN), azo-bis-isobutylnitrile, 2,2'-azobis (2-methylproprionate) (DAMP), and 2,2'-azobis-(2-amidinopropane).

For example, the radical generator 2,2' azobis (2-amidinopropane) dihydrochloride (AAPH) decomposes into molecular nitrogen and two carbon radicals. The carbon radicals combine to produce stable products or react with molecular oxygen to give peroxyl radicals. The half life of AAPH is about 175 hours (37 °C at neutral pH). Therefore, the rate of free radical generation is essentially constant during the first several hours in solution. AAPH is used is often used for lipid peroxidation in aqueous dispersions of fatty acids, as such; it can be used alone or in combination with a lipohilic and/or a lipophobic radical generator. The solvent system disclosed herein allows for use of either or both lipohilic and/or a lipophobic radical generators as the apparatus measures total oxygen in the sample.

Solvent System. The solvent system for the ORAC(o) is a three-part system that includes a solvent, an aqueous phase and a detergent. For example, the solvent may be an organic solvent selected from alcohols, amines, esters, glycol ethers, glycols, terpenes and/or mixtures thereof. The organic solvent system is formulated to be less than about 50%, around 30 or 33 percent, less than 20% and in some cases less than 10% of solvent components.

In one embodiment the solvent is acetone, which may be from between about 10 and 90 percent vol/vol of the ORAC(o) solvent system, the aqueous portion from between about 10 and 90 percent vol/vol of the ORAC(o) solvent system and the detergent from 0.001 to 90% of the solvent system. For example, the ORAC(o) solvent system may be one-third water, one-third detergent ("one-third" solvent), and the sample at a concentration of, e.g., 1 mg/mL. Dilutions are then made using the same solvent. The detergent may be a nonionic detergent such as TWEEN®, (i.e., TWEEN®20), BRIJ®, or TRITON®; a zwitterionic detergent such as CHAPS®; a cationic detergent; or an anionic detergent such as cholate, deoxycholate, sodium dodecylsulfate, or TWEEN®-80; or a surfactant. The ratio of water to acetone to detergent may be from between about 5% to 90% to 90% to 5%, respectively. Unlike the ORAC(fl), which uses a two component system that uses of one-half acetone and one-half water, the detergents of the ORAC(o) solvent system permit a direct measurement of oxygen from the total sample. One variant is the ORAC(fl-lipo) that uses a randomly methylated β-cyclodextrin.

Uses for the ORAC(o) Assay: The ORAC(o) assay may be used to measure the total antioxidant activity of biological samples, for the evaluation of components for the nutritional supplements of the present invention and even for testing and evaluating competitor and/or the final dietary supplement of the present invention. For use in the evaluation of biological samples, these may be, e.g., serum, lipid-soluble serum fraction, water-soluble serum fraction, urine, lipid-soluble urine fraction, water-soluble urine fraction, LDL fraction, tissue homogenates, quality control of antioxidant supplements, food products, or preservatives, development of new antioxidant supplements, development of new food products, new preservatives, or new antioxidant therapies, quality control of food manufacturing and processing, assessing antioxidant activity of plants, or monitoring the antioxidant activity of cosmetic products, for example.

EXAMPLE 1: Comparison of Antioxidant Activity Using ORAC(fl) and ORAC(o) Assays.

Ingredients for an antioxidant composition were analyzed for antioxidant activity using a prior art oxygen radical absorption capacity method that measures fluorescence (ORAC(fl)), and using the method of the present invention that measures dissolved oxygen (ORAC(o)). The antioxidant activity of a product is its ability to protect the system from damage caused by peroxyl radicals.

Prior Art Method of Measuring Antioxidant Activity for Comparison. For the ORAC(fl) assay, the method of Ou *et al.* (Ou , B., Hampsch-Woodill, M. and Prior, R.L., J. Agric. Food Chem. 2001, 49, 4619-4626) was followed. Differences from the Ou procedure as published included the speed of the orbital shaker (Ou, 400 rpm; herein, 280 rpm), and the centrifuge speed (Ou, 14,000 rpm, 15 min; herein, 3200 rpm, 15 min and the length of the assay (Ou, 30 min; herein, 100 min). Fluorescein, sodium salt, was obtained from Aldrich (Milwaukee, WI). For the Ou method, a standard amount of fluorescein is added to an antioxidant product being tested, and the beginning level of fluorescence is measured. A free radical initiator is added, and the time and degree of disappearance of fluorescence are measured. Trolox®, 6-Hydroxy-2,5,7,8-tetramethylchroman-2-carboxylic Acid, is a cell-permeable, water-soluble derivative of vitamin E with potent antioxidant properties. Trolox® prevents peroxynitrite-mediated oxidative stress and apoptosis in rat thymocytes and is a synthetic antioxidant that is consistent from lot-to-lot and is used as the standard and run for comparison with each sample. A blank (control) used in calculations of the ORAC(o) value for each samples may be included in each run. Fluorescence vs. time is plotted. The blank (control) is subtracted from every curve. The net area under the curve of the antioxidant is compared to the net area under the curve of Trolox®. The larger the net area under the curve, the great the antioxidant capacity of the molecules in the sample. All ORAC(fl) analyses were performed on a COBAS FARA II centrifugal analyzer (Roche Diagnostic System Inc., Branchburg, NJ; excitation wavelength = 493 nm and emission filter = 515 nm). Results are given as micromoles of Trolox® equivalents per gram of sample.

Method of Measuring Antioxidant Activity. In operation, the ORAC(o) assay of the present invention was used to evaluate antioxidant compositions, combinations and the like of solutions against a target molecule (linoleic acid) with oxygen present at equilibrium with the air. A free radical initiator is added and the time it takes for oxygen to disappear is measured, yielding the rate of disappearance. Trolox® is used as the standard, and a blank is run as a control. Amount of dissolved oxygen vs. time is plotted allowing direct comparison of net areas under the curve. A detailed method is as follows: Buffers ($K_2HPO_4$ (F.W. 174.2), $NaH_2PO_4$ (FW 120.0)) were obtained from Sigma (St. Louis, MO). Linoleic acid 99%, Trolox® (6-hydroxy-2,5,7,8-tetramethylchroman-2-carboxylic acid) 97%, TWEEN® 20, and 2,2'-azobis (2-methylpropionamidine) dihydrochloride 97% (AAPH), were obtained from Aldrich (Milwaukee, WI). HPLC grade acetone was obtained from Fisher (Hampton, NH). A YSI 5300A biological oxygen meter was obtained from YSI (Yellow Springs, Ohio) and used according to the manufacturer's specifications.

ORAC(o) Method. The following stock solutions were used. Preparation of phosphate buffer (75 mM, pH 7.4); 750 mM $K_2HPO_4$ (F.W. 174.2); Weigh 65.33g into 500mL of volumetric flask, and add dH20 to the mark. Concentration is 750 mM. Store 750 mM $K_2HPO_4$ stock solution in a refrigerator (2 to 8°C) equipped with a calibrated thermometer. Target temperature is 4°C. 750mM $NaH_2PO_4$ (FW 120.0); Weigh 45.00 g into 500 mL of volumetric flask, and add dH20 to the mark. Concentration is 750 mM Store 750mM $NaH_2PO_4$ stock solution in a refrigerator (2 to 8°C) equipped with a calibrated thermometer. Target temperature is 4°C. Mix 40ml of 750 mM $K_2HPO_4$ stock solution and 10ml of 750mM $NaH_2PO_4$ stock solution (4 to 1 ratio). This will yield 50 mL of ORAC stock solution buffer. Store stock solution in a refrigerator (2 to 8°C) equipped with a calibrated thermometer. Target temperature is 4°C. Dilute mixture with dH20 (1:9) to make working solution of ORAC buffer, and measure pH. It should be 7.4. Keep covered at room temperature until ready to use.

Tween Preparation. Weigh out 10g of Tween 20, and add 90 g of deionized water. Cover and stir the mixture overnight. Tween stock solution keeps for one week. Keep on countertop.

Solvent Preparation. Mix 25 mL of acetone, 25 mL of deionized water, and 25 mL of Tween 20. Use this solvent to prepare samples. Before a run is performed: the probe should be inspected for damage paying particular attention to the membrane, replace if needed. Probes should be stored with the tips in deionized water. Only one probe and one reaction chamber should be used for this assay. Run a blank and Trolox® (at 10 μg/ml or 0.03995 μmol/ml) every 8 hours. Samples are to be run at a concentration of 10 μg/ml. The sample concentration and Trolox® concentration should be the same:

The dilutions are prepared by weighing out 1 mg of sample and adding 1 ml of solvent to perform the initial extraction. Shake for one hour, place 0.5 ml of the initial extraction solution in a glass scintillation vial, and then add 4.5 ml of solvent, making the first dilution. Vortex the first dilution for about 30 seconds. Its concentration is 0.1 mg/ml or 100 ppm. Take 0.5 ml of the first dilution, put it into another glass scintillation vial, and then add 4.5 ml of solvent, vortex for about 30 seconds, to yield a concentration of 0.01 mg/ml or 10 ppm. Take 0.5 ml of the vial sample and put it into another glass scintillation vial, and then add 4.5 ml of solvent, vortex for about 30 seconds, to yield a concentration of 0.01 mg/ml or 10 ppm. The vials are placed in a 4° C refrigerator. In a 37° C water bath allow linoleic acid to thaw, and prepare linoleic acid solution (with minimal light) by adding 0.18 mL Tween stock solution, 0.18 mL buffer, and 0.44mL deionized water to 70.8 mg linoleic acid. Keep solution tube wrapped in foil. Make linoleic acid solution fresh every 8 hours. To prepare the oxygen radical generator, weigh 67.8mg of AAPH, and dissolve in 0.9 mL buffer. Keep AAPH refrigerated, it may be used for up to eight hours. Shake for 1 hour. The Trolox® solution should be kept refrigerated at all times. To begin measuring anti-oxidant activity, add 1.86 mL of buffer into each reaction container, 2.36mL water, to the scintillation vials at 37° C and allow three minutes of stirring while the reaction matrix comes to temperature. Add 0.284 mL of the sample, and place the probe tip into the vessel without touching the reaction solution, and allow one minute of stirring. The measurements are best taken in a light-tight environment.

Following data capture, remove the probe from the chamber, add 0.357mL each of linoleic acid solution, and replace the end of the probe into the chamber immediately. Add 0.357ml of AAPH solution, and place the probe into the solution tube carefully but quickly so that no bubbles are left in the reaction area, and no solution is in the overflow ridge on the probe case. Next, place probe into the solution and take a reading. Capture data and calculate anti-oxidant activity.

Samples were prepared at a concentration of 1mg/mL in "one-third solvent" (also referred to as the ORAC(o) solvent system). A "one-third" solvent is equal parts of water, acetone, and a solution of TWEEN®20 diluted 1:9 with water. Samples were shaken for 1 hour at room temperature on an orbital shaker at 280 rpm. The sample solution was ready for analysis after further dilution (generally to 10 μg/mL) with "one-third" solvent. "One-third" solvent was also used as the blank.

The ORAC(o) assay was carried out using the YSI 5300A biological oxygen meter. Linoleic acid was prepared by adding 0.18 mL of 75 mM phosphate buffer (pH 7.4), 0.18mL of 10 weight percent TWEEN®20 stock solution, and 0.44 mL of deionized water to 70.8 mg linoleic acid. AAPH was prepared by adding 0.9 mL of buffer to 67.8 mg of AAPH. In the final reaction volume (5.218 mL), linoleic acid (21.59 mM) was used as the target of free radical attack, and AAPH (19.00 mM) was used as a peroxyl radical generator. Trolox® (at 10 $\mu$g/mL) was used as a standard. Readings were taken every second until a zero reading was observed.

The formula for calculating the ORAC(o) value (Oxygen Radical Absorbance Capacity oxygen specific electrode) is:

$$ORAC(o) = \frac{\dfrac{AUC_{SMP}-AUC_{BLNK}}{AUC_{TRLX}-AUC_{BLNK}} \times 1000 \text{ (mg/gr)} \times [TRLX \text{ (}\mu mol/ml\text{)}]}{[SMP \text{ (mg/ml)}]}$$

Alternatively, the ORAC(o) may be calculated in milliliters when evaluating a liquid formula. This calculation yields a quantity known as micromoles of Trolox® equivalents per gram of sample. A negative value ORAC(o) reflects less radical quenching activity than obtained with a blank which indicates that a composition is a pro-oxidant, i.e., an agent that promotes oxidation, rather than acting as an antioxidant. An assumption of the ORAC(o) assay is that oxygen is not being absorbed or released by the sample, however, any effect in the oxygen level of the sample can be evaluated and used to compensate into the calculated anti-oxidant value.

Comparison of ORAC(o) and ORAC(fl) Assay Parameters. The advantages of the ORAC(o) as compared to the ORAC(fl) were measured in a direct vs. indirect measurement of antioxidant potential. The ORAC(fl) is an indirect oxygen radical detection method because it relies on the assumption that the fluorescein (target molecule) is the only fluorescent component being measured. However, many antioxidant compounds fluoresce naturally (e.g., blueberries); and combinations of these compounds from radical-radical reactions fluoresce also. Fluorescence from the sample, therefore, can skew the results of an assay based on fluorescence. The ORAC(o) method, on the other hand, is a direct measurement of oxygen uptake, that is, a direct measurement of the disappearance of oxygen into free radicals. This method of measurement of antioxidant capacity is not dependent upon whether the oxygen is attached to water- or lipid-soluble components. A comparison of the assay parameters of the ORAC(fl) and the ORAC(o) methods is provided in Table 1.

**Table 1:** Comparison of Assay Parameters for ORAC(fl) and ORAC(o).

| Assay Parameter During Assay | ORAC(fl) | ORAC(o) |
|---|---|---|
| Temperature | 37°C | 37°C |
| AAPH concentration | $1.28 \times 10^{-2}$ M in phosphate buffer | 19.00 mM in phosphate buffer |
| Target molecule concentration | fluorescein, 43.8 nM in phosphate buffer | 21.59 mM linoleic acid, in mixture of 10% TWEEN® 20, buffer and water (.18 mL, .18 mL, .44 mL) |
| Sample concentration | initially at 500 mg in 20 mL, supernatants diluted to 10 $\mu$g/mL with buffer | 1 mg/mL initially, diluted to 10$\mu$g/mL |
| Sample solvent | acetone/water, 50/50 v/v dilutions in phosphate buffer | water/acetone/10% by weight TWEEN® 20, v/v/v ("one-third" solvent) dilutions in "one-third" solvent |
| Buffer for assay, concentration | 75 mM phosphate, pH 7.4 | 75 mM phosphate, pH 7.4 |
| Final assay volume | 400 $\mu$l | 5.218 mL |
| Blank (control) | buffer | "one-third" solvent |
| Standard | 62.5 $\mu$M Trolox® in buffer | Trolox® at 10 $\mu$g/mL (38.755 $\mu$M) in "one-third" solvent |

(continued)

| Assay Parameter During Assay | ORAC(fl) | ORAC(o) |
|---|---|---|
| Method of measuring anti-oxidant capacity of the sample | fluorescein yields a decrease in fluorescence as it is oxidized by radicals, antioxidant protects and delays decrease | oxygen is taken up by the radical initiator, and by linoleic acid carbon radicals; antioxidant protects and decreases oxygen uptake |

One distinct advantage of the present invention is that the user does not need to learn new techniques or purchase equipment to incorporate the present apparatus and method into their laboratory environment. For example, when measuring sample saturation, the present invention uses many of the same buffers, conditions, extraction and/or separation steps as in the well-established indirect measurement system developed by, e.g., Ou, et al. Ou, et al., prepared samples in acetone/water (50:50, v/v) at a concentration of 500 mg in 20 mL, rotated them on a rotary shaker at 400 rpm for 1 hour, centrifuged them at 14,000 rpm for 15 minutes, and diluted them with 75 mM potassium phosphate buffer at pH 7.4 (Ou, et al., Development and Validation of Oxygen Radical Absorbance Capacity Assay for Lipophilic Antioxidants Using Randomly Methylated-p-Cyclodextrin as the Solubility Enhancer, J. Agric. Food Chem. 2002, 50, 1815-1821). The present inventors found that much of a test sample would not go into solution under the Ou conditions (ORAC(fl) conditions), the more soluble components displace the less soluble components. Consequently, only that portion of the sample that was solubilized under the ORAC(fl) conditions was included in the ORAC(fl) sample and measurement. Therefore, a supernatant solution made according to the ORAC(fl) method for analysis is unlikely to be representative of the total anti-oxidant activity of the actual sample. Since a solution of a sample in the ORAC(fl) buffer does not always reflect the contents of the sample, results can be skewed using the ORAC(fl).

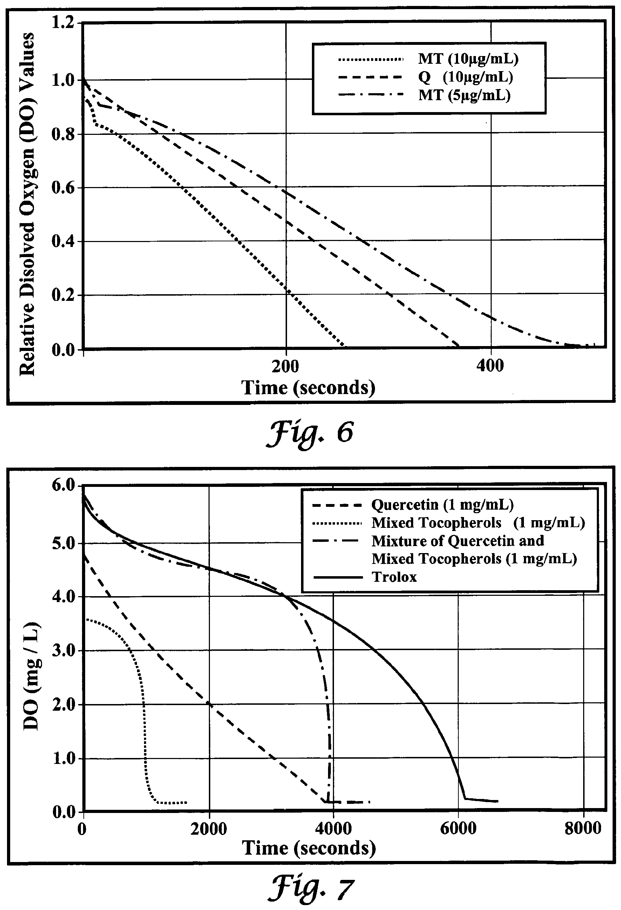

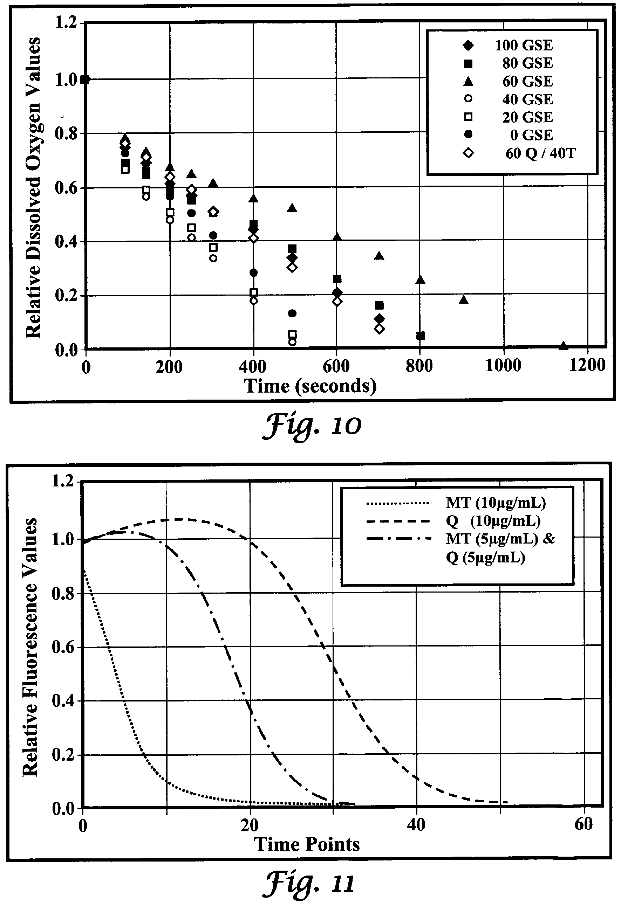

The problem with sample dissolution was recognized and a lack of contribution of mixed tocopherols to an antioxidant measurement carried out on a mixture of quercetin and mixed tocopherols when using the ORAC(fl) was observed. As shown in FIG. 7, using the ORAC(o) system, it was found that the antioxidant effect of the combination of quercetin at 5 $\mu$g/mL and mixed tocopherols at 5 $\mu$g/mL as compared to each ingredient separately at 10 $\mu$g/mL. In contrast, the indirect ORAC(fl) system shows no further effect from the same combination beyond the value for quercetin alone (see FIG. 11). Lack of activity from such a combination with mixed tocopherols and saturation problems are absent from the ORAC(o) method since the concentration of sample is more dilute and since the solvent for the sample contains acetone, water, and a detergent for solubilizing all components of a sample. Presence of TWEEN®20 in the solvent for the sample accounted for detection of activity from mixed tocopherols. As a control for Trolox® activity, FIG. 7 shows the quercetin at 5 $\mu$g/mL and mixed tocopherols at 5 $\mu$g/mL as compared to each ingredient separately at 10 $\mu$g/mL but also includes the Trolox® control and demonstrates the ability of ORAC(o) to detect the level of oxygen radicals remaining in the sample over time.

The ORAC(o) provides a significant savings as compared to the more expensive ORAC(fl) (automated circa $250,000; non-automated circa $50,000). In contrast, the ORAC(o) apparatus disclosed herein takes advantage of off-the-shelf oxygen sensor systems that may be easily miniaturized and/or automated that may be developed for sale to doctors offices and even home use at a fraction of the costs of large fluorimeter detection systems. Validation, as conducted by the Association of Official Analytical Chemists (AOAC) guidelines for single laboratory validation, provided results in Tables 2, 3, and 4.

**Table 2**. 5 Day Trial for Precision (Repeatability)

| | Area Under Curve (AUC) |
|---|---|
| Day 1 | 202.655447939563 |
| Day 2 | 212.901122995373 |
| Day 3 | 187.01466464995 |
| Day 4 | 202.856617129109 |
| Day 5 (2nd Analyst) | 213.124092629655 |
| 5 Day Mean | 203.710389068730 |
| Standard Deviation | 10.649840914564 |
| Relative Standard Deviation | 5.23% |
| HORRAT | 1.981060606061 |

The values tor each day are an average of 3 runs of the quercetin sample at a concentration of 10$\mu$g/mL. The

HORRAT value is the ratio between observed $RSD_R$ values and the $RSD_R$ values predicted by the Horwitz equation known to those of skill in the art, and is regarded as an indication of the acceptability of a method with respect to its precision. In a single laboratory performance study, a series of HORRAT ratios between 0.5 and 2.0 indicate acceptable precision of a method. The HORRAT value for the 5-day trial is 1.98. A determination of the analytical range as a linear range is provided in Table 3.

**Table 3.** Determination of Analytical Range

| Sample ($\mu$g/ml) | Area Under Curve |
|---|---|
| 1 | 124.567114093960 |
| 10 | 209.388111888112 |
| 100 | 693.611111111111 |
| 500 | 2327.96518987343 |
| 1000 | 2870.09540636041 |

y = 2.8335x + 332.16; R2 = 0.9231 for 1-1000 plot
y = 4.3353x + 176.66; $R^2$ = 0.9967 for 0-500 plot

Linear integrity appears to decline as the sample concentration nears 1000 $\mu$g/mL. With the determination of the analytical range as a linear range, the value of the area under the curve of variable concentrations up to 500 $\mu$g/mL is determined. Table 4 provides results for precision of single-day results.

**Table 4.** Single Day Trial for Precision (Repeatability)

| | Area under curve |
|---|---|
| Run 1 | 207.369402985075 |
| Run 2 | 198.18118466899 |
| Run 3 | 205.035211267606 |
| Run 4 | 201.586572438162 |
| Run 5 | 202.110714285714 |
| 5 Run Mean | 202.856617129109 |
| Standard Deviation | 3.505016471434 |
| Relative Standard Deviation | 1.73% |
| HORRAT | 0.654480870834 |

The single day precision trial involved 5 separate runs of the quercetin sample at a concentration of 10$\mu$g/mL. The HORRAT value of Table 4 for the ORAC (o) method is 0.65448. The ORAC(o) assay was used to optimize ratios of the ingredients in an antioxidant composition as set forth in Example 2. One embodiment of the composition has weight ratios of quercetin, 49.18%; mixed tocopherols, 32.79%; grape skin extract, 9.84%; green tea extract, 6.56%; and bush plum, 1.64% gave an antioxidant value using the ORAC(o) of 17,254 micromoles Trolox® equivalents per gram. The same composition gave an antioxidant value using the ORAC(fl) of 5,281 micromoles Trolox® equivalents per gram. These data demonstrate that the ORAC(o) and ORAC(fl) methods of measuring antioxidant activity differ in the results obtained due to the limitations of the indirect ORAC(fl) method.

EXAMPLE 2: A Synergistic Antioxidant Composition.

According to the dietary supplement of the present invention, five ingredients were combined into an antioxidant composition, each of which is prominent in the diet of long-lived peoples from regions around the world. The present inventors selected ingredients based on a comprehensive search of the diets in areas known for longevity. The present inventors recognized that the diets in these regions were rich in flavonols and tocopherols. It was further recognized by the inventors that certain natural compounds interact favorably with molecules that reactivate their anti-oxidant activity, e.g., vitamin C. In fact, extensive research supports the role of Vitamin C in the regeneration of, e.g., $\alpha$-tocopherol (Pizzorno and Murray, Textbook of Natural Medicine, 1999, 2nd Ed. New York, Churchill Livingston).

Based on this research, the inventors combined isolated and purified extracts from natural and other sources that include high percentages of bioavailable compounds from the various regions of long-lived peoples into one formulation. In one example of the present invention, the following basic ingredients were selected: flavonols, mixed tocopherols, grape skin extract, green tea extract, or bush plum are prominent in the diet of peoples of the Andean village of Vilcabamba in Ecuador, the land of Huza in the Karakoram Range in Kashmir, or in Abkhazia in the Georgian State of the former USSR, for example, as cited in Leaf A., Launois J. "A Scientist Visits Some of the World's Oldest People," National Geographic. 1973 January; of the Italian island of Sardinia (Koenig R. "Sardinia's Mysterious Male Methuselahs," Science. 2001, March 16), and of Australia.

The compositions of the present invention demonstrated a synergistic antioxidant activity. Not wanting to be bound by theory, the various ingredients of the antioxidant composition have activity for protecting intracellular cytosol, cellular membranes, and extracellular fluid such that the body is protected throughout. The bush plum component is high, for example, in natural vitamin C content, which can get into the cell, is hydrophilic, and is available for protecting the cytosol; the grape skin extract and green tea extract are hydrophilic, cannot enter the cell and are available for protecting extracellular fluid; and mixed tocopherols are lipophilic, and together with flavonols (e.g., quercetin), which are both hydrophilic and lipophilic, protect membranes. Furthermore, any fiber that is included in the diet, or even in the dietary supplement itself, may provide adequate vehicle for elimination.

Components of the Synergistic Antioxidant Composition. With the availability of the ORAC(o) apparatus and the methods of the present invention, the present inventors were able to measure the total antioxidant activity of combination of both lipophilic and lipophobic anti-oxidants and other agents that aid in potentiating the anti-oxidant activity of these agents, e.g., vitamin-C and the like. Using the ORAC(o) system, an antioxidant composition having synergistic activity was developed that includes flavonoids, e.g., quercetin, a mixture of at least two forms of vitamin E, and optionally, grape skin extract, green tea extract and Australian bush plum. The synergism is particularly observed in a weight ratio of quercetin to the mixture of vitamin E forms of 40/60 to 90/10%. One embodiment of the composition includes the following weight ratios: quercetin, 49.18%; mixed tocopherols, 32.79%; grape skin extract, 9.84%; green tea extract, 6.56%; and bush plum, 1.64%.

FIG. 6 is a graph that shows the results of an ORAC(o) assay comparing quercetin, mixed tocopherols, and mixture of tocopherols and quercetin and the synthetic anti-oxidant standard: Trolox® (Hoffman-La Roche).

Flavonoids such as Quercetin. The flavonoid of the composition can be a flavone, a flavonol, an isoflavone, an isoflavonol, an analogue thereof, a pharmaceutically acceptable salt thereof, or a mixture thereof. Examples of a flavonol include quercetin, kaempferol, and myricetin. The particular flavonoid or flavonoid analogue or salt included in the composition is determined by running an ORAC(o) antioxidant determination. An activity within 80% percent of that of quercetin is contemplated to provide an analogue. Reference to a flavonoid, in particular, quercetin, also is intended to refer to the aglycone or a glycoside thereof where the sugar is arabinose, rhamnose, galactose or glucose, for example. The rhamnose glycoside of quercetin is known as rutin or quercetrin, and the rhamnose glycoside of myricetin is known as myricitrin. Analogues of quercetin include those compounds which comprise a substituting group other than an -OH group at one or more of the positions 3, 5, 7, 3', and 4'. Other substituting groups include: alkyl less than 5 carbon atoms, acetyl, sulphyl, or malonyl. For analogues of quercetin, only one or two of the positions are substituted with anything other than -OH groups.

Flavonoids such as quercetin are readily synthesized *in vitro.* However, flavonoids (including quercetin) are present and may be isolated and purified from, e.g., naturally occurring foodstuffs, in particular, fruits and vegetables, such as apples, pears, grapes, onions, red wine, bell peppers, red currants, black currants, lemons, cherries, cranberries, gooseberries, tomatoes, olives, radishes, kohlrabi, horseradish, potatoes, and asparagus. Quercetin may be obtained from Pharmline (Florida, NY).

Bush Plum: The Australian bush plum (*Terminalia ferdinandiana*) contains about 5% vitamin C and a variety of ingredients as demonstrated by an HPLC chromatogram (data not shown). These ingredients are believed to include flavones and flavonoids. The HPLC chromatogram is from a reverse phase $C_{18}$ column using a stepped gradient of 0.1% trifluoroacetic acid and 100% methanol, at a flow rate of 1mL/min using a sample of methanol and water-extracted bush plum powder. The conditions were developed to separate flavonoids and to separate vitamin C. The absorbance was measured at 245 nm.

Pulp and skin of a bush plum are removed from the seed of the fruit and made into a slurry in water. The slurry is freeze-dried and ground. For antioxidant compositions herein, the freeze-dried material is weighed in the desired amount. Bush plum is present in the composition of the present invention in an amount of from 0% to 87.9%, or in another embodiment, about 2% by weight.

Grape skin extract. Grape skin extract is made from grape skins, and contains 30-82% polyphenols and may be obtained from Polyphenolics, Madera, CA; Hunan Kinglong, Bio-Resource Co. Ltd, Changsha Economic Development Zone, China; or from Pharmline, Florida, NY.

Green tea extract. Green tea extract is an extract from the leaves of *Camellia sinensis,* contains 35-95% polyphenols, and may be obtained from Amax NutraSource Inc., Eugene, OR; Blue California, Rancho Santa Margarita, CA; or from PL Thomas & Co., Morristown, N.J.

Other Ingredients. The antioxidant compositions of the present invention may include one or more, nontoxic, pharmaceutically acceptable carrier such as lactose, starch, sucrose, glucose, methyl cellulose, magnesium stearate, dicalcium phosphate, calcium sulfate, mannitol, sorbitol, cyclodextrin, cyclodextrin derivatives, or the like. Using these one or more carriers capsule or tablets may be easily formulated and can be made easy to swallow or chew. Tablets may contain suitable carriers, binders, lubricants, diluents, disintegrating agents, coloring agents, flavoring agents, flow-inducing agents, or melting agents. A tablet may be made by compression or molding, optionally with one or more additional ingredients. Compressed tablets may be prepared by compressing the active ingredient in a free flowing form (e.g., powder, granules) optionally mixed with a binder (e.g., gelatin, hydroxypropylmethylcellulose), lubricant, inert diluent, preservative, disintegrant (e.g., sodium starch glycolate, cross-linked carboxymethyl cellulose) surface-active or dispersing agent. Suitable binders include starch, gelatin, natural sugars such as glucose or beta-lactose, corn sweeteners, natural and synthetic gums such as acacia, tragacanth, or sodium alginate, carboxymethylcellulose, polyethylene glycol, waxes, or the like. Lubricants used in these dosage forms include sodium oleate, sodium stearate, magnesium stearate, sodium benzoate, sodium acetate, sodium chloride, or the like. Disintegrators include, for example, starch, methyl cellulose, agar, bentonite, xanthan gum, or the like. Molded tablets may be made by molding in a suitable machine a mixture of the powdered active ingredient moistened with an inert liquid diluent.

Capsules or tablets may optionally be coated or scored and may be formulated so as to provide slow- or controlled-release of the antioxidant composition. Timed-release compositions for controlled release of agents generally contain agent particles mixed with or coated with a material that is resistant to enteric degradation or disintegration for a selected period of time. Release of the agent may occur by leeching, erosion, rupture, diffusion or similar actions.

Carriers may promote antioxidant stability as well as providing time release. A mixture of plant carbohydrates termed AMBROTOSE® Phyto Formula may be combined with the antioxidant composition. Such a combination extends the shelf life of the antioxidant composition and provides for a time release form. AMBROTOSE® Phyto Formula contains, in a weight/weight ratio of about 30/30/20/19/1, gum Arabic(acacia), xanthan gum, gum tragacanth, gum ghatti, (which may be obtained from TicGum) and an aloe vera gel extract (e.g., inner leaf gel, Carrington Labs, Irving, TX, MANAPOL® powder or similar product). The AMBROTOSE® Phyto Formula is blended with the antioxidant composition of the present invention in a weight ratio of 2:1 to 1:2. In another embodiment, AMBROTOSE® Phyto Formula is blended with the antioxidant composition in a weight ratio of 2:1.

Capsules or tablets may contain further plant components in weight percentages less than about 0.1% to 90% depending on the specific formulation. In the case of carriers, the carriers themselves will generally have no effect on the nutritional significance to the composition, however, these carriers may have a significant in the timing, location and release profile of the release of the nutritionally effective amounts of the present invention. For example, one or more of the anti-oxidants of the present invention may be released in one or more boluses so as to spike the anti-oxidant levels as detected in, e.g., blood or urine, in a series of release events. In another embodiment, the anti-oxidants may be released somewhat evenly, or may even be provided as a gradient with spikes in blood or urine levels. In fact, the present invention may even include release of the lipophobic and the lipophilic anti-oxidants at different times and or locations during digestion. For example, one anti-oxidant may be provided in an effervescent carrier for immediate release, while a different anti-oxidant is provided for release by, e.g., stomach acid or in the intestinal tract.

Formulation Processes. A process of formulating a roller compacted antioxidant composition comprises blending AMBROTOSE® Phyto Formula with the antioxidant composition set forth herein. The resultant blend is transferred to a roller compactor and compacted between rollers to form a compact. The pressure imparted on the blend enhances the physical adhesion between the ingredients. The compact is subsequently milled to form a granulation. A granulation is then formed into the desired dosage form, such as capsules or tablets. In one example, a Fitzpatrick Chilsonator Model 4LX10D roller compactor may be used with rolls that are notched across the face and perpendicular to the rotation, having a fixed force of 10 ton, and a Fitzmill screen of about 0.093. The roller compaction device may have variable rotation speed, force application, and gap width capabilities, for example, a Gerteis Polygran dry roller compactor system (Gerteis, Germany). The roller compactor functions by uniformly applying pressure on a blend by passing the blend between two counter-rotating rollers. The pressure imparted on the blend by the rollers compresses the blend into a compact, such as a sheet or ribbon, which is typically milled to produce granules. Alternatively, granulation may be achieved by slugging, milling or sieving as may be required. Granules having a #20-80 mesh are selected.

A longer shelf life of the roller compacted combination of the antioxidant composition with AMBROTOSE® Phyto Formula is believed due to the reduction in the amount of surface area of the antioxidant composition exposed to oxygen. The roller compacted combination also eliminates the need for excipient fillers in the capsule or tablet-making process. Additional benefits of a combination of AMBROTOSE® Phyto Formula with the antioxidant composition set forth herein include: provision of non-soluble fiber which may serve as a sink for unpaired electrons in the gut, and provision of monosaccharides for correct structure of cellular glycoforms responsible for cell-mediated communication in repair of cells damaged by free radicals.

Dosage. Useful dosage formulations for administration of the compositions of the present invention include capsules or tablets of 100, 200, 300, 400, 500, 600, 700, 800, 900 or 1000 mg of antioxidant composition. In one embodiment, no fillers, carriers, or stabilizers are added to the composition. In another embodiment, AMBROTOSE® Phyto Formula is blended with the antioxidant composition of the present invention in a weight ratio of 2:1, 1:1, or 1:2. In another embodiment, AMBROTOSE® Phyto Formula is blended with the antioxidant composition in a weight ratio of 2:1. In another embodiment, a capsule or tablet provides 500 mg of antioxidant composition blended with AMBROTOSE® Phyto Formula. For this embodiment, two tablets or capsules may be taken per day. Appropriate coatings may be applied to increase palatability or delay absorption.

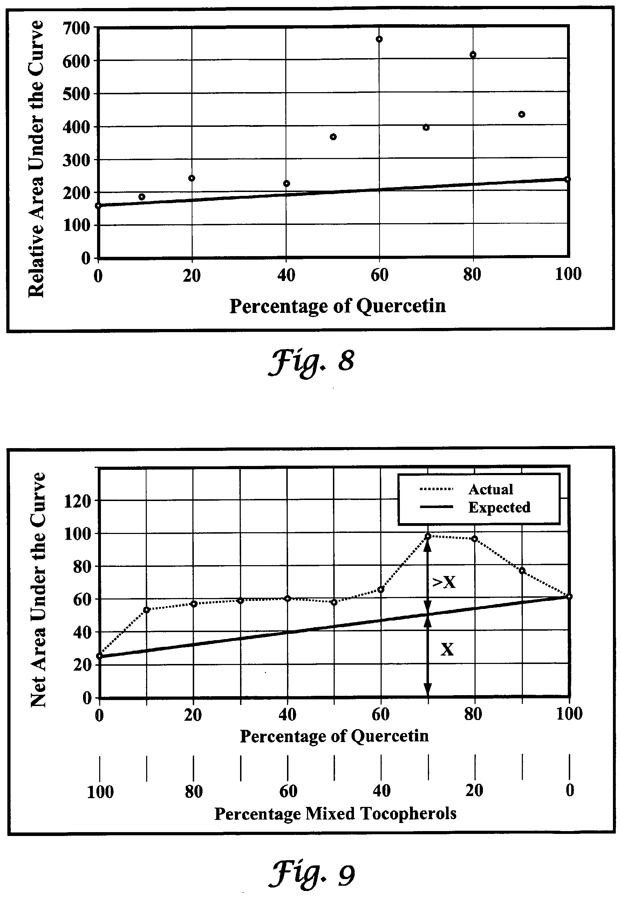

FIG. 8 and FIG. 9, show the net area under the curve (AUC) results for varying ratios of quercetin and mixed tocopherols using the ORAC(o) method to measure antioxidant capacity. The total concentration of sample (Q + MT) is 10 µg/mL for each percentage assayed. For example, at 0% quercetin, the concentration of mixed tocopherols is 10 µg/mL, and there is no quercetin in the sample. At 10% quercetin, the sample has 1 µg/mL of quercetin and 9 µg/mL of mixed tocopherols. At 20% quercetin, the sample has 2 µg/mL of quercetin and 8 µg/mL of mixed tocopherols. The synergy is readily observed between 40 and 100% quercetin, and most readily observed from 40 to 90% quercetin. The straight line represents the additive effect, i.e., at 10% quercetin, the line represents the sum of 90% of the activity of mixed tocopherols alone and 10% of the activity of quercetin alone. A synergistic effect is found above this line.

The primary anti-oxidant ingredients of the composition of the present invention (flavonoids, as represented by quercetin, and a mixture of at least two forms of vitamin E) comprise from 12.1% to 100%, 30% to 85%, or in another embodiment, about 82% by weight of the five ingredients. In one embodiment, the amount of quercetin is 49.18%, and the amount of vitamin E forms is 32.79% of the total weight of the five ingredients.

The secondary ingredients (or potentiators) of the composition of the present invention (grape skin extract, green tea extract, and bush plum) comprise from 0% to 87.9%, 15% to 70%, or about 18% by weight of the five ingredients. As shown in FIG. 10, the optimal ratio of grape skin extract and green tea extract was determined in the presence of 49.18% quercetin, 32.79% mixed tocopherols, and 1.64% bush plum. The optimal ratio of grape skin extract to green tea extract is 60/40 to 80/20. In one embodiment, the grape skin extract is 9.84% and the green tea extract is 6.56 % of the total weight of the antioxidant composition. Bush plum (*Terminalia ferdinandiana*) is provided as an ingredient of the composition in an amount from between about 0% to 87.9%, or in another embodiment of about 2%. In the embodiment of FIG. 10, the bush plum is 1.64 % of the composition.

The ORAC(o) assay shows that an antioxidant blend having the following weight ratios: quercetin, 49.18%; mixed tocopherols, 32.79%; grape skin extract, 9.84%; green tea extract, 6.56%; and bush plum, 1.64% has an antioxidant activity of 17,254 micromoles Trolox® equivalents per gram.

Stability of AMBROTOSE AO®. AMBROTOSE AO®, a blend of AMBROTOSE® (Mannatech, USA) and the antioxidant formulation of the present invention at a 2:1 weight ratio, has been shown to maintain its activity under accelerated stability conditions (40°C at 75% relative humidity) equating to a shelf-life of roughly six months.

Validation of the ORAC(o) assay as compared to the ORAC(fl) assay. FIG. 11 is a graph of ORAC(fl) results that shown no contribution to AO activity by α-tocopherol when mixed at different ratios with quercetin. The graph shows a linear increase in AUC that is directly proportional to increase in quercetin concentration in the ORAC(fl) assay in the standard acetone:water solution. The lack of contribution to AO activity may be due to the failure of the α-tocopherol to dissolve in acetone:water.

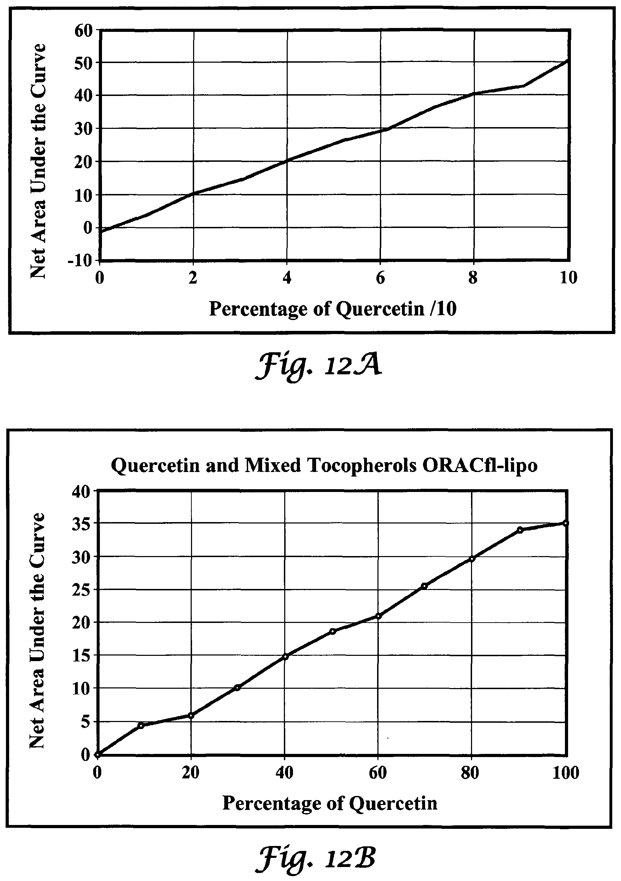

FIGS. 12A and 12B are graphs of ORAC(fl-lipo) results using mixed-tocopherol and the relative AUC results using the solvent/water/detergent solution to dissolve the sample and detect both lipohilic and lipophobic anti-oxidant capacity. In FIG. 11 (above) it was shown that no statistically significant activity could be detected for α-tocopherol using the standard ORAC(fl) method for detecting AO contribution of lipophilic AOs. As shown in FIG. 12A, the ORAC(fl-lipo) AUC for α-tocopherol was compared between the published ORAC(fl-lipo) method (acetone:water) and an acetone:water:Tween-20 and a large increase in the AUC of the ORAC(fl-lipo) assay was detected. FIG. 12B demonstrates that there is no synergy detected using the known ORAC(fl-lipo) method when combining quercetin and mixed-tocopherols as the results follow a straight line through the abscissa, which indicates lack of synergy using this assay.

Obtaining these results with known assays are not surprising based on a search of the literature and the methods recommended therein. Current standards for the evaluation of so called "High-ORAC Foods" (see, e.g., www.ars.usda.gov) indicate that the standard is to measure the anti-oxidant potential of lipophilic apart and separate from lipophobic anti-oxidants. The present invention eliminates the need for this dichotomy, a dichotomy that likely results in the disconnect researchers have observed when relating test-tube ORAC values to the effect on serum ORAC values. When measuring the anti-oxidant effects in serum as compared to an ORAC (fl) evaluation, the Agricultural Research Service of the USDA found that, e.g., spinach scored lower than strawberries in the test-tube ORAC assay, but spinach had a greater effect on serum ORAC values than strawberries. Using the present invention and the compositions described herein, the present inventors have remedied the disconnect by developing a system that allows interaction among antioxidant with varying solubilities, which better reflects the activity of antioxidants within the human body. The indirect ORAC(fl) and/or ORAC (fl-lipo) methods fail to measure total anti-oxidant activity and fail to detect the synergistic effects of certain foods.

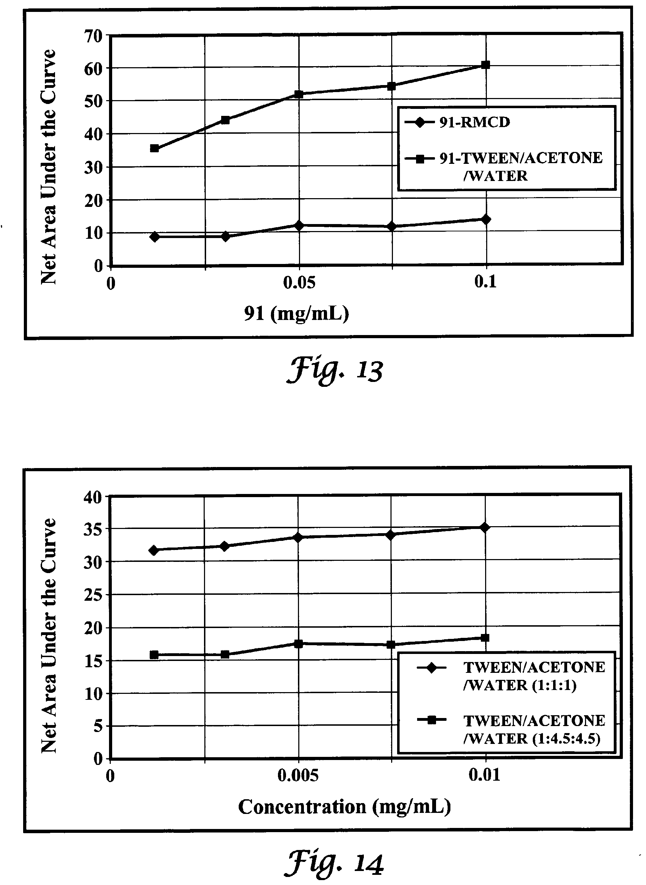

FIG. 13 is a graph of ORAC(fl) AUC results using α-tocopherol with a variation in the amount of detergent used to show its effect on AUC measurements. In this assay, the one third solvent was used in its full one third and in a mixture with a smaller amount of Tween-20. FIG. 14 is a graph of an ORAC(fl) assay measuring the anti-oxidant activity measured for a fixed ratio of quercetin:α-tocopherol ratio dissolved in two different rations of solvent:water to detergent mixtures. The results in FIG. 13 and FIG. 14, show that the detergent (Tween-20) did account for increase in AUC and therefore shows that detection of ORAC(fl) activity is increased as compared to the standard acetone:water mixture.

EXAMPLE 3. ORAC(o) Detection of Anti-oxidant Synergy and Potentiation.

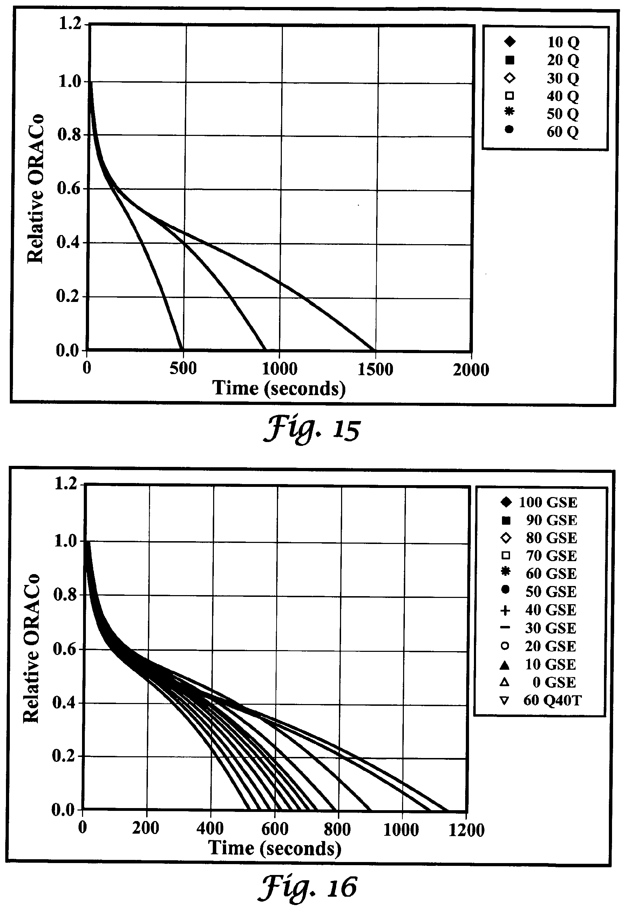

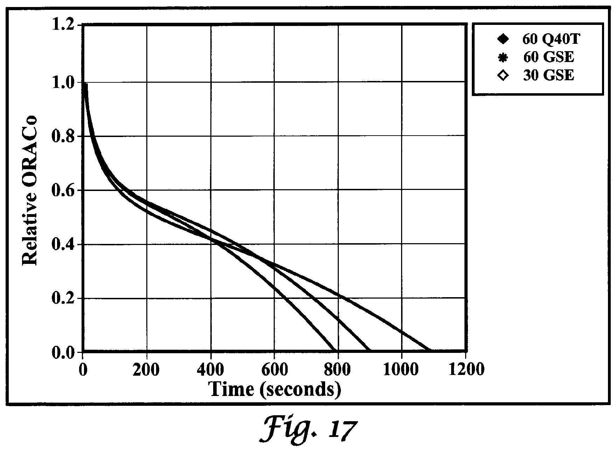

To show that the ORAC(o) assay was able to detect both lipophilic and lipophobic anti-oxidant activity, a series of studies were conducted to show the effect of specific combinations of known concentrations of lipophilic and lipophobic anti-oxidant and the potentiation of the same with the addition of, e.g., grape seed extract and green tea extract as compared to grape seed extract and green tea extract alone. FIG. 15 is a graph showing the ORAC(o) values obtained with different ratios of quercetin and mixed tocopherols. FIG. 16 is a graph showing the ORAC(o) values for different ration of grape seed extract (GES) and green tea extract (GTE). After the determination of the maximum ratio for anti-oxidant activity for GSE and GTE, the two were combined with the optimized ratio for quercetin and mixed tocopherols. FIG. 17 is a graph that shows the ORAC(o) values of the combination of the maximum quercetin:mixed tocopherol and the grape seed extract and green tea extract ratios. It was found that the combined quercetin and mixed tocopherols, GSE, GTE maximized the anti-oxidant potential of the supplement.

EXAMPLE 4: Antioxidant Effect of AMBROTOSE AO® in a Small Number of Healthy Individuals, Open Label Study, Non-Placebo controlled.

An antioxidant is defined as any substance that can delay or prevent the oxidation of biological substrates. Diets rich in fruits and vegetables that contain antioxidants have been associated with decreased incidence of pathological conditions thought to result from oxidative stress. However, supplementation with isolated antioxidant compounds has often proven ineffective in improving health, in some cases, and dangerous in others.[1,3,4] It has been suggested that the antioxidant benefits of a high intake of fruits and vegetables may not result exclusively from single antioxidants but rather from a concerted action of a combination of different antioxidants present in these foods.[5,6] This focus on single antioxidants is a possible explanation as to why scientists have yet to duplicate the protective effects of an antioxidant-rich diet using nutritional supplementation.[7]

Recent studies have suggested that glyconutritional (GN) supplements, nutritional supplements providing sugars that support cellular communication and immune function, have antioxidant properties both *in vitro* and *in vivo.* Rat liver cells grown in culture medium containing GN supplements showed higher levels of reduced glutathione relative to controls, thereby demonstrating increased antioxidant protection.[8]

A pilot clinical study demonstrated a reduction in biomarkers of oxidative stress and an increase in biomarkers of oxidative defense in people consuming GN supplements. Significant increases were observed in total iron-binding capacity and folic acid, and a significant decrease was observed in copper/ceruloplasmin ratio within 76 days following the daily addition of 2 gr GN supplement to the normal diet. Trends were also observed which suggested a decrease in serum alkenals, homocysteine, 8-hydroxydeoxyguanosine (8-OHdG) and total cholesterol, and an increase in oxygen radical absorbance capacity ($ORAC_{\beta-PE}$).[9]

Recent studies have demonstrated that a wide range of nutrients (some known, others not yet identified) account for the antioxidant activity of fruits and vegetables.[10,11,12,13,14] Some of the known beneficial antioxidants include polyphenols, and vitamins C and E.[6] Certain foods and beverages, including grapes (and red wine), green tea, and the Australian bush plum (*Terminalia ferdinandiana*), contain relatively high levels of such antioxidants.[6,15,16] This study measured the antioxidant protection provided by a novel nutritional supplement that combined a GN supplement with a blend derived from green tea, grapes, mixed tocopherols, quercetin and the Australian bush plum.

Methods: Blood samples were collected by Cover-Tek, Inc., Dallas, TX. All blood and urine samples were analyzed by Genox Corporation, Baltimore, MD, and all statistical analyses were performed by Decision Analyst, Arlington, TX.

A number of methods for testing the antioxidant potential of samples have been published.[17] The test used in this study was the measurement of whole serum $ORAC_{\beta-PE}$. This method uses β-phycoerythrin, a fluorescent probe, to determine the antioxidant scavenging capacity of serum samples, specifically against peroxyl radicals, when compared to a known standard, Trolox®. The results are expressed in micromoles Trolox Equivalents (TE) per gram.[18] Other standardized analytical techniques used in the study were the measurement of urine lipid hydroperoxides and alkenals, which relate indirectly to the amount of free radical damage to lipids, and urine 8-OHdG, which relates indirectly to DNA damage.[19,20,21]

The antioxidant nutritional supplement used in this study, Ambrotose AO®, was developed using an in *vitro* evaluation of ingredients: quercetin, mixed tocopherols, grape extract, green tea extract, the Australian plum. The *in vitro* antioxidant values of each ingredient were determined using the standard $ORAC_{fl}$ method (which uses a different fluorescent probe, fluorescein). The antioxidant values of the ingredient mixtures were determined using a newly developed method that measures dissolved oxygen directly, the $ORAC_o$. Since lipid- and water-soluble antioxidants work in concert *in vivo,* the $ORAC_o$, which simultaneously measures the contributions and interactions of lipid- and water-soluble compounds, is an improvement over fluorescence-based methods ($ORAC_{fl}$, $ORAC_{fl-lipo}$ and $ORAC_{\beta-PE}$). These methods, at best, measure the activities of lipid- or water-soluble compounds alone. $ORAC_o$ therefore provides a more accurate determination of the total *in vitro* antioxidant activity of a blend of both water- and lipid-soluble ingredients. The water and lipid-soluble ingredients were combined and evaluated with the $ORAC_o$ to establish maximal synergy and the optimal *in vitro* $ORAC_o$ value of the blend.

The subsequent blend was roller-compacted with Ambrotose® complex at a ratio of 1:2 to create Ambrotose AO®. Many of the natural gums in Ambrotose® have been used to control the release of compounds providing a sustained delivery. This study was designed to determine the *in vivo* antioxidant activity, using standardized tests, of different amounts of Ambrotose AO® as determined directly by serum $ORAC_{\beta-PE}$ and indirectly by urine lipid hydroperoxide, alkenal and 8-OHdG analyses. Prior and Cao recently suggested that a battery of tests, rather than any one single test, is necessary because some pathological conditions, such as renal failure, can alter any one test quite unrelated to oxidative stress.[22]

Informed consent was obtained from all participants. Twelve healthy male and female adult volunteers taking no nutritional supplements or any drug that would interfere with the study were enrolled. The study subjects, four males and eight females ages 22-61, consumed increasing amounts of the antioxidant supplement. To evaluate $ORAC_{\beta-PE}$ variability over time and to test the reproducibility of the laboratory results, a blood and urine sample was collected from an additional subject who was taking no supplements. During the study, participants continued their normal pre-study daily routines and diets.

Table 5 provides information about the study subjects.

| Table 5. Characteristics of Human Volunteers | | | |
|---|---|---|---|
| Subject | Gender | Age | Tobacco User |
| 1 | F | 33 | Yes |
| 2 | M | 41 | No |
| 3 | F | 34 | No |
| 4 | F | 61 | Yes |
| 5 | F | 44 | Yes |
| 6 | M | 22 | No |
| 7 | F | 36 | No |
| 8 | M | 23 | No |
| 9 | F | 38 | Yes |
| 10 | F | 56 | No |
| 11 | F | 53 | No |
| 12 | M | 31 | Yes |

The twelve study subjects had morning fasting serum $ORAC_{\beta-PE}$ and urine analyses performed after an initial washout period of 2 weeks on no supplements and at the end of 2 weeks on each increasing amount of the antioxidant supplement. The amounts used were 500 mg (1 capsule) each day for the first 14 days of supplement use (Period 1), 1.0g (2 capsules) each day for the second 14-day period (Period 2), and 1.5 g (3 capsules) each day during the third 14-day period (Period 3). Additionally, a blood and urine sample from the individual not consuming supplements was analyzed in triplicate to test the precision of the analyses. Blood and urine samples were collected by the independent phlebotomist, immediately packed in dry ice and transported to a local hospital laboratory for preparation. Prepared serum and urine samples were then shipped in dry ice overnight to Genox for independent analysis per the Genox protocol.[23] All samples were then stored at Genox in dry ice, and $ORAC_{\beta-PE}$ and urine measurements were all done at the same time at the conclusion of the study to minimize any analytical reagent variability.

Results: ORAC Values and Percent Change from Baseline Data. The $ORAC_{\beta-PE}$ values and percentage change from baseline for the twelve participants taking Ambrotose AO® are given in Table 6. Baseline is after the 2-week washout, Period 1 after 2 weeks on 500 mg, Period 2 after 2 weeks on 1.0 g and Period 3 after 2 weeks on 1.5 g. Serum vial labels of some Period 2 samples became detached during shipping to Genox. $ORAC_{\beta-PE}$ values could only be specifically assigned to three of the study participants for this period and not for the rest of the study participants or the additional non-study individual.

**Table 6. ORAC$_{\beta\text{-PE}}$ Values and Percent Change from Baseline**

| Subject | ORAC | | | | % Change from Baseline | | |
|---|---|---|---|---|---|---|---|
| | Baseline | Period 1 | Period 2* | Period 3 | Period 1 | Period 2 | Period 3 |
| 1 | 3300.3 | 5709.4 | | 3117.8 | 73.0% | | -5.5% |
| 2 | 3754.5 | 4618.6 | 6731.8 | 6189.0 | 23.0% | 79.3% | 64.8% |
| 3 | 3695.0 | 3867.9 | | 3995.7 | 4.7% | | 8.1% |
| 4 | 3954.3 | 3199.1 | | 2703.8 | -19.1% | | -31.6% |
| 5 | 3107.3 | 3295.2 | | 4476.2 | 6.0% | | 44.1% |
| 6 | 3313.0 | 5073.8 | | 3156.6 | 53.1% | | -4.7% |
| 7 | 3785.5 | 5069.9 | | 4390.4 | 33.9% | | 16.0% |
| 8 | 3341.2 | 4207.7 | | 4003.2 | 25.9% | | 19.8% |
| 9 | 7568.9 | 6053.2 | 8977.6 | 7734.1 | -20.0% | 18.6% | 2.2% |
| 10 | 4271.8 | 6132.5 | | 6765.0 | 43.6% | | 58.4% |
| 11 | 5619.2 | 6527.0 | 6420.0 | 6844.3 | 16.2% | 14.3% | 21.8% |
| 12 | 5642.9 | 5025.4 | | 4398.7 | -10.9% | | -22.0% |

Statistical Analysis of Raw ORAC Data. A repeated-measures ANOVA was conducted to determine if there was a difference among the Baseline, Period 1, Period 2, and Period 3 raw ORAC data. There were significant differences among the time periods overall ($F(3,24) = 4.02$, $p = .020$). The *post hoc* analyses revealed that Period 2 was significantly different from Baseline ($t(24) = -3.47$, $p = .002$). Period 1 was significantly different from Period 2 ($t(24) = -2.77$, $p = .011$). Period 2 was significantly different from Period 3 ($t(24) = 2.87$, $p = .009$). The mean for each time period is shown in Table 7.

**Table 7. Mean ORACbeta-PE Values for Each Time Period**

| Time Period | Number of Subjects | Mean ORAC | Score |
|---|---|---|---|
| Baseline | 12 | 4279.5 | 847.9 |
| Period 1 | 12 | 4898.3 | 699.1 |
| Period 2 | 3 | 7376.5 | 3466.6 |
| Period 3 | 12 | 4814.6 | 1053.1 |

Statistical Analysis of Percent Change from Baseline ORAC Data. In contrast to the raw data used in the analysis reported above (Table 6), this analysis examined differences among the time periods expressed as percent change from baseline. A repeated-measures ANOVA was conducted to assess differences among the percent change from Baseline of Period 1, Period 2, and Period 3 ORAC data. The omnibus test yielded no significance overall ($F(2,13) = 0.71$, $p = 0.510$). Additionally, the *post hoc* analyses revealed no significant differences among the periods. The mean for each time period is shown in Table 8.

**Table 8. ORACbeta-PE Percent Change from Baseline by Time Period**

| Time Period | Number of Subjects | Mean of Percent Change | ORAC Score ± |
|---|---|---|---|
| Period 1 | 12 | 19.1% | 18.1% |
| Period 2 | 3 | 37.4% | 90.3% |
| Period 3 | 12 | 14.3% | 19.0% |

The average lipid hydroperoxide, total alkenal and 8-OHdG values are summarized in Table 9. They are corrected for urine concentration variability by dividing by urine creatinine as measured at the same time on the same sample.

| Table 9. Average Urine Biomarkers for Each 2 Week Time Period | | | | |
|---|---|---|---|---|
| Time Period | Number of Subjects | Hydroperoxide/creatinine $\mu$M/(mg/dl) | Alkenal/creatinine $\mu$M/ (mg/dl) | 8-OHdG/creatinine (mg/ml)/ (mg/dl) |
| Baseline | 12 | 0.0920 | 0.0761 | 0.0724 |
| Period 1 | 12 | 0.0808 | 0.0808 | 0.0861 |
| Period 2 | 12 | 0.0782 | 0.0835 | 0.0740 |
| Period 3 | 12 | 0.0764 | 0.0806 | 0.1025 |

Air Quality. Air quality is known to affect levels of oxidative stress. Increasing concentrations of common pollutants, such as ozone and nitrogen dioxide, decrease air quality. This leads to a greater potential for the generation of ROS.[24,25]. A summary of the average U.S. Environmental Protection Agency (EPA) air quality indexes for each two-week period in the Dallas/Fort Worth (DFW) area, where the subjects lived, is given in Table 6. The EPA uses color codes for area air quality maps: Green: "Good" (0 - 79 parts per billion [ppb] ozone), Yellow: "Moderate" (80 - 99 ppb), Orange: "Unhealthy for Sensitive Groups" (100 - 124 ppb), Red: "Unhealthy"(125 - 149 ppb) and Purple: "Very Unhealthy" (greater than 150 ppb). A numerical value for each daily EPA map of the study area was calculated by assigning numbers to the colors: Green: 1, Yellow: 2, Orange: 3, Red: 4 and Purple: 5 and estimating the numerical average for each day based on the area of each color on the published EPA maps. The daily numerical values were then averaged for each two-week period of the study (Table 10).

| Table 10. Average DFW EPA Air Quality Index for Each Time Period | |
|---|---|
| Time Period | Average EPA Air Quality Index |
| Baseline | 1.0 (range 1.0-1.0) |
| Period 1 | 1.0 (range 1.0-1.1) |
| Period 2 | 1.4 (range 1.0-2.5) |
| Period 3 | 1.8 (range 1.0-2.5) |

Allowing for the uncertainty in assigning values to the study participants in Period 2 as outlined above, the lowest possible average $ORAC_{\beta\text{-PE}}$ for the 12 study participants was calculated. Assuming that the lowest 9 values from the serum samples whose labels could not be identified belonged to the participants and combining them with the 3 known values yields the lowest possible average and thus the most conservative estimate of change from baseline. The 9 lowest $ORAC_{\beta\text{-PE}}$ values were, 4727.9, 5233.9, 5104.3, 4599.9, 5699.9, 5364.8, 3987.3, 4179.7 and 4584.9. When combined with the 3 known Period 2 values from Table 5, this gave an average $ORAC_{\beta\text{-PE}}$ value of 5467.70 for the 12 study participants in Period 2. This is 27.8% above the baseline average of 4279.49.

Discussion. Studies have shown that the consumption of diets rich in fruits and vegetables is protective against oxidative stress.[7,26,27] Dietary guidelines advocate the daily consumption of 2-4 servings of fruit and 3-5 servings of vegetables.[28] Despite this knowledge and these recommendations, very few individuals in the U.S. consume the recommended daily amounts. In a U.S. Department of Agriculture (USDA) sponsored clinical study, researchers found that increased consumption of fruits and vegetables, specifically from the usual five to an experimental ten servings a day, significantly increased serum $ORAC_{\beta\text{-PE}}$ values by up to approximately 13% after two weeks.[7] In the current study, the increase in average serum $ORAC_{\beta\text{-PE}}$ values using each amount of supplement were: 19.1% with 500 mg, 37.4% with 1.0 g, and 14.3% with 1.5 g. These data suggest that the optimal amount that results in the greatest rise in serum $ORAC_{\beta\text{-PE}}$ over baseline in healthy people is 1.0 g. The conservative estimate of a 27.8% percent increase with 1.0 g Ambrotose AO® is more than twice that the published 13% reported individuals consuming five additional fruits and vegetables daily.

The urine lipid hydroperoxide/creatinine values decreased with increasing supplement use. The decrease for each amount was 12.1% with 500 mg, 15.0% with 1.0 g, and 17.0% with 1.5 g, suggesting that protection from lipid damage increased with increasing amounts of supplement over the range studied. It is unclear why the lipid hydroperoxide data do not correlate exactly with the $ORAC_{\beta\text{-PE}}$ values. Serum $ORAC_{\beta\text{-PE}}$ is a measure of the antioxidant protection of the blood in regard to its ability to quench free radicals at the time of the measurement. Urine lipid hydroperoxides are a marker of lipid oxidative damage at some time in the past. The temporal relationship between the actual lipid damage and the appearance of lipid hydroperoxides in the urine is not well defined. It may well be that these temporal differences account, in part, for this variance. In addition to urine lipid hydroperoxides, urine 8-OHdG and alkenals were also measured at each time period. No significant differences, nor trends were observed. This again may relate to the temporal relationship between actual lipid and DNA damage and the appearance of the biomarkers in the urine.

Participants in the study lived in the DFW area. The published EPA daily air quality assessments for DFW were averaged for each 2-week period. Air quality was getting worse over the course of the study. This would normally be expected to increase oxidative stress by increasing ROS and hence increasing biomarkers of oxidative damage, such as lipid hydroperoxides.[29] On the contrary, increased protection was evident as measured by increased serum ORAC values. In addition, a downward trend in urine lipid hydroperoxide values and the stability of urine 8-OHdG and alkenals provides evidence of decreased oxidative damage. Taken together, these data suggest that the antioxidant effects afforded by the supplement could have been greater than the effects that were actually measured.

Conclusions. While it is recommended that individuals consume fruits and vegetables according to published guidelines, the reality is that the vast majority will not. These preliminary data suggest that a nutritional supplement containing optimal antioxidant ingredients that preserve antioxidant activity in the finished product increases antioxidant protection in healthy individuals as measured by serum $ORAC_{\beta-PE}$ and decreases lipid oxidative damage as measured by urine lipid hydroperoxides. The protection in healthy people as demonstrated by the increase in serum $ORAC_{\beta-PE}$ over baseline after two weeks was greater using 500 mg, 1.0 g and 1.5 g of Ambrotose AO® than that documented in published data with the addition of five fruits and vegetables to the diet for two weeks (19.1%, 37.4% and 14.3% vs. 13%).

As shown herein, antioxidant supplements were examined using four measures of oxidative stress in healthy people and an increase in serum $ORAC_{\beta-PE}$ was found. While the optimal range among healthy subjects as suggested by $ORAC_{\beta-PE}$ values was 1 g per day, research has shown that individuals with low serum ORAC values may benefit from high-dose antioxidant supplementation.[30] Hence, those who suffer from increased oxidative stress or otherwise stressed individuals may benefit from larger doses.

While the $ORAC_o$ was used in the antioxidant blend formulation, an independent laboratory used an established method, the $ORAC_{\beta-PE}$, to analyze human clinical trial plasma samples. *In vivo* assessment of antioxidant effectiveness of the Ambrotose AO® product did not rely on the use of the $ORAC_o$ method. These data demonstrate that the present antioxidant supplement increases antioxidant protection in consumers as measured by serum ORAC($\beta$-PE) and decreases lipid oxidative damage as measured by urine lipid hydroperoxides.

Example 5. Polysaccharide material as a time releasing agent for bioactive molecules when roller compaction with or above 10,000 psi is implemented. The present inventors recognized that certain formulations and methods developed to study the disintegration of its formulations that use, e.g., Ambrotose AO®, failed to release as expected, i.e., immediate release of the components, e.g., the anti-oxidant quercetin. The study began with the use of well-known, formal analytical techniques used to process all of the examples shown here. The method contains two levels of detail because it was initially designed to be fully quantitative, and measure the anti-oxidant quercetin as the analyte. Initially, visual interpretations of components other than quercetin indicated an unexpected release profile, i.e., the dissolution conditions under which the comparisons were made were identical but the actual results were not.

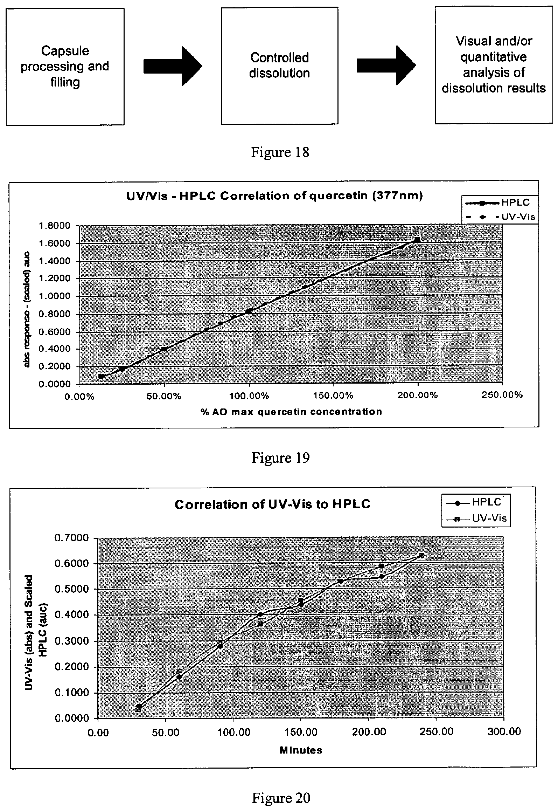

Figure 18 shows the three modules selected for initial investigation. Four separate samples were tested in three steps for: quercetin, grape skin, and two other products (anti-oxidant Formula A and anti-oxidant Formula B). Note that the "Controlled dissolution" step was the same for all examples. Briefly, the dissolution based methods for Ambrotose AO® modified release substantiation included HPLC and UV-Vis using quercetin as the marker. Furthermore, visual observations of the formulations were consistent with the HPLC and UV/Vis observations.

The current specifications for dietary supplement release qualification in many countries includes provisions for the complete disintegration of tablet or capsule product forms in an apparatus that roughly simulates the physical conditions of the gut. These conditions apply to both United States Pharmacopoeia (USP) and British Pharmacopoeia (BP) based specifications. Disintegration testing is best described as simulating the supplement material's physical availability to interact with the digestive system. The conditions under which a component is introduced into the body influences the speed and efficiency of its availability. The speed and efficiency of nutrient availability may be described as an "availability curve." A narrow and intense curve could be representative of an intravenous shot for example, while fibrous bound nutrients such as those found in foodstuff that have to be digested before they become available would have a broader curve due to more gradual release. Depending on the desired behavior, a distribution model can be developed using both fast-acting as well as time-releasing carrier media.

The dissolution profiles of active agents in a sustained-release liquid formulation of the present invention were tested using, e.g., a standard low pH or a water dissolution profile assays. Briefly, the roller compacted Ambrotose® was tested for percent release by dissolution in a 37 degree Celsius water bath in one liter of deionized water into which 5.0 grams of potassium chloride was dissolved with paddles that mix the sample at 150 revolutions per minute (RPMs). A 5 milliliter test sample of the liquid formulation, neat or titrated, is added to the bath using a syringe (which may be washed in the water/KCl mix). Aliquots of 2 ml may be sampled at, e.g., 0.5, 1, 3 and 8 hours. Alternatively, the dissolution profile may be tested by dissolving the dosage forms using a Distek Model 2100B dissolution bath configured as USF Apparatus II (paddles). The paddle rotation was set at 50 rpm. The dissolution medium may be, e.g., 900 ml of pH 6.8 phosphate buffer at 37°C.

Unfortunately, the current accepted approach to disintegration testing, which follows the USP and BP guided specifications of total visual disintegration within thirty minutes, is deficient in accurately assessing the availability of supplements with more gradual curves which take longer to become accessible to the gut. The Ambrotose AO® capsule was designed specifically for sustained release and gradual availability, and does not completely disintegrate within the thirty minute window. This should not be seen as a failure of Ambrotose AO® to become available in the allotted runtime, rather a bias in the current method toward fast-acting components. The USP and BP disintegration requirements indirectly force a relatively narrow availability. In some applications this is unwanted or potentially toxic depending on the component involved. Provided is a simple method using dissolution, UV-Vis, and HPLC technologies to show the desired extended release activities of Ambrotose AO®. The prime goal is to qualify the UV-Vis alone as sufficient in measuring this activity, with the more involved HPLC method simply used to correlate with the UV-Vis performance.

Methods. Quercetin was chosen as the analyte in this method due to its high UV-Vis activity at 377nm, relatively high composition percentage in Ambrotose AO®, and well-behaved chromatography. A standard material of the same quercetin stock used in the formulation of Ambrotose AO® was used to provide a reference curve within the concentration range expected from the dissolution test. It should be noted that an Australian lot of Mannatech Ambrotose AO® was used in this study as the initial charge was to justify that formulations failure of TGA administered disintegration tests per the BP.

The dissolution conditions were modeled after both USP and BP for similar assays. One exception is that a higher than average rotation speed for the dissolution apparatus was chosen to keep the analysis time reasonable while still maintaining sound results. Dual measurement techniques were utilized to correlate the results although in practice a single method may be chosen, and the simpler UV-Vis method alone would be ideal. Observations of the failed 30 minute disintegration test were performed on the same test samples. Next, the availability curve of Ambrotose AO® based on a quercetin marker was plotted over 0.5, 1, 2, 3, 4 and 8 hours within a specified dissolution conditions. This simple and consistent method can be repeated by any properly-equipped laboratory, and could be translated into a quality assurance environment for release testing if necessary.

Equipment. Dissolution system: A Hanson dissolution system SR8-Plus was utilized for this method. It was run using the USP apparatus type II (paddle) configuration with the paddle depths calibrated to the given specifications. The dissolution media, based on similar assays, was 800 ml of deionized (d.i.) water (18+ Mohm) per basket, a bath temperature of 37°C, and paddle speed of 200 rpm. For the sampling a 1ml pipette is needed along with 12 x 75mm glass test tubes (or equivalent), and methanol for dilutions in preparation of analysis in the instruments. The dilution of the samples, which is specified in the procedure, places the maximum quercetin concentration in the test bath within an optimal range for analysis on both the UV-Vis and HPLC.

UV-Vis spectrophotometer: A UV-Vis spectrophotometer (Cary 50 Bio, Varian), along with a quartz glass cuvette were used for measurements. Single wavelength readings were taken at 377nm. The same quartz cuvette was used for all readings, and the instrument was zeroed using the initial $T_0$ samples from each dissolution test bath respectively. In preparation of the UV-Vis readings, the system was checked for the ability to maintain a consistent zero as well as readings at the desired 377nm.

HPLC system. A Shimadzu *LC-VP* binary pump HPLC system was used for measurements. The detector utilized was a Shimadzu PDA SPD-M10A monitored at 377 nm with a 6.25 Hz scanning rate. The column used was a Phenomenex Hydro RP, 4.6mm x 150mm, with 5um particle size. The column and PDA flow cell were maintained at a temperature of 30°C. Injections of 20ul were made for all samples and standards. All samples and standards were passed through a 0.20uM CA filter (Whatman) prior to injection on the HPLC. Integrations were automated for consistent peak generation. The Shimadzu VP 7.2 version software package was used to control the HPLC as well as integrations. Following is the gradient program used in the analysis. Solvent A was 0.1% formic acid in deionized (d.i.) water, and solvent B was 0.1% formic acid in methanol. All solvents were degassed prior to system equilibration, and the total runtime for the analysis was 30 minutes. Quercetin peaks can be expected to elute around 14.8 minutes given these conditions.

Table 11. Gradient program for Quercetin HPLC system

| Minutes | % Concentration B |
|---|---|
| 0.00 | 50 |
| 5.00 | 50 |
| 20.00 | 70 |
| 20.01 | 85 |
| 22.00 | 85 |

(continued)

| Minutes | % Concentration B |
|---------|-------------------|
| 22.01 | 50 |
| 30.00 | 50 |

Standards and Samples. A range of standards for both UV-Vis and HPLC were created using serial dilutions of Pharmline quercetin HD (lot# 152560). Standards were given a concentration range correlating to a single dissolved Ambrotose AO® capsule in an 800ml volume of d.i. water (~0-100ppm). All samples were obtained from the same bottle of AUS Ambrotose AO® (lot# N04081226) which was accepted for release providing an acceptable test lot. Both the standards and test samples were processed in an identical manner being first dissolved in d.i. water, then being sampled as described in the procedure and diluted 1:3 with methanol prior to analysis on the respective instruments.

After the conditions of the dissolution system have stabilized, the Ambrotose AO® capsules are added one per bath. It is suggested to run a minimum of three baths to get an accurate representation of the dissolution activity. The moment the capsules are introduced into the bath becomes time $T_0$, and all readings for the quercetin availability curve are measured relative to this initial $T_0$ reading. Samples are then taken at half-hour intervals and at a volume of 1 ml. In addition a 1ml volume of d.i. water is replaced in the bath after sampling to maintain the controlled volume. An initial sample is taken for $T_0$ as soon as capsules are introduced into the bath. Samples then continue to be taken up to 4-6 hours from $T_0$ until it is apparent that dissolution of the capsule is complete. Once all samples have been taken they are then diluted 1:3 with methanol and shaken in their tubes to ready them for analysis. For the quercetin standard a 100ppm solution is made in d.i. water, and serial dilutions are generated from that stock. The standard dilutions are then sampled at 1ml volumes and diluted 1:3 with methanol and shaken in the same fashion as the samples to ready them for analysis. A minimum of five serial dilution points is taken with the standard over the anticipated range of the quercetin concentration.

The photometer is zeroed using the quartz cuvette (the same which is used for all readings) filled with $T_0$ from the sample bath being measured, or 75% methanol in d.i. water in the case of standard curve measurements. The quartz cuvette is rinsed with a volume of d.i. water to remove any residual quercetin between readings. The endpoint of the dissolution is defined as the point in time in which an increase in UV-Vis activity of the sample is no longer detected after two consecutive readings (one hour time). This "plateau" marks the point of full quercetin dissolution from the capsule. If during the UV-Vis readings there is a rogue value due to a particle blocking the light source path etc., a Q test is used to disregard the reading and have it taken again. It is apparent when this occurs as the reading remain, normally, rather constant. A minimum of three readings is taken per sample to get an acceptable average. After UV-Vis measurements have been taken, the samples are filtered and run individually on the HPLC conditions given for comparison. Data from both instruments is then correlated along with the response from the standard curve to show the gradually increasing response from the Ambrotose AO® samples.

Results. Although the instrumentation may be different, a summary of the data generated from this study is included for reference. It helps to understand the expected behavior of the Ambrotose AO® dissolution under the given experimental conditions. Below is a table, and graph, summarizing the correlation between the UV-Vis absorbance response and scaled HPLC area under curve versus time for the quercetin standard dilutions. The range shown is from 0-200% of expected quercetin activity from Ambrotose AO®. Averages for three separate runs of the experiment are given, and the runs shown were performed on the same date.

Table 12. UV-Vis and HPLC correlation table of standard

| UV/Vis - HPLC correlation of AO stock quercetin as standard Pharmline quercetin dihydrate HD (84% quercetin) | | | | |
|---|---|---|---|---|
| % Conc. of AO quercetin max | actual ppm | UV-Vis (abs) 377nm | HPLC (auc) 377nm | auc scaled to match abs (auc / 1700000) |
| 12.50% | 3.13 ppm | 0.0819 | 139412 | 0.0820 |
| 25.00% | 6.25 ppm | 0.1628 | 284334 | 0.1673 |
| 50.00% | 12.50 ppm | 0.3924 | 673170 | 0.3960 |
| 100.00% | 25.00 ppm | 0.8283 | 1393657 | 0.8198 |
| 200.00% | 50.00 ppm | 1.6170 | 2775937 | 1.6329 |

The correlation of values from the standard proved encouraging based on the low variations seen between the two instruments. In addition the quercetin response was found to be linear through the entire range of possible concentrations expected from the Ambrotose AO® dissolution tests. Table 13 shows a similar comparison given the correlation of curves between that of UV-Vis absorbance and scaled HPLC area under curve versus time for each sample. As can be seen, a similarly low deviation is found between the two instruments. This suggests confidence that the simpler UV-Vis method can be run standalone as a quantitative tool to measure the total quercetin time release from Ambrotose AO® capsules.

**Table 13. UV-Vis and HPLC correlation table of samples**

| | UV/Vis - HPLC correlation of Ambrotose AO® samples | | |
|---|---|---|---|
| | Average UV-Vis (abs) | Scaled HPLC (auc) | Raw HPLC (auc) |
| **30 min** | 0.0337 | **0.0470** | 79838 |
| **60 min** | 0.1820 | **0.1592** | 270556 |
| **90 min** | 0.2937 | **0.2766** | 470176 |
| **120 min** | 0.3623 | **0.4012** | 681963 |
| **150 min** | 0.4539 | **0.4362** | 741491 |
| **180 min** | 0.5269 | **0.5284** | 898248 |
| **210 min** | 0.5864 | **0.5458** | 927924 |
| **240 min** | 0.6283 | **0.6274** | 1066522 |

The curve of the samples shows the gradual and consistent increase is the response correlating with quercetin dissolution. This response can be seen equally for both instruments without any major deviations. Repeated trials have shown this increase to extend out on average to 4 hours given the dissolution conditions. Further repetitions can be performed with different lots of samples to generate even more correlations. The results shown in Tables 12 and 13, are plotted in the graphs shown in Figures 19 and 20, respectively. The pellets were held at 10,000 psi for a minimum of 120 seconds before being reground with a mortar and pestle and re-capsulated for the dissolution test.

Discussion. During dissolution studies as measured by absorbance over time curve, the Ambrotose AO® has increased activity up to 240 minutes beyond the initial reading. What is shown is the gradual and consistent disintegration of the Ambrotose AO® capsule into the solution, whereby it can be interpreted as a gradual availability in the gut. It must also be noted that visual disintegration alone from the compact tablet or capsule form is not the completion point for total dissolution. Some of the micro-encapsulated materials may still be dispersed in solution binding the quercetin which in turn causes it to not yet be readily available.

A visual analysis of the study confirmed that at approximately $T_{60}$ a good portion of the capsule material has separated from the bulk capsule, and yet the availability response curve continues to increase. This method can be used as a justification of the failed USP and BP disintegration methods by the capsules and a means to measure modified release of dietary supplements. Specifically these results were generated in support of the modified release claims of Ambrotose AO®(Australia), and has the potential to be extended to other dietary supplement tablet or capsule modified release. The method also gives confidence in a simple UV-Vis measurement to carry similar results to that of the more involved HPLC. Quality control specifications can be built around this fact based on a minimal quercetin response relative to the given max measured at specified time intervals. The sampling rate for these types of results can be reduced once sufficient examples continue to support the results.

Table 14. QUERCETIN dissolution test

| Time | AO Quercetin* | Silica Quercetin** | AO Quercetin Tablet*** |
|---|---|---|---|
| **0 min** | ++++ | - | - |
| **60 min** | ++++ | ++++ | + |

*Pure quercetin was mixed 1:2 with Ambrotose®.
**Pure quercetin mixed 1:2 with silica gel and encapsulated (vegetable).
***Ambrotose-quereetin mix pressed to 10,000 psi, reground and encapsulated (vegetable).

Table 15. GRAPE SKIN

| Time | AO Grape Seed* | Silica Grape Seed** | AO Grape Seed Tablet*** |
|---|---|---|---|
| **0 min** | ++++ | - | - |

(continued)

| Time | AO Grape Seed* | Silica Grape Seed** | AO Grape Seed Tablet*** |
|---|---|---|---|
| 60 min | ++++ | ++++ | -/+ |

*Pure grape skin extract was mixed 1:2 with Ambrotose®.
**Pure grape skin extract mixed 1:2 with silica gel and encapsulated (vegetable).
***Ambrotose- grape skin extract mix pressed to 10,000 psi, reground and encapsulated (vegetable).

Table 16. Anti-Oxidant Formula A

| Time | AO and Formula A* | Silica Formula A** | AO Formula A Tablet*** |
|---|---|---|---|
| 0 min | ++++ | - | - |
| 60 min | ++++ | ++++ | ++ |

*Pure Formula A was mixed 1:2 with Ambrotose®.
**Pure Formula A mixed 1:2 with silica gel and encapsulated (vegetable).
***Ambrotose-Formula A Mix then pressed to 10,000 psi, reground and encapsulated (vegetable).

Table 17. Anti-Oxidant Formula B

| Time | AO and Formula B* | Silica Formula B** | AO Formula B Tablet*** |
|---|---|---|---|
| 0 min | ++++ | - | - |
| 60 min | ++++ | ++++ | ++ |

*Pure Formula B was mixed 1:2 with Ambrotose®.
**Pure Formula B mixed 1:2 with silica gel and encapsulated (vegetable).
***Ambrotose-Formula B Mix then pressed to 10,000 psi, reground and encapsulated (vegetable).

Therefore, using identical conditions: capsule, weights, etc., quercetin dissolution were tested over time against an Ambrotose AO® capsule that was emptied and refilled (to ensure that the capsule compaction was the same for all samples), with a capsule of the same composition with the exception of being non-roller compacted. The roller compacted Ambrotose AO® had a 4x - 8x increase in time to reach $Q_{max}$ (the maximum quercetin dissolution). Ambrotose® in a loose form was release immediately. A visual comparison shows the roller compacted capsule maintained its form well past the point of breakdown of the non roller compacted version. Without wishing to be bound by theory, it is possible that the long-chain polymers of Ambrotose® may be acting like chains and the quercetin (or any other active bio-molecule for that matter) "leaches" out at a controlled rate. As such, the long-chain polysaccharides, e.g., chains of from 2 to about 50,000 saccharide monomers may be compressed from between about 100, 500, 1000, 2000 or even 10,000 psi with one or more nutritionally effective amounts: anti-oxidants, vitamins, minerals, amino acids, nucleic acids, saccharides, mixtures and combinations thereof.

It will be understood that particular embodiments described herein are shown by way of illustration and not as limitations of the invention. The principal features of this invention can be employed in various embodiments without departing from the scope of the invention. Those skilled in the art will recognize, or be able to ascertain using no more than routine experimentation, numerous equivalents to the specific procedures described herein. Such equivalents are considered to be within the scope of this invention and are covered by the claims.

All publications and patent applications mentioned in the specification are indicative of the level of skill of those skilled in the art to which this invention pertains.

All of the compositions and/or methods disclosed and claimed herein can be made and executed without undue experimentation in light of the present disclosure.

REFERENCES

1. Koepke CM, Le L, McAnalley S, et al. Results of clinical trials with antioxidants: a review. GlycoScience &Nutrition (Official Publication of GlycoScience corn: The Nutrition Science Site). 2003;4(3): 1-7.

2. Ochi HI Cheng RZ, Kantha SS, et al. The JaICA-genox oxidative stress profile--an overview on the profiling technique in the oxidative stress assessment and management. Biofactors. 2000;13(1-4):195-203.

3. Vivekananthan DP, Penn MS, Sapp SKI et al. Use of antioxidant vitamins for the prevention of cardiovascular disease: meta-analysis of randomised trials. Lancet. 2003;361(9374):2017-2023.

4. Morris CD, Carson S. Routine vitamin supplementation to prevent cardiovascular disease: a summary of the evidence for the U.S. Preventive Services Task Force. Ann Intern Med. 2003;139(1):56-70.

5. Boileau TW, Liao Z, Kim S, et al. Prostate carcinogenesis in N-methyl-N-nitrosourea (NMU)-testosterone-treated rats fed tomato powder, lycopene, or energy-restricted diets. JNatl Cancer Inst. 2003;95(21):1578-1586.

6. Ramberg J, Le L, Vennum E, et al. Why are natural source dietary supplements best? A review based on the vitamin C literature. GlycoScience & Nutrition (Official Publication of GlycoScience com: The Nutrition Science Site). 2003;4(4):1-8.

7. Cao GI Booth SL, Sadowski JA, et al. Increases in human plasma antioxidant capacity after consumption of controlled diets high in fruit and vegetables. Am J Clin Nutr. 1998:68(5):1081-1087.

8. Barhoumi R, Burghardt RG, Busbee DL, et al. Enhancement of glutathione levels and protection from chemically initiated glutathione depletion in rat liver cells by glyconutritionals. Proc Fisher Inst Med Res. 1997;1(1):12-16.

9. Goux WJ, Boyd S, Tone CM, et al. Effect of glyconutritionals on oxidative stress in human subjects: a pilot study. GlycoScience & Nutrition (Official Publication of GlycoScience com: The Nutrition Science Site). 2001;2(12): 1-10.

10. Leonard SS, Cutler D1 Ding MI, et al., Antioxidant properties of fruit and vegetable juices: more to the story than ascorbic acid. Ann Clin Lab Sci. 2002;32(2):193-200.

11. Rice-Evans CAI Miller NJ. Antioxidant activities of flavonoids as bioactive components of food. Biochem Soc Trans. 1996;24(3):790-795.

12. Gil MI, Ferreres F, Tomas-Barberan FA. Effect of postharvest storage and processing on the antioxidant constituents (flavonoids and vitamin C) of fresh-cut spinach. J Agric Food Chem. 1999;47(6):2213-2217.

13. Kurilich AC, Jeffery EH, Juvik JA, et al. Antioxidant capacity of different broccoli (Brassica oleracea) genotypes using the oxygen radical absorbance capacity (ORAC) assay. J Agric Food Chem. 2002;50(18):5053-5057.

14. Wang SY, Zheng W, Galletta GJ. Cultural system affects fruit quality and antioxidant capacity in strawberries. J Agric Food Chem. 2002;50(22):6534-6542.

15. Brand JC, Cherikoff V, Lee A, et al. An outstanding food source of vitamin C. Lancet. 1982;2(8303):873.

16. Ramberg J. Green Tea. GlycoScience & Nutrition (Official Publication of GlycoScience com: The Nutrition Science Site). 2003;4(5):1-9.

17. McAnalley S, Koepke C, Lam L, et al. In vitro methods for testing antioxidant potential: a review. GlycoScience & Nutrition (Official Publication of GlycoScience com: The Nutrition Science Site). 2003;4(2): 1-9.

18. Cao GI Alessio HM, Cutler RG. Oxygen-radical absorbance capacity assay for antioxidants [see comments]. Free Radic Biol Med. 1993;14(3):303-311.

19. Esterbauer HI Jurgens GI Quehenberger 0, et al. Autooxidation of human low density lipoprotein: loss of polyunsaturated fatty acids and vitamin E and generation of aldehydes. J Lipid Res. 1987;28(5):495-509.

20. Ohishi N, Ohkawa HI Miike A, et al. A new assay method for lipid peroxides using a methylene blue derivative. Biochem Int. 1985;10(2):205-211.

EP 1 858 489 B1

21. Shigenaga MK, Ames BN. Assays for 8-hydroxy-2'-deoxyguanosine: a biomarker of in vivo oxidative DNA damage. Free Radic Biol Med. 1991;10(3-4):211-216.

22. Prior RL, Cao G. In vivo total antioxidant capacity: Comparison of different analytical methods. Free Radic Biol Med 1999;27((11/12)):1173-1181.

23. Genox Corporation. Oxygen Radical Absorption Capacity Assay for measuring antioxidant activity. October 2001.

24. Rahman I, MacNee W. Role of oxidants/antioxidants in smoking-induced lung diseases. Free Radic Biol Med 1996;21(5):669-681.

25. Cross CE, van der Vliet A, O'Neill CAI et al. Reactive oxygen species and the lung. Lancet. 1994;344(8927):930-933.

26. Steinmetz KA, Potter JL). Vegetables, fruit, and cancer prevention: a review. Am. Diet Assoc. 1996;96(10):1027-109.

27. Byers TI Guerrero N. Epidemiologic evidence for vitamin C and vitamin E in cancer prevention. Am J Clin Nutr 1995;62(6 Suppl):1385S-1392s.

28. Nutrition and Your Health: Dietary Guidelines for Americans. Home and Garden Bulletin No. 232, USDA and USDHHS: 1995.

29. Halliwell B, Gutteridge JMC. Free Radicals in Biology and Medicine. 3rd Edition. New York: Oxford University Press, 2000.

30. Schmidt MC, Askew EW, Roberts DE, et al. Oxidative stress in humans training in a cold, moderate altitude environment and their response to a phytochemical antioxidant supplement. Wilderness Environ Med. 2002;13(2):94-105.

**Claims**

1. A modified-release dietary supplement comprising:

   a nutritionally effective amount of one or more saccharides comprising one or more long-chain polysaccharides and a nutritionally effective amount of one or more nutritional supplements selected from anti-oxidants, vitamins, minerals, amino acids, nucleic acids, mixtures and combinations thereof,
   wherein the dietary supplement is compressed at a pressure greater than 100 psi (689.4 kPa), wherein the long-chain polysaccharides comprise both a time releasing agent for bioactive molecules and a portion of the dietary supplement,
   wherein the one or more long-chain polysaccharides are isolated from gum tragacanth, guar gum, grain flour, Larch tree extract, aloe vera extract, gum ghatti, starch, gum arabic, xanthan gum, chondroitin sulfate, acetylated polymannose, and mixtures or combinations thereof;
   wherein the one or more nutritional supplements antioxidants comprise an isolated and purified lipophobic oxygen-radical quencher and an isolated and purified lipophilic oxygen-radical quencher, wherein the lipophobic and the lipophilic oxygen-radical quenchers combined have an oxygen-radical quencher value of greater than 6,000 pMol Trolox Equivalents (TE)/gram, and
   wherein the long-chain polysaccharides that comprise the time releasing agent release the nutritional supplements after the dietary supplement is ingested.

2. The supplement of claim 1, **characterised in that** the supplement is inside a capsule, a vegetable capsule or a hard gelatin capsule.

3. The supplement of claim 1 or 2, **characterised in that** the long-chain polysaccharides that comprise the time releasing agent release 85% of the nutritional supplements from between 1 to 8 hours after the dietary supplement is ingested.

4. The supplement of claims 1 to 3, **characterised in that** the dietary supplement comprises one or more excipients.

5. The supplement of claims 1 to 4, **characterised in that** the one or more saccharides comprise galactose, galactosamine, glucosamine, glucose, mannose, acetylated-mannose, N-acetylneuraminic acid, fucose, N-acetylgalactosamine, N-acetylglucosamine, arabinose, arabinogalactan, galacturonic acid, glucuronic acid, iduronic acid, xylose and mixtures or combinations thereof.

6. The supplement of claims 1 to 5, **characterised in that** the dietary supplement is compressed by roller compaction at a pressure between 2,000 to 10,000 psi (13,789 to 68,940 kPa), preferably between 5,000 to 10,000 psi (34,473 to 68,940 kPa).

7. The supplement of claims 1 to 5, **characterised in that** the dietary supplement is compressed at a pressure greater than 10,000 psi (68,940 kPa).

8. The supplement of claims 1 to 7, **characterised in that** the one or more saccharides comprise from 0.1 to 75 weight percent, preferably from 1 to 10 weight percent of each of isolated and purified galactose, glucose, mannose, N-acetylneuraminic acid, fucose, N-acetylgalactosamine, N-acetylglucosamine and xylose.

9. The supplement of claims 1 to 8, **characterised in that** the one or more long-chain polysaccharide and the nutritional supplements are roller compacted at a pressure greater than 2,000 psi (13,789 kPa) .

10. The supplement of claims 1 to 9, **characterised in that** the lipophobic oxygen-radical quencher is selected from the group consisting of one or more vitamin E selected from the group consisting of alpha, beta, delta, epsilon, gamma, zeta, eta, xi1, xi2, and sigma tocopherols, and alpha, beta, delta and gamma tocotrienols, analogs thereof, pharmaceutically acceptable salts thereof, and combinations thereof.

11. The supplement of claims 1 to 10, **characterised in that** the lipophilic oxygen-radical quencher is selected from the group consisting of flavonols, quercetin, kaempferol, myricetin, apigenin and derivatives, analogs, pharmaceutically acceptable salts thereof, and combinations thereof.

12. The supplement of claims 1 to 11, **characterised in that** the nutritional supplements further comprise a nutritionally effective amount of six or more saccharides selected from galactose, galactosamine, glucosamine, glucose, mannose, acetylated-mannose, N-acetylneuraminic acid, fucose, N-acetylgalactosamine, N-acetylglucosamine, arabinose, arabinogalactan, galacturonic acid, glucuronic acid, iduronic acid, xylose and mixtures or combinations thereof.

13. The supplement of claims 1 to 12, **characterised in that** the one or more saccharides comprise galactose, mannose, fucose, N-acetylgalactosamine, N-acetylglucosamine and xylose.

14. The supplement of claims 1 to 13, **characterised in that** the one or more saccharides comprises from about 1 to about 10 weight percent of each of isolated and purified galactose, glucose, mannose, N-acetylneuraminic acid, fucose, N-acetylgalactosamine, N-acetylglucosamine and xylose.

15. The supplement of claims 1 to 14, **characterised in that** the long-chain polysaccharides comprise from between 2 to 50,000 monomers, preferably 200 to 50,000 monomers, more preferably 2,000 to 50,000 monomers.

16. The supplement of claims 1 to 15, **characterised in that** the lipophobic and the lipophilic oxygen-radical quencher when provided to a patient provide an increase of over 13% as measured by ORAC(β-PE) from the patient's baseline antioxidant level.

17. The supplement of claims 1 to 16, **characterised in that** the lipophobic and the lipophilic oxygen-radical quencher comprise between about 1 and 30 percent by weight of the supplement.

18. The supplement of claims 1 to 17, comprising one or more lipophilic anti-oxidants, and one or more lipophobic anti-oxidants, **characterised in that** the supplement is roller compacted at a pressure greater that 5,000 psi (34,473 kPa) and 85% of the nutritional supplements are released from between 2 to 6 hours and the lipophobic and the lipophilic antioxidants combined have a dissolved oxygen value of greater than 6,000 μMol Trolox Equivalents (TE)/gram.

**19.** A method of making a modified-release dietary supplement comprising the steps of:

mixing one or more saccharides comprising one or more long-chain polysaccharides and a nutritionally effective amount of one or more nutritional supplements selected from anti-oxidants, vitamins, minerals, amino acids, nucleic acids, herbal extracts, mixtures and combinations thereof, wherein the long-chain polysaccharides comprise both a time releasing agent for bioactive molecules and a portion of the dietary supplement, wherein the one or more nutritional supplements comprise anti-oxidants that comprise an isolated and purified lipophobic oxygen-radical quencher and an isolated and purified lipophilic oxygen-radical quencher, wherein the lipophobic and the lipophilic oxygen-radical quenchers combined have an oxygen-radical quencher value of greater than 6,000 μMol Trolox Equivalents (TE)/gram; and wherein compressing the dietary supplement at a pressure greater than 2,000 psi (13,789 kPa), wherein the long-chain polysaccharides that comprise the time releasing agent release the nutritional supplements after the dietary supplement is ingested.

**20.** The method of claim 19, **characterised in that** the supplement is placed inside an external capsule, a vegetable capsule or a hard gelatin capsule.

**21.** The method of claims 19 or 20, **characterised in that** the long-chain polysaccharides that comprise the time releasing agent release 85% of the nutritional supplements from between 1 to 8 hours after the dietary supplement is ingested.

**22.** The method of claims 19 to 21, **characterised in that** the supplement further comprises one or more excipients.

**23.** The method of claims 19 to 22, **characterised in that** the one or more saccharides comprise galactose, galactosamine, glucosamine, glucose, mannose, acetylated-mannose, N-acetylneuraminic acid, fucose, N-acetylgalactosamine, N-acetylglucosamine and xylose or combinations thereof.

**24.** The method of claims 19 to 23, wherein the dietary supplement is compressed at a pressure between 2,000 to 10,000 psi (13,789 to 68,940 kPa), preferably between 5,000 to 10,000 psi (34,473 to 68,940 kPa).

**25.** The method of claims 19 to 23, **characterised in that** the dietary supplement is compressed at a pressure of greater than 10,000 psi (68,940 kPa).

**26.** The method of claims 19 to 25, **characterised in that** the compression is by roller compaction.

**27.** The method of claima 19 to 26, **characterised in that** the one or more saccharides comprise from 0.1 to 75 weight percent, preferably 1 to 10 weight percent, of each of isolated and purified galactose, glucose, mannose, N-acetylneuraminic acid, fucose, N-acetylgalactosamine, N-acetylglucosamine and xylose.

**28.** The method of claims 19 to 27, **characterised in that** the one or more long-chain polysaccharides are selected from gum tragacanth, guar gum, grain flour, Larch tree extract, aloe vera extract, gum ghatti, starch, gum arabic, xanthan gum, chondroitin sulfate, acetylated polymannose, mixtures and combinations thereof.

**29.** The method of any one of claims 19 to 28, **characterised in that** the lipophobic oxygen-radical quencher is selected from the group consisting of one or more vitamin E selected from the group consisting of alpha, beta, delta, epsilon, gamma, zeta, eta, xi1, xi2, and sigma tocopherols, and alpha, beta, delta and gamma toco-trienols, analogs thereof, pharmaceutically acceptable salts thereof, and combinations thereof.

**30.** The method of any one of claims 19 to 29, **characterised in that** the lipophilic oxygen-radical quencher is selected from the group consisting of flavonols, quercetin, kaempferol, myricetin, apigenin and derivatives, analogs, pharmaceutically acceptable salts thereof, and combinations thereof.

**Patentansprüche**

**1.** Nahrungsergänzungsmittel mit modifizierter Freisetzung, aufweisend:

eine ernährungsphysiologisch wirksame Menge eines oder mehrerer Saccharide, die ein oder mehrere langkettige Polysaccharide und eine ernährungsphysiologisch wirksame Menge eines oder mehrerer Nahrungsergänzungsmittel, ausgewählt aus Antioxidantien, Vitaminen, Mineralien, Aminosäuren, Nukleinsäuren, Mischungen und Kombinationen davon, aufweisen,

wobei das Nahrungsergänzungsmittel bei einem Druck von mehr als 100 psi (689,4 kPa) komprimiert wird, wobei die langkettigen Polysaccharide sowohl ein Time-Release Mittel für bioaktive Moleküle als auch einen Teil des Nahrungsergänzungsmittels aufweisen,

wobei das eine oder die mehreren langkettigen Polysaccharide aus Traganth, Guargummi, Getreidemehl, Lärchenbaumextrakt, Aloe-Vera-Extrakt, Ghattigummi, Stärke, Gummiarabicum, Xanthangummi, Chondroitinsulfat, acetylierter Polymannose und Mischungen oder Kombinationen davon isoliert sind;

wobei das eine oder die mehreren Nahrungsergänzungsmittel-Antioxidantien einen isolierten und gereinigten lipophoben Sauerstoffradikalfänger und einen isolierten und gereinigten lipophilen Sauerstoffradikalfänger aufweisen, wobei die lipophoben und die lipophilen Sauerstoffradikalfänger kombiniert einen Sauerstoffradikalfänger-Wert von mehr als 6 000 μMol Trolox-Äquivalenten (TE)/Gramm aufweisen, und

wobei die langkettigen Polysaccharide, die das Time-Release Mittel aufweisen, die Nahrungsergänzungsmittel freisetzen, nachdem das Nahrungsergänzungsmittel eingenommen wurde.

2. Ergänzungsmittel nach Anspruch 1, **dadurch gekennzeichnet, dass** das Ergänzungsmittel sich in einer Kapsel, einer Pflanzenkapsel oder einer Hartgelatinekapsel befindet.

3. Ergänzungsmittel nach Anspruch 1 oder 2, **dadurch gekennzeichnet, dass** die langkettigen Polysaccharide, die das Time-Release Mittel aufweisen, 85 % der Nahrungsergänzungsmittel zwischen 1 und 8 Stunden nach der Einnahme des Nahrungsergänzungsmittels freisetzen.

4. Ergänzungsmittel nach Anspruch 1 bis 3, **dadurch gekennzeichnet, dass** das Nahrungsergänzungsmittel einen oder mehrere Hilfsstoffe aufweist.

5. Ergänzungsmittel nach Anspruch 1 bis 4, **dadurch gekennzeichnet, dass** das eine oder die mehreren Saccharide Galactose, Galactosamin, Glucosamin, Glucose, Mannose, acetylierte Mannose, N-Acetylneuraminsäure, Fucose, N-Acetylgalactosamin, N-Acetylglucosamin, Arabinose, araBinogalactan, Galacturonsäure, Glucuronsäure, Iduronsäure, Xylose und Mischungen oder Kombinationen davon aufweisen.

6. Ergänzungsmittel nach Anspruch 1 bis 5, **dadurch gekennzeichnet, dass** das Nahrungsergänzungsmittel durch Walzenkompaktierung bei einem Druck zwischen 2 000 bis 10 000 psi (13 789 bis 68 940 kPa), vorzugsweise zwischen 5 000 bis 10 000 psi (34 473 bis 68 940 kPa), komprimiert wird.

7. Ergänzungsmittel nach Anspruch 1 bis 5, **dadurch gekennzeichnet, dass** das Nahrungsergänzungsmittel bei einem Druck von mehr als 10 000 psi (68 940 kPa) komprimiert wird.

8. Ergänzungsmittel nach Anspruch 1 bis 7, **dadurch gekennzeichnet, dass** das eine oder die mehreren Saccharide von 0,1 bis 75 Gewichtsprozent, vorzugsweise von 1 bis 10 Gewichtsprozent, von jeweils isolierter und gereinigter Galactose, Glucose, Mannose, N-Acetylneuraminsäure, Fucose, N-Acetylgalactosamin, N-Acetylglucosamin und Xylose aufweisen.

9. Ergänzungsmittel nach Anspruch 1 bis 8, **dadurch gekennzeichnet, dass** das eine oder die mehreren langkettigen Polysaccharide und die Nahrungsergänzungsmittel bei einem Druck von mehr als 2 000 psi (13 789 kPa) walzenkompaktiert werden.

10. Ergänzungsmittel nach Anspruch 1 bis 9, **dadurch gekennzeichnet, dass** der lipophobe Sauerstoffradikalfänger aus der Gruppe, bestehend aus einem oder mehreren Vitaminen E, ausgewählt aus der Gruppe, bestehend aus Alpha-, Beta-, Delta-, Epsilon-, Gamma-, Zeta-, Eta-, xi1-, xi2- und Sigma-Tocopherolen und Alpha-, Beta-, Delta- und Gamma-Tocotrienolen, Analoga davon, pharmazeutisch verträglichen Salzen davon und Kombinationen davon, ausgewählt ist.

11. Ergänzungsmittel nach Anspruch 1 bis 10, **dadurch gekennzeichnet, dass** der lipophile Sauerstoffradikalfänger aus der Gruppe, bestehend aus Flavonolen, Quercetin, Kaempferol, Myricetin, Apigenin und Derivaten, Analoga, pharmazeutisch verträglichen Salzen davon und Kombinationen davon, ausgewählt ist.

12. Ergänzungsmittel nach Anspruch 1 bis 11, **dadurch gekennzeichnet, dass** die Nahrungsergänzungsmittel ferner eine ernährungsphysiologisch wirksame Menge von sechs oder mehr Sacchariden, ausgewählt aus Galactose, Galactosamin, Glucosamin, Glucose, Mannose, acetylierter Mannose, N-Acetylneuraminsäure, Fucose, N-Acetylgalactosamin, N-Acetylglucosamin, Arabinose, Arabinogalactan, Galacturonsäure, Glucuronsäure, Iduronsäure, Xylose und Mischungen oder Kombinationen davon, aufweisen.

13. Ergänzungsmittel nach Anspruch 1 bis 12, **dadurch gekennzeichnet, dass** das eine oder die mehreren Saccharide Galactose, Mannose, Fucose, N-Acetylgalactosamin, N-Acetylglucosamin und Xylose aufweisen.

14. Ergänzungsmittel nach Anspruch 1 bis 13, **dadurch gekennzeichnet, dass** das eine oder die mehreren Saccharide von etwa 1 bis 10 Gewichtsprozent von jeder der isolierten und gereinigten Galactose, Glucose, Mannose, N-Acetylneuraminsäure, Fucose, N-Acetylgalactosamin, N-Acetylglucosamin und Xylose aufweisen.

15. Ergänzungsmittel nach Anspruch 1 bis 14, **dadurch gekennzeichnet, dass** die langkettigen Polysaccharide zwischen 2 und 50 000 Monomeren, vorzugsweise 200 bis 50 000 Monomeren, besonders bevorzugt 2 000 bis 50 000 Monomeren aufweisen.

16. Ergänzungsmittel nach Anspruch 1 bis 15, **dadurch gekennzeichnet, dass** der lipophobe und der lipophile Sauerstoffradikalfänger, wenn sie einem Patienten bereitgestellt werden, einen Anstieg von über 13 %, gemessen durch ORAC (β-PE), gegenüber dem Antioxidans-Ausgangswert des Patienten bereitstellen.

17. Ergänzungsmittel nach Anspruch 1 bis 16, **dadurch gekennzeichnet, dass** der lipophobe und das lipophile Sauerstoffradikalfänger zwischen etwa 1 und 30 Gewichtsprozent des Ergänzungsmittels aufweisen.

18. Ergänzungsmittel nach Anspruch 1 bis 17, das ein oder mehrere lipophile Antioxidantien und ein oder mehrere lipophobe Antioxidantien aufweist, **dadurch gekennzeichnet, dass** das Ergänzungsmittel bei einem Druck von mehr als 5 000 psi (34 473 kPa) walzenkompaktiert wird und 85 % der Nahrungsergänzungsmittel zwischen 2 und 6 Stunden freigesetzt werden und das lipophobe und das lipophile Antioxidans zusammen einen Wert an gelöstem Sauerstoff von mehr als 6 000 μMol Trolox-Äquivalenten (TE)/Gramm aufweisen.

19. Verfahren zur Herstellung eines Nahrungsergänzungsmittels mit modifizierter Freisetzung, welches die folgenden Schritte aufweist:

Mischen eines oder mehrerer Saccharide, die ein oder mehrere langkettige Polysaccharide und eine ernährungsphysiologisch wirksame Menge eines oder mehrerer Nahrungsergänzungsmittel, ausgewählt aus Antioxidantien, Vitaminen, Mineralien, Aminosäuren, Nukleinsäuren, Kräuterextrakten, Mischungen und Kombinationen davon, aufweisen, wobei die langkettigen Polysaccharide sowohl ein Time-Release Mittel für bioaktive Moleküle als auch einen Teil des Nahrungsergänzungsmittels aufweisen,
wobei das eine oder die mehreren Nahrungsergänzungsmittel Antioxidantien aufweisen, die einen isolierten und gereinigten lipophoben Sauerstoffradikalfänger und einen isolierten und gereinigten lipophilen Sauerstoffradikalfänger aufweisen, wobei die lipophoben und die lipophilen Sauerstoffradikalfänger kombiniert einen Sauerstoffradikalfänger-Wert von mehr als 6 000 μMol Trolox-Äquivalenten (TE)/Gramm aufweisen; und
wobei das Nahrungsergänzungsmittel bei einem Druck von mehr als 2 000 psi (13 789 kPa) komprimiert wird, wobei die langkettigen Polysaccharide, die das Time-Release Mittel aufweisen, die Nahrungsergänzungsmittel freisetzen, nachdem das Nahrungsergänzungsmittel eingenommen wurde.

20. Verfahren nach Anspruch 19, **dadurch gekennzeichnet, dass** das Ergänzungsmittel in einer äußeren Kapsel, einer Pflanzenkapsel oder einer Hartgelatinekapsel platziert wird.

21. Verfahren nach Anspruch 19 oder 20, **dadurch gekennzeichnet, dass** die langkettigen Polysaccharide, die das Time-Release Mittel aufweisen, 85 % der Nahrungsergänzungsmittel zwischen 1 und 8 Stunden nach der Einnahme des Nahrungsergänzungsmittels freisetzen.

22. Verfahren nach Anspruch 19 bis 21, **dadurch gekennzeichnet, dass** das Ergänzungsmittel ferner einen oder mehrere Hilfsstoffe aufweist.

23. Verfahren nach Anspruch 19 bis 22, **dadurch gekennzeichnet, dass** das eine oder die mehreren Saccharide Galactose, Galactosamin, Glucosamin, Glucose, Mannose, acetylierte Mannose, N-Acetylneuraminsäure, Fucose, N-Acetylgalactosamin, N-Acetylglucosamin und Xylose oder Kombinationen davon aufweisen.

**24.** Verfahren nach Anspruch 19 bis 23, wobei das Nahrungsergänzungsmittel bei einem Druck zwischen 2 000 bis 10 000 psi (13 789 bis 68 940 kPa), vorzugsweise zwischen 5 000 bis 10 000 psi (34 473 bis 68 940 kPa), komprimiert wird.

**25.** Verfahren nach Anspruch 19 bis 23, **dadurch gekennzeichnet, dass** das Nahrungsergänzungsmittel bei einem Druck von mehr als 10 000 psi (68 940 kPa) komprimiert wird.

**26.** Verfahren nach Anspruch 19 bis 25, **dadurch gekennzeichnet, dass** das die Kompression durch eine Walzenkompaktierung geschieht.

**27.** Verfahren nach Anspruch 19 bis 26, **dadurch gekennzeichnet, dass** das eine oder die mehreren Saccharide von 0,1 bis 75 Gewichtsprozent, vorzugsweise 1 bis 10 Gewichtsprozent, von jeweils isolierter und gereinigter Galactose, Glucose, Mannose, N-Acetylneuraminsäure, Fucose, N-Acetylgalactosamin, N-Acetylglucosamin und Xylose aufweisen.

**28.** Verfahren nach Anspruch 19 bis 27, **dadurch gekennzeichnet, dass** das eine oder die mehreren langkettigen Polysaccharide aus Traganth, Guargummi, Getreidemehl, Lärchenbaumextrakt, Aloe-Vera-Extrakt, Ghattigummi, Stärke, Gummiarabicum, Xanthangummi, Chondroitinsulfat, acetylierter Polymannose und Mischungen oder Kombinationen davon isoliert sind.

**29.** Verfahren nach einem der Ansprüche 19 bis 28, **dadurch gekennzeichnet, dass** der lipophobe Sauerstoffradikalfänger aus der Gruppe, bestehend aus einem oder mehreren Vitaminen E, ausgewählt aus der Gruppe, bestehend aus Alpha-, Beta-, Delta-, Epsilon-, Gamma-, Zeta-, Eta-, xi1-, xi2- und Sigma-Tocopherolen und Alpha-, Beta-, Delta- und Gamma-Tocotrienolen, Analoga davon, pharmazeutisch verträglichen Salzen davon und Kombinationen davon, ausgewählt ist.

**30.** Verfahren nach einem der Ansprüche 19 bis 29, **dadurch gekennzeichnet, dass** der lipophile Sauerstoffradikalfänger aus der Gruppe, bestehend aus Flavonolen, Quercetin, Kaempferol, Myricetin, Apigenin und Derivaten, Analoga, pharmazeutisch verträglichen Salzen davon und Kombinationen davon, ausgewählt ist.

**Revendications**

**1.** Supplément alimentaire à libération modifiée comprenant :

une quantité efficace sur le plan nutritionnel d'un ou de plusieurs saccharides comprenant un ou plusieurs polysaccharides à longue chaîne et une quantité efficace sur le plan nutritionnel d'un ou de plusieurs suppléments nutritionnels sélectionnés parmi des antioxydants, vitamines, minéraux, acides aminés, acides nucléiques, des mélanges et des combinaisons de ceux-ci,
dans lequel le supplément alimentaire est compressé à une pression supérieure à 100 psi (689,4 kPa), dans lequel les polysaccharides à longue chaîne comprennent à la fois un agent de libération dans le temps pour les molécules bioactives et une portion du supplément alimentaire,
dans lequel les un ou plusieurs polysaccharides à longue chaîne sont isolés à partir d'une gomme adragante, d'une gomme de guar, d'une farine de grain, d'un extrait de mélèze, d'un extrait d'aloe vera, d'une gomme ghatti, d'un amidon, d'une gomme arabique, d'une gomme de xanthane, d'une chondroïtine sulfate, d'un polymannose acétylé, et des mélanges ou des combinaisons de ceux-ci ;
dans lequel les un ou plusieurs suppléments nutritionnels antioxydants comprennent un désactivateur lipophobe d'oxygène radicalaire isolé et purifié et un désactivateur lipophile d'oxygène radicalaire isolé et purifié, dans lequel les désactivateurs d'oxygène radicalaire lipophobe et lipophile combinés présentent une valeur de désactivateur d'oxygène radicalaire supérieure à 6 000 $\mu$Mol d'équivalents Trolox (TE)/gramme, et
dans lequel les polysaccharides à longue chaîne qui comprennent l'agent de libération dans le temps libèrent les suppléments nutritionnels après que le supplément alimentaire est ingéré.

**2.** Supplément selon la revendication 1, **caractérisé en ce que** le supplément est à l'intérieur d'une capsule, d'une capsule végétale ou d'une capsule de gélatine dure.

**3.** Supplément selon la revendication 1 ou 2, **caractérisé en ce que** les polysaccharides à longue chaîne qui comprennent l'agent de libération dans le temps libèrent 85 % des suppléments nutritionnels entre 1 et 8 heures après que le supplément alimentaire est ingéré.

**4.** Supplément selon les revendications 1 à 3, **caractérisé en ce que** le supplément alimentaire comprend un ou plusieurs excipients.

**5.** Supplément selon les revendications 1 à 4, **caractérisé en ce que** les un ou plusieurs saccharides comprennent du galactose, de la galactosamine, de la glucosamine, du glucose, du mannose, du mannose acétylé, de l'acide N-acétylneuraminique, du fucose, de la N-acétylgalactosamine, de la N-acétylglucosamine, de l'arabinose, de l'arabinogalactane, de l'acide galacturonique, de l'acide glucuronique, de l'acide iduronique, du xylose et des mélanges ou des combinaisons de ceux-ci.

**6.** Supplément selon les revendications 1 à 5, **caractérisé en ce que** le supplément alimentaire est compressé par compaction au rouleau à une pression située entre 2 000 et 10 000 psi (13 789 à 68 940 kPa), de préférence entre 5 000 et 10 000 psi (34 473 à 68 940 kPa).

**7.** Supplément selon les revendications 1 à 5, **caractérisé en ce que** le supplément alimentaire est compressé à une pression supérieure à 10 000 psi (68 940 kPa).

**8.** Supplément selon les revendications 1 à 7, **caractérisé en ce que** les un ou plusieurs saccharides comprennent de 0,1 à 75 pour cent en poids, de préférence de 1 à 10 pour cent en poids de chacun du galactose, du glucose, du mannose, de l'acide N-acétylneuraminique, du fucose, de la N-acétylgalactosamine, de la N-acétylglucosamine et du xylose isolés et purifiés.

**9.** Supplément selon les revendications 1 à 8, **caractérisé en ce que** les un ou plusieurs polysaccharides à longue chaîne et les suppléments nutritionnels sont compactés au rouleau à une pression supérieure à 2 000 psi (13 789 kPa).

**10.** Supplément selon les revendications 1 à 9, **caractérisé en ce que** le désactivateur lipophobe d'oxygène radicalaire est sélectionné dans le groupe constitué d'une ou de plusieurs vitamines E sélectionnées dans le groupe constitué des alpha, bêta, delta, epsilon, gamma, zêta, êta, xi1, xi2, et sigma tocophérols, et des alpha, bêta, delta et gamma tocotriènols, des analogues de ceux-ci, des sels pharmaceutiquement acceptables de ceux-ci, et des combinaisons de ceux-ci.

**11.** Supplément selon les revendications 1 à 10, **caractérisé en ce que** le désactivateur lipophile d'oxygène radicalaire est sélectionné dans le groupe constitué des flavonols, de la quercétine, du kaempférol, de la myricétine, de l'apigénine et des dérivés, des analogues, des sels pharmaceutiquement acceptables de ceux-ci, et des combinaisons de ceux-ci.

**12.** Supplément selon les revendications 1 à 11, **caractérisé en ce que** les suppléments nutritionnels comprennent en outre une quantité efficace sur le plan nutritionnel de six saccharides ou plus sélectionnés parmi le galactose, la galactosamine, la glucosamine, le glucose, le mannose, le mannose acétylé, l'acide N-acétylneuraminique, le fucose, la N-acétylgalactosamine, la N-acétylglucosamine, l'arabinose, l'arabinogalactane, l'acide galacturonique, l'acide glucuronique, l'acide iduronique, le xylose et des mélanges ou des combinaisons de ceux-ci.

**13.** Supplément selon les revendications 1 à 12, **caractérisé en ce que** les un ou plusieurs saccharides comprennent du galactose, du mannose, du fucose, de la N-acétylgalactosamine, de la N-acétylglucosamine et du xylose.

**14.** Supplément selon les revendications 1 à 13, **caractérisé en ce que** les un ou plusieurs saccharides comprennent d'environ 1 à environ 10 pour cent en poids de chacun du galactose, du glucose, du mannose, de l'acide N-acétylneuraminique, du fucose, de la N-acétyl-galactosamine, de la N-acétylglucosamine et du xylose isolés et purifiés.

**15.** Supplément selon les revendications 1 à 14, **caractérisé en ce que** les polysaccharides à longue chaîne comprennent entre 2 et 50 000 monomères, de préférence 200 à 50 000 monomères, de manière davantage préférée 2 000 à 50 000 monomères.

**16.** Supplément selon les revendications 1 à 15, **caractérisé en ce que** les désactivateurs d'oxygène radicalaire lipophobe et lipophile quand ils sont fournis à un patient fournissent une augmentation de plus de 13 % telle que mesurée par ORAC (β-PE) à partir du taux d'antioxydant de base du patient.

**17.** Supplément selon les revendications 1 à 16, **caractérisé en ce que** les désactivateurs d'oxygène radicalaire lipophobe et lipophile comprennent entre environ 1 et 30 pour cent en poids du supplément.

**18.** Supplément selon les revendications 1 à 17, comprenant un ou plusieurs antioxydants lipophiles, et un ou plusieurs antioxydants lipophobes, **caractérisé en ce que** le supplément est compacté au rouleau à une pression supérieure à 5 000 psi (34 473 kPa) et 85 % des suppléments nutritionnels sont libérés entre 2 et 6 heures et les antioxydants lipophobe et lipophile combinés présentent une valeur d'oxygène dissous supérieure à 6 000 μMol d'équivalents Trolox (TE)/gramme.

**19.** Procédé de préparation d'un supplément alimentaire à libération modifiée comprenant les étapes de :

mélange d'un ou de plusieurs saccharides comprenant un ou plusieurs polysaccharides à longue chaîne et d'une quantité efficace sur le plan nutritionnel d'un ou de plusieurs suppléments nutritionnels sélectionnés parmi des antioxydants, vitamines, minéraux, acides aminés, acides nucléiques, extraits à base de plantes, des mélanges et des combinaisons de ceux-ci, dans lequel les polysaccharides à longue chaîne comprennent à la fois un agent de libération dans le temps pour les molécules bioactives et une portion du supplément alimentaire, dans lequel les un ou plusieurs suppléments nutritionnels comprennent des antioxydants qui comprennent un désactivateur lipophobe d'oxygène radicalaire isolé et purifié et un désactivateur lipophile d'oxygène radicalaire isolé et purifié, dans lequel les désactivateurs d'oxygène radicalaire lipophobe et lipophile combinés présentent une valeur de désactivateur d'oxygène radicalaire supérieure à 6 000 μMol d'équivalents Trolox (TE)/gramme ; et dans lequel la compression du supplément alimentaire à une pression supérieure à 2 000 psi (13 789 kPa), dans lequel les polysaccharides à longue chaîne qui comprennent l'agent de libération dans le temps libèrent les suppléments nutritionnels après que le supplément alimentaire est ingéré.

**20.** Procédé selon la revendication 19, **caractérisé en ce que** le supplément est placé à l'intérieur d'une capsule externe, d'une capsule végétale ou d'une capsule de gélatine dure.

**21.** Procédé selon la revendication 19 ou 20, **caractérisé en ce que** les polysaccharides à longue chaîne qui comprennent l'agent de libération dans le temps libèrent 85 % des suppléments nutritionnels entre 1 et 8 heures après que le supplément alimentaire est ingéré.

**22.** Procédé selon les revendications 19 à 21, **caractérisé en ce que** le supplément comprend en outre un ou plusieurs excipients.

**23.** Procédé selon les revendications 19 à 22, **caractérisé en ce que** les un ou plusieurs saccharides comprennent du galactose, de la galactosamine, de la glucosamine, du glucose, du mannose, du mannose acétylé, de l'acide N-acétylneuraminique, du fucose, de la N-acétylgalactosamine, de la N-acétylglucosamine et du xylose et ou combinaisons de ceux-ci.

**24.** Procédé selon les revendications 19 à 23, dans lequel le supplément alimentaire est compressé à une pression située entre 2 000 et 10 000 psi (13 789 à 68 940 kPa), de préférence entre 5 000 et 10 000 psi (34 473 à 68 940 kPa).

**25.** Procédé selon les revendications 19 à 23, **caractérisé en ce que** le supplément alimentaire est compressé à une pression supérieure à 10 000 psi (68 940 kPa).

**26.** Procédé selon les revendications 19 à 25, **caractérisé en ce que** la compression est par compaction au rouleau.

**27.** Procédé selon les revendications 19 à 26, **caractérisé en ce que** les un ou plusieurs saccharides comprennent de 0,1 à 75 pour cent en poids, de préférence de 1 à 10 pour cent en poids, de chacun du galactose, du glucose, du mannose, de l'acide N-acétylneuraminique, du fucose, de la N-acétylgalactosamine, de la N-acétylglucosamine et du xylose isolés et purifiés.

**28.** Procédé selon les revendications 19 à 27, **caractérisé en ce que** les un ou plusieurs polysaccharides à longue chaîne sont sélectionnés parmi une gomme adragante, une gomme de guar, une farine de grain, un extrait de mélèze, un extrait d'aloe vera, une gomme ghatti, un amidon, une gomme arabique, une gomme de xanthane, une chondroïtine sulfate, un polymannose acétylé, et des mélanges et des combinaisons de ceux-ci ;

**29.** Procédé selon l'une quelconque des revendications 19 à 28, **caractérisé en ce que** le désactivateur lipophobe d'oxygène radicalaire est sélectionné dans le groupe constitué d'une ou de plusieurs vitamines E sélectionnées dans le groupe constitué des alpha, bêta, delta, epsilon, gamma, zêta, êta, xi1, xi2, et sigma tocophérols, et des alpha, bêta, delta et gamma tocotriènols, des analogues de ceux-ci, des sels pharmaceutiquement acceptables de ceux-ci, et des combinaisons de ceux-ci.

**30.** Procédé selon l'une quelconque des revendications 19 à 29, **caractérisé en ce que** le désactivateur lipophile d'oxygène radicalaire est sélectionné dans le groupe constitué des flavonols, de la quercétine, du kaempférol, de la myricétine, de l'apigénine et des dérivés, des analogues, des sels pharmaceutiquement acceptables de ceux-ci, et des combinaisons de ceux-ci.

*Fig. 1*

50

EQUILIBRATE OXYGEN PROBE IN
SOLVENT: WATER:DETERGENT
**52**

DISSOLVE ANTI-OXIDANT
STANDARD IN
SOLVENT:WATER:DETERGENT
**54**

DISSOLVED SAMPLE IN
SOLVENT:WATER:DETERGENT
**66**

ADD OXYGEN RADICAL
TARGET
**56**

ADD OXYGEN RADICAL
TARGET
**68**

CHALLENGE ANTI-OXIDANT
STANDARD WITH OXYGEN
RADICAL GENERATOR IN
SOLVENT:WATER:DETERGENT
**58**

CHALLENGE ANTI-OXIDANT
SAMPLE WITH OXYGEN
RADICAL GENERATOR IN
SOLVENT:WATER:DETERGENT
**70**

MEASURE ANTI-OXIDANT
STANDARD IN
SOLVENT:WATER:DETERGENT
**60**

MEASURE SAMPLE IN
SOLVENT:WATER:DETERGENT
**72**

SUBTRACT
SOLVENT: WATER:DETERGENT
BACKGROUND
**62**

COMPARE SAMPLE VALUE TO KNOWN ANTI-
OXIDANT STANDARD VALUE
**64**

*Fig. 2*

## ORAC$_0$

| Initiator: | AAPH |
| Standard: | Trolox |
| Target Molecule: | Linoleic Acid |

Percent Oxygen

blank

Time

## ORAC$_{fl}$

| Initiator: | AAPH |
| Standard: | Trolox |
| Target Molecule: | Flourescein |

Fluorescence

Time

*Fig. 3*

Fig. 4

**ORAC₀**

| Initiator: | AAPH |
| Standard: | Trolox |
| Target Molecule: | Linoleic Acid |

**ORAC_fl**

| Initiator: | AAPH |
| Standard: | Trolox |
| Target Molecule: | Flourescein |

*Fig. 5*

*Fig. 6*

*Fig. 7*

*Fig. 8*

*Fig. 9*

*Fig. 10*

*Fig. 11*

**Fig. 12A**

**Fig. 12B**

*Fig. 13*

*Fig. 14*

*Fig. 15*

*Fig. 16*

*Fig. 17*

Figure 18

Figure 19

Figure 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9702829 A2
- WO 9806418 A1
- US 6063403 A
- US 6805880 B1
- US 4735935 A
- US 2003072770 A

### Non-patent literature cited in the description

- **OU , B. ; HAMPSCH-WOODILL, M. ; PRIOR, R.L.** *J. Agric. Food Chem.,* 2001, vol. 49, 4619-4626
- **OU et al.** Development and Validation of Oxygen Radical Absorbance Capacity Assay for Lipophilic Antioxidants Using Randomly Methylated-p-Cyclodextrin as the Solubility Enhancer. *J. Agric. Food Chem.,* 2002, vol. 50, 1815-1821
- **PIZZORNO ; MURRAY.** Textbook of Natural Medicine. Churchill Livingston, 1999
- **LEAF A. ; LAUNOIS J.** A Scientist Visits Some of the World's Oldest People. *National Geographic,* January 1973
- **KOENIG R.** Sardinia's Mysterious Male Methuselahs. *Science,* 16 March 2001
- **KOEPKE CM, LE L ; MCANALLEY S et al.** Results of clinical trials with antioxidants: a review. *GlycoScience &Nutrition (Official Publication of GlycoScience corn: The Nutrition Science Site),* 2003, vol. 4 (3), 1-7
- **OCHI HI CHENG RZ ; KANTHA SS et al.** The Jal-CA-genox oxidative stress profile--an overview on the profiling technique in the oxidative stress assessment and management. *Biofactors,* 2000, vol. 13 (1-4), 195-203
- **VIVEKANANTHAN DP ; PENN MS ; SAPP SKI et al.** Use of antioxidant vitamins for the prevention of cardiovascular disease: meta-analysis of randomised trials. *Lancet,* 2003, vol. 361 (9374), 2017-2023
- **MORRIS CD ; CARSON S.** Routine vitamin supplementation to prevent cardiovascular disease: a summary of the evidence for the U.S. *Preventive Services Task Force. Ann Intern Med.,* 2003, vol. 139 (1), 56-70
- **BOILEAU TW ; LIAO Z ; KIM S et al.** Prostate carcinogenesis in N-methyl-N-nitrosourea (NMU)-testosterone-treated rats fed tomato powder, lycopene, or energy-restricted diets. *JNatl Cancer Inst.,* 2003, vol. 95 (21), 1578-1586
- **RAMBERG J ; LE L ; VENNUM E et al.** Why are natural source dietary supplements best? A review based on the vitamin C literature. *GlycoScience & Nutrition (Official Publication of GlycoScience com: The Nutrition Science Site),* 2003, vol. 4 (4), 1-8
- **CAO GI BOOTH SL ; SADOWSKI JA et al.** Increases in human plasma antioxidant capacity after consumption of controlled diets high in fruit and vegetables. *Am J Clin Nutr.,* 1998, vol. 68 (5), 1081-1087
- **BARHOUMI R ; BURGHARDT RG ; BUSBEE DL et al.** Enhancement of glutathione levels and protection from chemically initiated glutathione depletion in rat liver cells by glyconutritionals. *Proc Fisher Inst Med Res.,* 1997, vol. 1 (1), 12-16
- **GOUX WJ ; BOYD S ; TONE CM et al.** Effect of glyconutritionals on oxidative stress in human subjects: a pilot study. *GlycoScience & Nutrition (Official Publication of GlycoScience com: The Nutrition Science Site),* 2001, vol. 2 (12), 1-10
- **LEONARD SS ; CUTLER D1 DING MI et al.** Antioxidant properties of fruit and vegetable juices: more to the story than ascorbic acid. *Ann Clin Lab Sci.,* 2002, vol. 32 (2), 193-200
- **RICE-EVANS CAI MILLER NJ.** Antioxidant activities of flavonoids as bioactive components of food. *Biochem Soc Trans.,* 1996, vol. 24 (3), 790-795
- **GIL MI ; FERRERES F ; TOMAS-BARBERAN FA.** Effect of postharvest storage and processing on the antioxidant constituents (flavonoids and vitamin C) of fresh-cut spinach. *J Agric Food Chem.,* 1999, vol. 47 (6), 2213-2217
- **KURILICH AC ; JEFFERY EH ; JUVIK JA et al.** Antioxidant capacity of different broccoli (Brassica oleracea) genotypes using the oxygen radical absorbance capacity (ORAC) assay. *J Agric Food Chem,* 2002, vol. 50 (18), 5053-5057
- **WANG SY ; ZHENG W ; GALLETTA GJ.** Cultural system affects fruit quality and antioxidant capacity in strawberries. *J Agric Food Chem,* 2002, vol. 50 (22), 6534-6542

- **BRAND JC ; CHERIKOFF V ; LEE A et al.** An outstanding food source of vitamin C. *Lancet,* 1982, vol. 2 (8303), 873
- **RAMBERG J. GREEN TEA.** *GlycoScience & Nutrition (Official Publication of GlycoScience com: The Nutrition Science Site),* 2003, vol. 4 (5), 1-9
- **MCANALLEY S ; KOEPKE C ; LAM L et al.** In vitro methods for testing antioxidant potential: a review. *GlycoScience & Nutrition (Official Publication of GlycoScience com: The Nutrition Science Site),* 2003, vol. 4 (2), 1-9
- **CAO GI ALESSIO HM ; CUTLER RG.** Oxygen-radical absorbance capacity assay for antioxidants [see comments. *Free Radic Biol Med.,* 1993, vol. 14 (3), 303-311
- **ESTERBAUER HI JURGENS GI QUEHENBERGER 0 et al.** Autooxidation of human low density lipoprotein: loss of polyunsaturated fatty acids and vitamin E and generation of aldehydes. *J Lipid Res,* 1987, vol. 28 (5), 495-509
- **OHISHI N ; OHKAWA HI MIIKE A et al.** A new assay method for lipid peroxides using a methylene blue derivative. *Biochem Int.,* 1985, vol. 10 (2), 205-211
- **SHIGENAGA MK ; AMES BN.** Assays for 8-hydroxy-2'-deoxyguanosine: a biomarker of in vivo oxidative DNA damage. *Free Radic Biol Med.,* 1991, vol. 10 (3-4), 211-216
- **PRIOR RL ; CAO G.** In vivo total antioxidant capacity: Comparison of different analytical methods. *Free Radic Biol Med,* 1999, vol. 27 (11/12), 1173-1181
- Oxygen Radical Absorption Capacity Assay for measuring antioxidant activity. *Genox Corporation,* October 2001
- **RAHMAN I ; MACNEE W.** Role of oxidants/antioxidants in smoking-induced lung diseases. *Free Radic Biol Med,* 1996, vol. 21 (5), 669-681
- **CROSS CE ; VAN DER VLIET A ; O'NEILL CAI et al.** Reactive oxygen species and the lung. *Lancet,* 1994, vol. 344 (8927), 930-933
- **STEINMETZ KA ; POTTER JL.** Vegetables, fruit, and cancer prevention. *a review. Am. Diet Assoc.,* 1996, vol. 96 (10), 1027-109
- **BYERS TI GUERRERO N.** Epidemiologic evidence for vitamin C and vitamin E in cancer prevention. *Am J Clin Nutr,* 1995, vol. 62 (6), 1385S-1392s
- Nutrition and Your Health: Dietary Guidelines for Americans. *Home and Garden Bulletin No. 232, USDA and USDHHS,* 1995
- **HALLIWELL B ; GUTTERIDGE JMC.** Free Radicals in Biology and Medicine. Oxford University Press, 2000
- **SCHMIDT MC ; ASKEW EW ; ROBERTS DE et al.** Oxidative stress in humans training in a cold, moderate altitude environment and their response to a phytochemical antioxidant supplement. *Wilderness Environ Med.,* 2002, vol. 13 (2), 94-105